# FASCICULE DE BREVET EUROPEEN

(11) **EP 0 966 473 B1**
(45) Date de publication et mention de la délivrance du brevet: **24.07.2002**
(21) Numéro de dépôt: 98912552.1
(22) Date de dépôt: 26.02.1998
(51) Int. Cl.: C07D 513/06, A61K 31/54, A61K 31/535, A61K 31/425, C07D 215/14, C07D 215/16, C07D 279/16, C07D 265/36, C07D 517/06

(54) **THIAZOLOBENZOHETEROCYCLES, LEUR PREPARATION ET LES MEDICAMENTS LES CONTENANT**
THIAZOLOBENZOHETERORINGE, IHRE HERSTELLUNG UND DIE SIE ENTHALTENDE ARZNEIMITTEL
THIAZOLOBENZOHETEROCYCLES, PREPARATION AND MEDICINES CONTAINING SAME

(30) Priorité: 28.02.1997 FR 9702436
(43) Date de publication de la demande: 29.12.1999
(73) Titulaire: Aventis Pharma S.A., 92160 Antony (FR)
(72) Inventeur: HARDY, Jean-Claude, F-95800 Cergy Saint Christophe (FR); BOUQUEREL, Jean, F-93700 Drancy (FR); NEMECEK, Patrick, F-94320 Thiais (FR); ALOUP, Jean-Claude, F-94290 Villeneuve le Roi (FR); MIGNANI, Serge, F-92290 Châtenay Malabry (FR); PEYRONEL, Jean-François, F-91120 Palaiseau (FR)
(86) Numéro de dépôt international: FR9800376
(87) Numéro de publication internationale: WO9838194

(56) Documents cités:
- EP-A- 0 348 872
- EP-A- 0 509 398
- EP-A- 0 705 835
- WO-A-90/15058
- US-A- 3 992 378
- CHEMICAL ABSTRACTS, vol. 59, no. 10, 11 novembre 1963 Columbus, Ohio, US; abstract no. 11471b, XP002046065 & A. RICHARDSON, JR.: JOURNAL OF ORGANIC CHEMISTRY, vol. 28, no. 10, 1963, EASTON US, pages 2581-2587, cité dans la demande
- PATENT ABSTRACTS OF JAPAN vol. 004, no. 173 (C-032), 29 novembre 1980 & JP 55 111406 A (TAKEDA CHEM. IND., LTD.) -& JP 55 111 406 A
- M. HOENEL ET AL.: JOURNAL OF THE CHEMICAL SOCIETY, PERKIN TRANSACTIONS 1, vol. 9, 1980, LETCHWORTH GB, pages 1933-1939, XP002046064
- CHEMICAL ABSTRACTS, vol. 79, no. 3, 23 juillet 1973 Columbus, Ohio, US; abstract no. 18669r, XP002046066 & K.V. RAO ET AL.: J. HETEROCYCL. CHEM., vol. 10, no. 2, 1973, pages 213-215,

## Description

Des dérivés de thiazolobenzohétérocycle sont décrits par A. RICHARDSON, J. Org. Chem, 28, 2581-87 (1963) mais aucune activité pharmacologique n'est mentionnée pour ces produits.

La présente invention concerne des composés de formule : 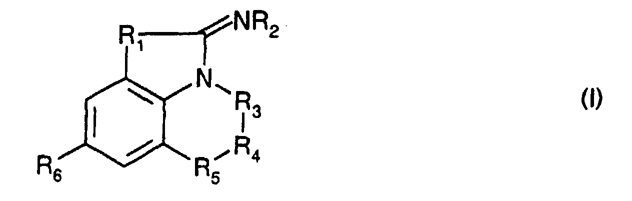 leurs sels, leurs procédés de préparation et les médicaments les contenant.

Dans la formule (I),
R₁ représente un atome de soufre ou de sélénium,
R₂ représente un atome d'hydrogène ou un radical alkyle,
-R₃-R₄-R₅- représente une chaîne de formule -CH₂-CH₂-CH₂-, -CH(R₇)-CH₂-CH₂-, -CH₂-CH₂-CH(R₈)-, -CH₂-CH(R₉)-CH₂-, -CH₂-CH₂-S-, -CH₂-CH₂-SO-, -CH₂-CH₂-SO₂-, -CH₂-CH₂-Se-, -CH₂-CH₂-O-, -CH₂-CH₂-CO-, -CH₂-CH₂-N(R₁₀)-, -CH₂-CO-N(R₁₀)-, -CH₂-CF₂-CH₂-, -CH₂-CF₂-CH(OH)-, -CH₂-CH(R₁₃)-S-, -CH₂-CH(R₁₃)-SO- ou -CH₂-CH(R₁₃)-SO₂-,
R₆ représente un radical polyfluoroalkyle, polyfluoroalcoxy ou polyfluoroalkylthio,
R₇ représente un radical alkyle, -CH₂OH, -CH₂-SO₂-alk ou -CH₂-NR₁₁R₁₂,
R₈ représente un radical alkyle, hydroxy, -CH₂OH, -NR₁₁R₁₂, -CH₂-NR₁₁R₁₂, -S-alk, -SO-alk, -SO₂-alk, thiényle, furyle, phényle ou phényle substitué par un substituant choisi parmi les atomes d'halogène et les radicaux alkyle et alcoxy,
R₉ représente un radical alkyle ou -CH₂OH,
R₁₀ représente un atome d'hydrogène ou un radical alkyle,
R₁₁ représente un atome d'hydrogène ou un radical alkyle, -CO-alk ou -CO-CF₃,
R₁₂ représente un atome d'hydrogène ou un radical alkyle,
   ou bien R₁₁ et R₁₂ forment avec l'atome d'azote auquel ils sont rattachés un hétérocycle à 5 ou 6 chaînons, saturé ou insaturé et comportant éventuellement un autre hétéroatome choisi parmi azote, oxygène et soufre, cet hétérocycle étant non substitué ou substitué par un ou plusieurs substituants choisis parmi les radicaux alkyle, phényle, halogénophényle et phénylalkyle,
R₁₃ représente un radical alkyle ou -CH₂OH,
alk représente un radical alkyle.

Dans les définitions qui précédent et celles qui seront citées ci-après, sauf mention contraire, les radicaux et portions alkyle et alcoxy contiennent 1 à 6 atomes de carbone en chaîne droite ou ramifiée et les atomes d'halogène sont les atomes de brome, de chlore, de fluor et d'iode.

Parmi les radicaux polyfluoroalkyle on peut citer les radicaux trifluorométhyle, 2,2,2-trifluoroéthyle, 1,1,2,2-tétrafluoroéthyle, perfluoroéthyle, perfluoropropyle, perfluorobutyle.

Parmi les radicaux polyfluoroalcoxy on peut citer les radicaux trifluorométhoxy, perfluoroéthoxy, 2,2,2-trifluoroéthoxy, 1,1,2,2-tétrafluoroéthoxy, 2,2,3,3,3-pentafluoropropoxy, perfluoropropoxy, perfluorobutoxy.

Parmi les radicaux polyfluoroalkythio on peut citer les radicaux trifluorométhylthio, perfluoroéthylthio, perfluoropropylthio.

Les radicaux polyfluoroalkyle, polyfluoroalcoxy et polyfluoroalkylthio préférés sont les radicaux trifluorométhyle, trifluorométhoxy, pentafluoroéthoxy et trifluorométhylthio.

Comme hétérocycle à 5 ou 6 chaînons, saturé ou insaturé et comportant éventuellement un autre hétéroatome choisi parmi azote, oxygène et soufre on peut citer la pyrrolidine, la pipéridine, la pipérazine, la morpholine et la thiomorpholine, ces hétérocycles étant non substitués ou substitués par un radical alkyle, phényle, halogénophényle ou phénylalkyle.

L'invention concerne également les sels d'addition des composés de formule (I) avec les acides minéraux ou organiques.

Les composés de formule (I) qui comportent un ou plusieurs centres asymétriques présentent des formes isomères, ces isomères et mélanges font partie de l'invention. Les racémiques et les énantiomères de ces composés font également partie de l'invention.

Les composés de formule (I) pour lesquels R₁ représente un atome de soufre ou de sélénium, R₂ représente un atome d'hydrogène, -R₃-R₄-R₅- représente une chaîne de formule -CH₂-CH₂-CH₂-, -CH(R₇)-CH₂-CH₂-, -CH₂-CH₂-CH(R₈)-, -CH₂-CH(R₉)-CH₂, -CH₂-CH₂-S-, -CH₂-CH₂-Se-, -CH₂-CH₂-O-, -CH₂-CH₂-CO-, -CH₂-CH₂-N(R₁₀)-, -CH₂-CO-N(R₁₀)-, -CH₂-CF₂-CH₂-, -CH₂-CF₂-CH(OH)-, -CH₂-CH(R₁₃)-S-, sous réserve que R₈ ne représente un radical hydroxy peuvent être préparés par action d'un thiocyanate de métal alcalin ou d'un sélénocyanate de métal alcalin sur un dérivé de formule : 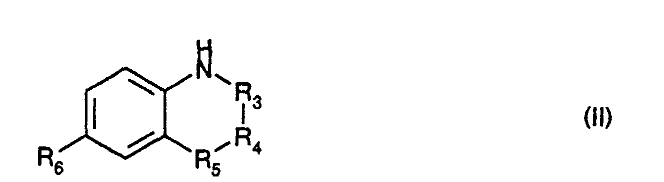 dans laquelle R₆ a les mêmes significations que dans la formule (I), -R₃-R₄-R₅- représente une chaîne de formule -CH₂-CH₂-CH₂-, -CH(R₇)-CH₂-CH₂-, -CH₂-CH₂-CH(R₈)-, -CH₂-CH(R₉)-CH₂, -CH₂-CH₂-S-, -CH₂-CH₂-Se-, -CH₂-CH₂-O-, -CH₂-CH₂-CO-, -CH₂-CH₂-N(R₁₀)-, -CH₂-CF₂-CH₂-, -CH₂-CF₂-CH(OH)-, -CH₂-CH(R₁₃)-S-, -CH₂-CO-N(R₁₀)- dans lesquelles R₇, R₈, R₉, R₁₀ et R₁₃ ont les mêmes significations que dans la formule (I) sous réserve que R₈ ne représente pas un radical hydroxy.

Cette réaction s'effectue généralement en présence de brome, de chlore, de chloramide ou de chlorure cuivrique, au sein d'un solvant organique tel que l'acide acétique, à une température comprise entre 15°C et la température d'ébullition du milieu réactionnel. Comme thiocyanate de métal alcalin, il est préférable d'utiliser le thiocyanate de potassium. Comme sélénocyanate de métal alcalin, il est préférable d'utiliser le sélénocyanate de potassium.

Les dérivés de formule (II), à l'exception de la 6-trifluorométhyl-1,2,3,4-tétrahydroquinoléine, la 2-méthyl-6-trifluorométhoxy-1,2,3,4-tétrahydroquinoléine, la 6-trifluorométhyl-1,2,3,4-tétrahydroquinoxaline et la 2-oxo-7-trifluorométhyl-3,4-dihydroquinoxaline décrites en particulier dans US 3 992 378, J.C.S. Perkin I (1980), 1933 et Chem. Abstracts (1973), 79: 18669r, sont nouveaux et en tant que tels font partie de l'invention.

Les composés de formule (I) pour lesquels R₂ représente un radical alkyle peuvent être préparés par alkylation d'un composé de formule (I) correspondant pour lequel R₂ représente un atome d'hydrogène.

Cette alkylation s'effectue par toute méthode permettant d'alkyler une fonction imine. De préférence, on opère au moyen d'un dérivé Ra-X dans lequel Ra représente un radical alkyle et X représente un groupe réactif tel qu'un atome d'halogène (de préférence chlore, brome ou iode) ou un radical tosyloxy, au sein d'un solvant organique inerte tel qu'un alcool aliphatique inférieur (éthanol, propanol, butanol par exemple), une cétone (acétone, méthyléthylcétone par exemple) ou le diméthylformamide, en présence d'une base telle qu'un carbonate de métal alcalin (carbonate de potassium par exemple), à une température comprise entre 20°C et la température d'ébullition du milieu réactionnel.

Les composés de formule (I) pour lesquels -R₃-R₄-R₅- représente une chaîne de formule -CH₂-CH₂-CH(R₈)- et R₈ représente un radical hydroxy peuvent être obtenus par réduction d'un composé de formule (I) correspondant pour lequel -R₃-R₄-R₅- représente une chaîne de formule -CH₂-CH₂-CO-.

Cette réaction s'effectue par toute méthode permettant de passer d'une cétone à un alcool. On opère généralement au moyen de borohydrure de sodium, au sein d'un alcool tel que le méthanol ou l'éthanol, à une température comprise entre 0 et 25°C.

Les composés de formule (I) pour lesquels -R₃-R₄-R₅- représente une chaîne de formule -CH₂-CH₂-SO-, -CH₂-CH₂-SO₂-, -CH₂-CH(R₁₃)-SO- ou -CH₂-CH(R₁₃)-SO₂- peuvent être préparés par oxydation d'un composé de formule (I) correspondant pour lequel -R₃-R₄-R₅- représente une chaîne de formule -CH₂-CH₂-S- ou -CH₂-CH(R₁₃)-S-.

Cette oxydation s'effectue selon les méthodes connues d'oxydation des dérivés soufrés comme celles décrites par M. HUDLICKY, Oxidations in Organic Chemistry, ACS Monograph, 186, 252-263 (1990). Par exemple, on opère par action d'un peracide organique ou un sel d'un tel acide (acide percarboxylique ou persulfonique, notamment l'acide perbenzoïque, l'acide 3-chloroperbenzoïque, l'acide 4-nitroperbenzoïque, l'acide peracétique, l'acide pertrifluoracétique, l'acide performique, l'acide monoperphtalique) ou les peracides minéraux ou un sel d'un tel acide (par exemple l'acide periodique ou persulfurique), au sein d'un solvant inerte tel qu'un solvant chloré (chloroforme, dichlorométhane par exemple), à une température comprise entre 0 et 25°C. On peut utiliser également le peroxyde d'hydrogène ou un périodate (périodate de sodium par exemple), au sein d'un solvant inerte tel qu'un alcool aliphatique inférieur, l'eau ou un mélange de ces solvants, à une température comprise entre 0 et 20°C. Il est également possible d'opérer au moyen de tertiobutylhydroperoxyde en présence de tétraisopropylate de titane ou d'oxone^{R} (peroxymonosulfate de potassium) au sein d'un alcool aliphatique inférieur ou un mélange eau-alcool, à une température voisine de 25°C.

Les composés de formule (I) pour lesquels -R₃-R₄-R₅- représente une chaîne de formule -CH(R₇)-CH₂-CH₂- dans laquelle R₇ représente un radical -CH₂-NR₁₁R₁₂ et R₂ représente un atome d'hydrogène peuvent également être préparés par action d'une amine HNR₁₁R₁₂ pour laquelle R₁₁ et R₁₂ ont les mêmes significations que dans la formule (I) sur un dérivé de formule : 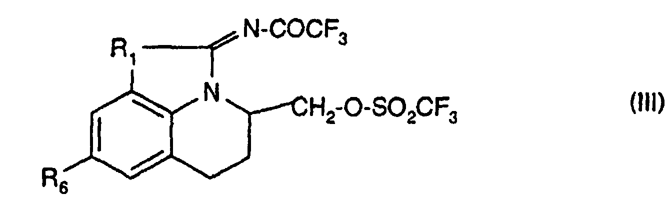 dans laquelle R₁ et R₆ ont les mêmes significations que dans la formule (I) suivie d'une hydrolyse pour libérer l'imine.

Cette réaction s'effectue généralement au sein d'un solvant inerte tel qu'un solvant chloré (chloroforme, dichlorométhane par exemple), à une température comprise entre 0 et 50°C. L'hydrolyse s'effectue de préférence au moyen d'un carbonate de métal alcalin (carbonate de potassium par exemple), en milieu hydroalcoolique, à une température voisine de 20°C.

Les dérivés de formule (III) peuvent être obtenus à partir des composés de formule (I) correspondants pour lesquels -R₃-R₄-R₅- représente une chaîne de formule -CH(R₇)-CH₂-CH₂- dans laquelle R₇ représente un radical -CH₂OH et R₂ représente un atome d'hydrogène selon le schéma réactionnel suivant :

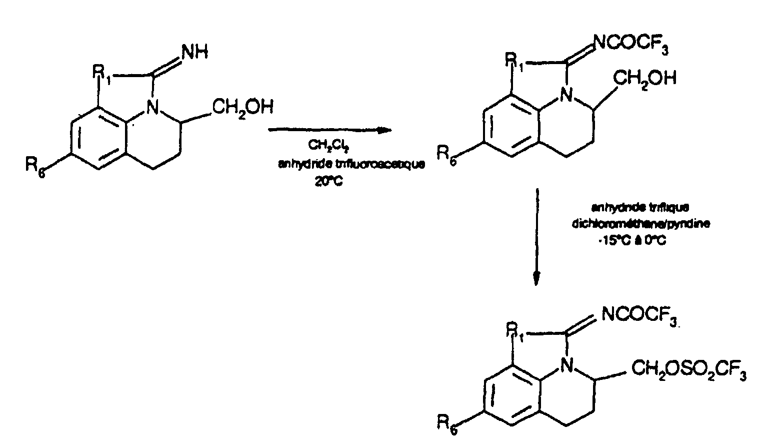

Dans ces formules, R₁ et R₆ ont les mêmes significations que dans la formule (I).

Les dérivés de formule (II) pour lesquels R₆ a les mêmes significations que dans la formule (I) et -R₃-R₄-R₅- représente une chaîne de formule -CH₂-CH₂-CH₂-, -CH(R₇)-CH₂-CH₂-, -CH₂-CH₂-CH(R₈)-, -CH₂-CH(R₉)-CH₂, R₇ représente un radical alkyle, -CH₂OH, -CH₂-NR₁₁R₁₂ ou -CH₂-SO₂-alk, R₈ représente un radical alkyle ou -CH₂-NR₁₁R₁₂ et R₉ représente un radical alkyle peuvent être obtenus par hydrogénation d'un dérivé de formule : 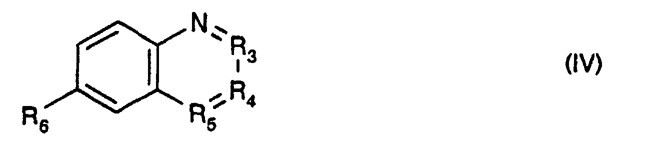 dans laquelle R₆ a les mêmes significations que dans la formule (I) et =R₃-R₄=R₅- représente une chaîne de formula =CH-CH=CH-, =C(R₇)-CH=CH-, =CH-CH=C(R₈), =CH-C(R₉)=CH-, R₇ représente un radical alkyle, -CH₂OH, -CH₂-NR₁₁R₁₂ ou -CH₂-SO₂-alk, R₈ représente un radical alkyle ou -CH₂-NR₁₁R₁₂, R₉ représente un radical alkyle, R₁₁ et R₁₂ ont les mêmes significations que dans la formule (I).

Cette hydrogénation s'effectue généralement soit au moyen d'hydrogène, sous une pression de 2 à 12 bar, au sein d'un solvant organique inerte tel qu'un alcool aliphatique inférieur (méthanol, éthanol par exemple) ou le tétrahydrofuranne ou un mélange de ces solvants, en présence d'un catalyseur d'hydrogénation tel que l'oxyde de platine, à une température voisine de 20°C soit au moyen d'agents réducteurs tels que le borohydrure de sodium en présence de chlorure de nickel ou de cyanoborohydrure de sodium, au sein d'un alcool (méthanol ou éthanol par exemple), à une température voisine de 20°C.

Les dérivés de formule (IV) pour lesquels =R₃-R₄=R₅- représente une chaîne de formule =CH-CH=CH-, =C(R₇)-CH=CH-, =CH-C(R₉)=CH-, R₇ représente un radical alkyle et R₉ représente un radical alkyle peuvent être obtenus à partir d'une 4-polyfluoroalkylaniline, 4-polyfluoroalcoxyaniline ou 4-polyfluoroalkylthioaniline par application ou adaptation des méthodes décrites dans les exemples et des méthodes décrites par G. JONES, Heterocyclic compounds, Quinolines, 32, part 1, Interscience, 93-318 (1977); J. Pharm. Sci, 68 (3), 336-8 (1979).

Les 4-polyfluoroalkylaniline, 4-polyfluoroalcoxyaniline et 4-polyfluoroalkylthioaniline sont commercialisées ou peuvent être obtenues par application ou adaptation des méthodes décrites dans J. Org. Chem., 29, 1 (1964), et dans les brevets US 3920444, US 2436100, DE 2606982, EP 205821 et EP546391.

Les dérivés de formule (IV) pour lesquels =R₃-R₄=R₅- représente une chaîne de formule =C(R₇)-CH=CH- dans laquelle R₇ représente un radical -CH₂-NR₁₁R₁₂ peuvent être obtenus à partir d'une 2-méthyl-6-polyfluoroalkyle quinoléine ou 2-méthyl-6-polyfluoroalcoxy quinoléine ou 2-méthyl-6-polyfluoroalkylthioquinoléine correspondante selon le schéma réactionnel suivant : 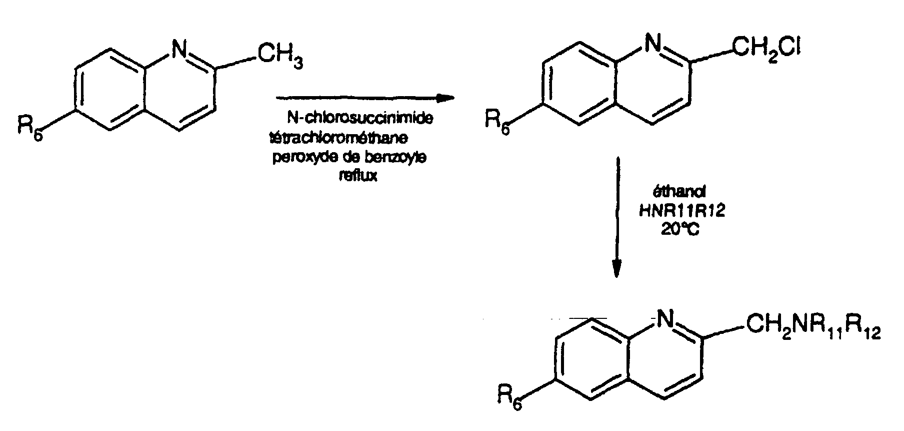 Dans ces formules R₆, R₁₁ et R₁₂ ont les mêmes significations que dans la formule (I).

Les dérivés de formule (IV) pour lesquels R₆ a les mêmes significations que dans la formule (I) et =R₃-R₄=R₅- représente une chaîne de formule =C(R₇)-CH=CH-, R₇ représente un radical -CH₂OH peuvent être obtenus par action d'anhydride acétique sur un 2-méthyl-6-polyfluoroalkylquinoléine-1-oxyde ou 2-méthyl-6-polyfluoroalcoxyquinoléine-1-oxyde ou 2-méthyl-6-polyfluoroalkylthioquinoléine-1-oxyde à la température d'ébullition du milieu réactionnel, suivie d'une hydrolyse par exemple par action d'une solution diluée d'un hydroxyde alcalin, au sein d'un solvant tel qu'un mélange eaudioxanne, à la température d'ébullition du milieu réactionnel.

Les dérivés de formule (IV) pour lesquels =R₃-R₄=R₅- représente une chaîne de formule =C(R₇)-CH=CH- et R₇ représente un radical -CH₂-SO₂-alk peuvent être obtenus par action d'un 6-polyfluoroalkylquinoléine-1-oxyde, 6-polyfluoroalcoxyquinoléine-1-oxyde ou 6-polyfluoroalkylthioquinoléine-1-oxyde et d'anhydride acétique sur un dérivé alk-SO₂-CH₂-COCH₃ dans lequel alk représente un radical alkyle, au sein de l'éther diméthylique de l'éthyléneglycol, à une température variant de 0 à 80°C.

Les 2-méthyl-6-polyfluoroalkylquinoléine-1-oxyde, 2-méthyl-6-polyfluoroalcoxyquinoléine-1-oxyde ou 2-méthyl-6-polyfluoroalkylthioquinoléine-1-oxyde et les 6-polyfluoroalkylquinoléine-1-oxyde, 6-polyfluoroalcoxyquinoléine-1-oxyde ou 6-polyfluoroalkylthioquinoléine-1-oxyde peuvent être obtenus par oxydation des quinoléines correspondantes, au moyen d'un agent d'oxydation tel que l'acide 3-chloroperbenzoique, au sein d'un solvant organique inerte tel qu'un solvant chloré (chloroforme, dichlorométhane par exemple), à une température voisine de 20°C.

Les dérivés de formule (IV) pour lesquels =R₃-R₄=R₅- représente une chaîne de formule =C(R₇)-CH=CH- ou =CH-CH=C(R₈)- et R₇ et R₈ représentent des radicaux -CH₂-NR₁₁R₁₂ peuvent être obtenus par action d'une amine HNR₁₁R₁₂ dans laquelle R₁₁ et R₁₂ ont les mêmes significations que dans la formule (I) sur un dérivé de formule (IV) correspondant pour lequel =R₃-R₄=R₅- représente une chaîne de formule =C(R₇)-CH=CH- ou =CH-CH=C(R₈)- et R₇ et R₈ représentent des radicaux -CH₂OH sous forme d'un dérivé réactif.

Cette réaction s'effectue généralement au sein d'un solvant organique inerte tel qu'un éther (tétrahydrofuranne, dioxanne par exemple), un solvant chloré (chloroforme par exemple), en présence d'une base, à une température comprise entre 20°C et la température d'ébullition du milieu réactionnel. Comme dérivé réactif, on peut citer le chlorure, le tosylate ou le mésylate.

Les dérivés de formule (IV) pour lesquels =R₃-R₄=R₅- représente une chaîne de formule =CH-CH=C(R₈)- et R₈ représente un radical alkyle peuvent être obtenus par application ou adaptation de la méthode décrite par KRAINER et coll., Chem. Heterocycl. Compd. 9, 217-219 (1973) ou Khim. Geterotsikl. Soedin., 9 (2), 235-238 (1973).

Les dérivés de formule (II) pour lesquels -R₃-R₄-R₅- représente une chaîne de formule -CH₂-CH₂-CH(R₈)- et R₈ représente un radical -CH₂-OH, thiényle, furyle, phényle ou phényle substitué par un substituant choisi parmi les atomes d'halogène et les radicaux alkyle et alcoxy ou -NR₁₁R₁₂ dans lequel R₁₁ et R₁₂ représentent des atomes d'hydrogène ou R₁₁ représente un radical -CO-alk et R₁₂ représente un radical alkyle ou R₁₁ représente un atome d'hydrogène et R₁₂ représente un radical alkyle ou R₁₁ et R₁₂ forment avec l'atome d'azote auquel ils sont attachés un hétérocycle à 5 ou 6 chaînons, saturé ou insaturé et comportant éventuellement un autre hétéroatome choisi parmi azote, oxygène et soufre et éventuellement substitué par un radical alkyle, phényle, halogénophényle ou phénylalkyle peuvent être obtenus par action d'un dérivé de formule : 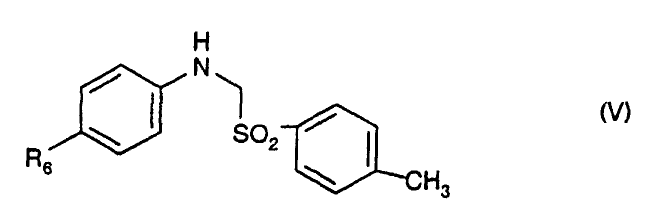 dans laquelle R₆ a les mêmes significations que dans la formule (I) sur un dérivé Rb-CH=CH₂ pour lequel Rb représente un radical -CH₂-OH, thiényle, furyle, phényle ou phényle substitué par un substituant choisi parmi les atomes d'halogène et les radicaux alkyle et alcoxy ou -NR₁₁R₁₂ dans lequel soit R₁₁ et R₁₂ forment avec l'atome d'azote auquel ils sont rattachés un radical phtalimido suivie d'une hydrolyse pour obtenir le dérivé pour lequel R₁₁ et R₁₂ sont des atomes d'hydrogène, soit R₁₁ représente un radical -CO-alk et R₁₂ représente un radical alkyle suivie éventuellement d'une hydrolyse pour obtenir les dérivés pour lesquels R₁₁ représente un atome d'hydrogène et R₁₂ représente un radical alkyle, soit R₁₁ et R₁₂ forment avec l'atome d'azote auquel ils sont attachés un hétérocycle à 5 ou 6 chaînons, saturé ou insaturé et comportant éventuellement un autre hétéroatome choisi parmi azote, oxygène et soufre et éventuellement substitué par un radical alkyle, phényle, halogénophényle ou phénylalkyle.

Cette réaction s'effectue généralement au sein d'un solvant organique inerte tel qu'un solvant chloré (chloroforme, dichlorométhane par exemple), en présence d'un acide de Lewis tel que le tétrachlorure d'étain, le tétrachlorure de titane, l'éthérate de trifluorure de bore, à une température variant de -80°C à une température voisine de 20°C. L'hydrolyse du phtalimido et du dérivé acylé s'effectue généralement au moyen d'un acide tel que l'acide chlorhydrique, en milieu aqueux, à une température comprise entre 20°C et la température d'ébullition du milieu réactionnel.

Les dérivés de formule (V) peuvent être obtenus par action de formaldéhyde et de p-toluènesulfinate de sodium puis d'une 4-polyfluoroalkylaniline, 4-polyfluoroalcoxyaniline ou 4-polyfluoroalkythioaniline, en milieu aqueux, en présence d'acide chlorhydrique, à une température voisine de 25°C.

Les dérivés Rb-CH=CH₂ sont commercialisés ou peuvent être obtenus par application ou adaptation des méthodes décrites par ABARCA et coll, Tetrahedron, 43 (1), 269-274 (1987); NEGISHI et coll., Heterocycles, 18 spec. Issue, 117-22 (1982); REIJENDAM et coll., Tetrahedron, 26, 1291 (1970); HACHIHAMA et coll., Chem.Abstr., 44, 9720f (1950); TAGAKI et coll., Tetrahedron Lett., 2587 (1974); Chem.Abstr., 65, 18503e (1966); Chem.Abstr., 71, 49326r (1969); Chem.Abstr., 63, 18119f (1965) et Chem.Abstr., 58, 8083b.

Les dérivés de formule (II) pour lesquels -R₃-R₄-R₅- représente une chaîne de formule -CH₂-CH₂-CH(R₈)- et R₈ représente un radical -NR₁₁R₁₂ dans lequel R₁₁ représente un atome d'hydrogène et R₁₂ représente un radical -CO-alk peuvent être obtenus par acylation d'un dérivé de formule (II) correspondant pour lequel R₁₁ et R₁₂ représentent des atomes d'hydrogène.

Cette réaction s'effectue généralement au moyen d'un halogènure d'acyle et, en particulier, d'un chlorure ou bromure d'acyle, au sein d'un solvant inerte tel que le tétrahydrofuranne, à une température comprise entre 20°C et la température d'ébullition du milieu réactionnel.

Les dérivés de formula (II) pour lesquels -R₃-R₄-R₅- représente une chaîne de formule -CH₂-CH₂-CH(R₈)- et R₈ représente un radical -NR₁₁R₁₂ dans lequel R₁₁ représente un radical alkyle (2-6C) et R₁₂ représente un radical alkyle peuvent être obtenus par réduction d'un dérivé de formula (II) correspondant pour lequel R₁₂ représente un radical alkyle et R₁₁ représente un radical -CO-alk.

Cette réduction s'effectue généralement au moyen du complexe borane-sulfure de méthyle, au sein du tétrahydrofuranne, à une température voisine de 20°C.

Les dérivés de formule (II) pour lesquels -R₃-R₄-R₅- représente une chaîne de formule -CH₂-CH₂-CH(R₈)- dans laquelle R₈ représente un radical -NR₁₁R₁₂ pour lequel R₁₁ et R₁₂ forment avec l'atome d'azote auquel ils sont rattachés la pyrrolidine, la morpholine, la pipéridine ou la pipérazine peuvent également être obtenus par réduction d'un dérivé de formule : 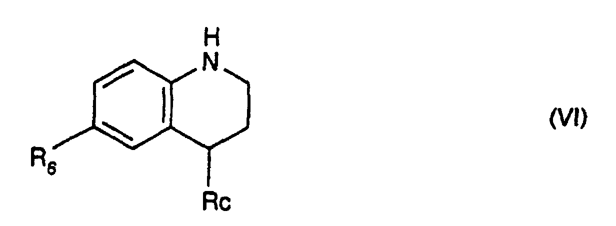 dans laquelle Rc représente un radical 2-oxo-pyrrolidin-1-yl, 3-oxo-morpholin-4-yl, 2-oxo-pipéridin-1-yl ou 2-oxo-pipérazin-1-yl et R₆ a les mêmes significations que dans la formule (I).

Cette réduction s'effectue généralement au moyen du complexe borane-sulfure de méthyle, au sein du tétrahydrofuranne, à une température voisine de 20°C.

Les dérivés de formule (VI) sont eux-mêmes obtenus selon le procédé mentionné ci-dessus à partir d'un dérivé de formule (V) et d'un dérivé Rb-CH=CH₂ pour lequel Rb représente un radical 2-oxo-pyrrolidin-1-yl, 3-oxo-morpholin-4-yl, 2-oxo-pipéridin-1-yl ou 2-oxo-pipérazin-1-yl.

Les dérivés de formule (II) pour lesquels -R₃-R₄-R₅- représente une chaîne de formule -CH₂-CH₂-CH(R₈)- dans laquelle R₈ représente un radical -NR₁₁R₁₂, R₁₂ représente un atome d'hydrogène ou un radical alkyle et R₁₁ représente un radical -CO-CF₃ peuvent être obtenus par action d'anhydride trifluoroacétique avec un dérivé de formule (II) correspondant pour lequel -R₃-R₄-R₅- représente une chaîne de formule -CH₂-CH₂-CH(R₈)- dans laquelle R₈ représente un radical -NR₁₁R₁₂, R₁₂ représente un atome d'hydrogène ou un radical alkyle et R₁₁ représente un atome d'hydrogène.

Cette réaction s'effectue généralement au sein de la pyridine, à une température voisine de -30°C.

Les dérivés de formule (II) pour lesquels -R₃-R₄-R₅- représente une chaîne de formule -CH₂-CH₂-CH(R₈)- dans laquelle R₈ représente un radical -NR₁₁R₁₂ ou -S-alk peuvent être obtenus par action d'un dérivé HR₈ pour lequel R₈ représente un radical -NR₁₁R₁₂ ou -S-alk, R₁₁, R₁₂ et alk ayant les mêmes significations que dans la formule (I) sur un dérivé de formule : 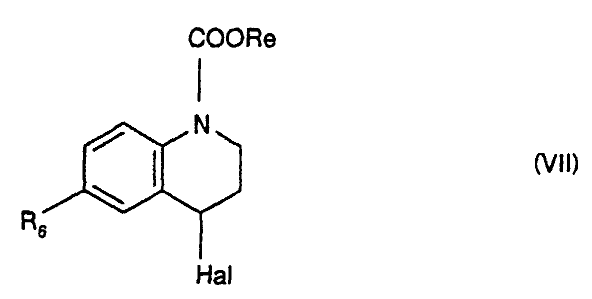 dans laquelle R₆ a les mêmes significations que dans la formule (I), Hal représente un atome d'halogène et de préférence un atome de chlore ou de brome et Re représente un radical tert-butyle puis déprotection de l'azote cyclique.

Lorsque R₈ représente un radical -NR₁₁R₁₂ cette réaction s'effectue généralement au sein d'un alcool (éthanol, méthanol par exemple), à une température voisine de 20°C. Lorsque R₈ représente un radical -S-alk, cette réaction s'effectue généralement au sein du diméthylformamide, en présence d'un hydrure de métal alcalin (hydrure de sodium de préférence), à une température comprise entre 0 et 25°C. La déprotection s'effectue de préférence au moyen d'acide trifluoroacétique, au sein du dichlorométhane, à une température voisine de 20°C.

Les dérivés de formule (VII) peuvent être obtenus par halogénation d'une 6-polyfluoroalkyle-1,2,3,4-tétrahydroquinoléine-1-carboxylate de tert-butyle ou une 6-polyfluoroalcoxy-1,2,3,4-tétrahydroquinoléine-1-carboxylate de tert-butyle ou une 6-polyfluoroalkylthio-1,2,3,4-tétrahydroquinoléine-1-carbo xylate de tert-butyle correspondante.

Cette halogénation s'effectue par toute méthode connue de l'homme de l'art qui ne modifie pas le reste de la molécule. Par exemple, il est possible de bromer au moyen de N-bromosuccinimide, au sein d'un solvant inerte tel qu'un solvant chloré comme le tétrachlorure de carbone, en présence de peroxyde de benzoyle, à une température voisine de 20°C.

Les dérivés de formule (II) pour lesquels -R₃-R₄-R₅- représente une chaîne de formula -CH₂-CH₂-CH(R₈)- dans laquelle R₈ représente un radical -SO-alk
ou -SO₂-alk peuvent être obtenus par oxydation d'un dérivé de formula (II) correspondant pour lequel -R₃-R₄-R₅- représente une chaîne de formula -CH₂-CH₂-CH(R₈)- dans laquelle R₈ représente un radical -S-alk.

Cette oxydation s'effectue comme mentionné précédemment pour la préparation des composés de formule (I) pour lesquels -R₃-R₄-R₅- représente une chaîne de formule -CH₂-CH₂-SO-, -CH₂-CH₂-SO₂-, -CH₂-CH(R₁₃)-SO- ou -CH₂-CH(R₁₃)-SO₂-.

Les dérivés de formule (II) pour lesquels -R₃-R₄-R₅- représente une chaîne de formule -CH₂-CH₂-S-, -CH₂-CH₂-O-, -CH₂-CH₂-Se- ou -CH₂-CH₂-N(R₁₀)-peuvent être obtenus par réduction d'un dérivé de formule : 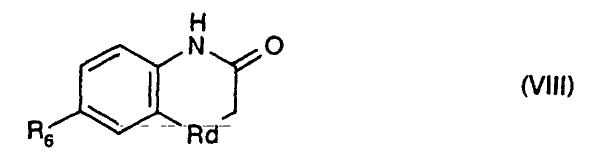 dans laquelle Rd représente un atome d'oxygène, de soufre, de sélénium ou N(R₁₀) et R₆ et R₁₀ ont les mêmes significations que dans la formule (I).

Cette réaction s'effectue au moyen d'un agent réducteur tel que le tétrahydroaluminate de lithium, au sein d'un solvant organique inerte tel que le tétrahydrofuranne, à une température voisine de 20°C.

Les dérivés de formule (VIII) peuvent être obtenus à partir d'un dérivé de formule : 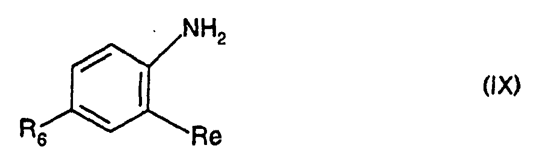 dans laquelle Re représente un radical OH, SH, SeH ou NH(R₁₀), R₆ et R₁₀ ont les mêmes significations que dans la formule (I), par application ou adaptation des méthodes décrites dans les exemples et par X. HUANG, Synthesis, 851-852 (1984), W.C. LUMMA et coll., J. Med. Chem., 24, 93-101 (1981) et E.J. JACOBSEN et coll., J. Med. Chem., 39, 158-175 (1996).

Les dérivés de formule (IX) peuvent être obtenus par application ou adaptation des méthodes décrites par R. BELCHER et coll., J. Chem. Soc., 3846 (1954); B.L. MYLARY, J. Med. Chem., 34, 108-122 (1991); D.W. COMBS et coll., J. Med. Chem., 35, 172-176 (1992), W.C. LUMMA et coll., J. Med. Chem., 24, 93-101 (1981) et A.V. ZEIGER et coll., J. Org. Chem., 42 (3), 542 (1977).

Les dérivés de formule (II) pour lesquels -R₃-R₄-R₅- représente une chaîne de formule -CH₂-CH₂-CO- peuvent être obtenus par oxydation d'un dérivé de formule (II) correspondant pour lequel -R₃-R₄-R₅- représente une chaîne de formule -CH₂-CH₂-CH₂-.

Cette réaction s'effectue généralement au moyen d'hydroperoxyde de tert-butyle et d'anhydride chromique en solution aqueuse, au sein d'un solvant inerte tel qu'un solvant chloré (dichlorométhane, chloroforme par exemple), à une température voisine de 20°C. De préférence, l'azote du dérivé de formule (II) correspondant pour lequel -R₃-R₄-R₅- représente une chaîne de formule -CH₂-CH₂-CH₂ est préalablement protégé.

Les dérivés de formule (II) pour lesquels -R₃-R₄-R₅- représente une chaîne de formule -CH₂-CO-N(R₁₀)- peuvent être obtenus par action de glycinate d'éthyle sur un dérivé de formule : 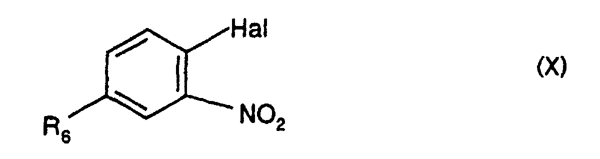 dans laquelle R₆ a les mêmes significations que dans la formule (I) et Hal représente un atome d'halogène et en particulier fluor suivie d'une réduction-cyclisation. Lorsque R₁₀ représente un radical alkyle, on alkyle avant la réduction-cyclisation.

Cette réaction s'effectue à une température comprise entre 20°C et la température d'ébullition du milieu réactionnel. La réduction-cyclisation s'effectue en une seule étape par traitement avec de l'étain en présence d'acide chlorhydrique, au sein d'éthanol aqueux, à la température d'ébullition du milieu réactionnel ou avec du nickel de Raney. L'alkylation s'effectue les méthodes décrites précédemment pour la préparation des composés de formule (I) pour lesquels R₂ représente un radical alkyle.

Les dérivés de formula (X) peuvent être obtenus par application ou adaptation de la méthode décrite dans Chem. Abstr. 113, 233655.

Les dérivés de formule (II) pour lesquels -R₃-R₄-R₅- représente une chaîne de formule -CH₂-CO-N(R₁₀)- peuvent être également obtenus selon le schéma réactionnel suivant :

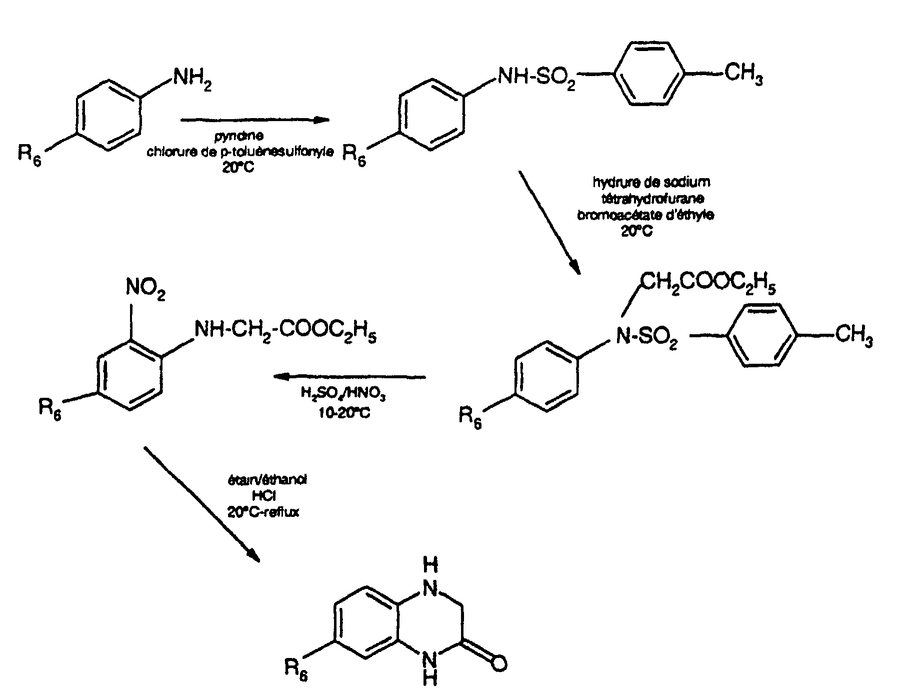

Dans ces formules R₆ a les mêmes significations que dans la formule (I).

Les dérivés de formule (II) pour lesquels -R₃-R₄-R₅- représente une chaîne de formule -CH₂-CF₂-CH₂- peuvent être obtenus par réduction d'un dérivé de formule (II) correspondant pour lequel -R₃-R₄-R₅- représente une chaîne de formule -CH₂-CF₂-CH(OH)-.

Cette réaction s'effectue généralement au moyen de triéthylsilane et d'acide trifluoroacétique, à une température voisine de 20°C.

Les dérivés de formule (II) pour lesquels -R₃-R₄-R₅- représente une chaîne de formula -CH₂-CF₂-CH(OH)- ou -CH₂-CH(R₁₃)-S dans laquelle R₁₃ représente un radical alkyle peuvent être obtenus par réduction d'un dérivé de formule : 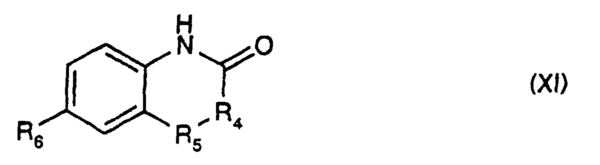 dans laquelle R₆ a les mêmes significations que dans la formule (I), -R₄-R₅-représente un radical -CF₂-CH(OH)- ou -CH(alk)-S et alk représente un radical alkyle.

Cette réaction s'effectue généralement au moyen du complexe borane-méthylsulfure, au sein du toluène, à la température d'ébullition du milieu réactionnel.

Les dérivés de formule (XI) peuvent être obtenus selon les schémas réactionnels suivants : 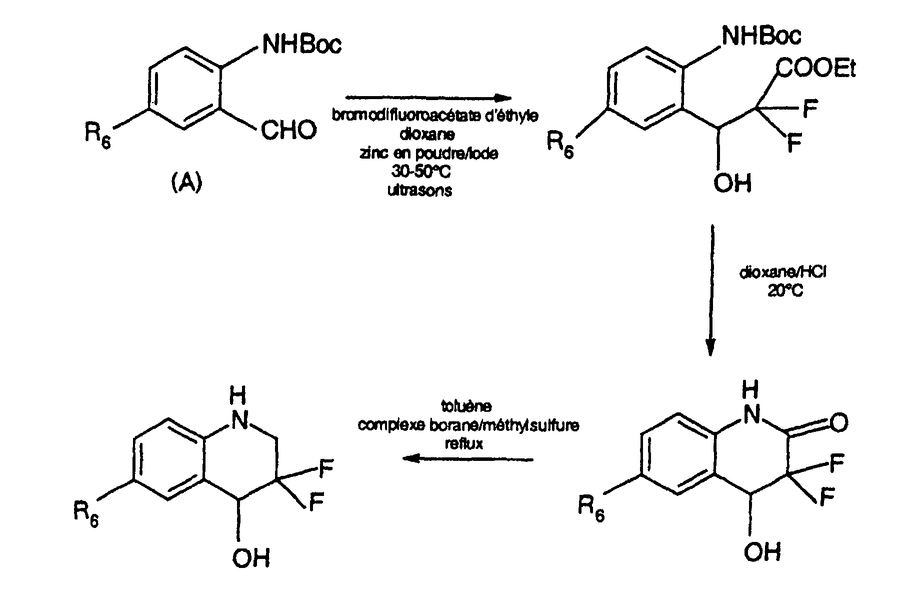 dans ces formule R₆ a les mêmes significations que dans la formule (I), Et représente éthyle et Boc représente un radical tert-butoxycarbonyle, 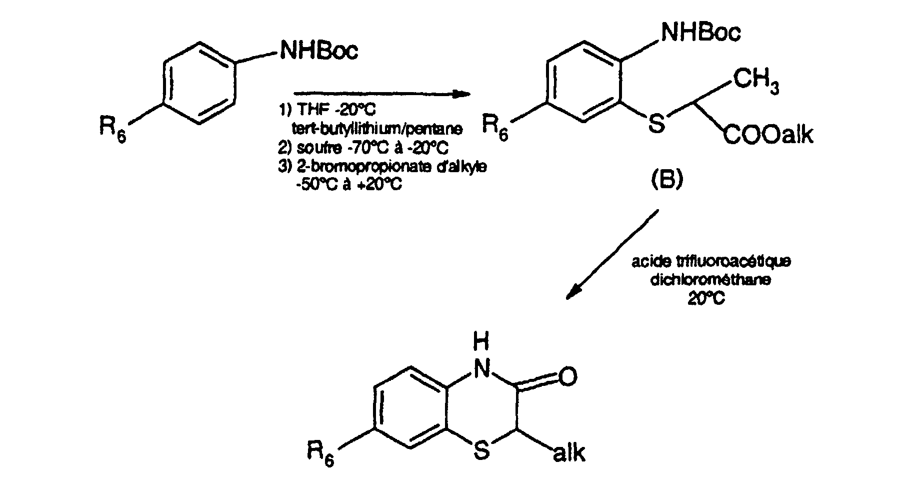 dans ces formule R₆ a les mêmes significations que dans la formule (I), alk représente un radical alkyle et Boc représente un radical tert-butoxycarbonyle.

Les aldéhydes (A) peuvent être obtenus par application ou adaptation de la méthode décrite dans Chem. Abstr., 107, 39815x.

Les dérivés de formule (II) pour lesquels -R₃-R₄-R₅- représente une chaîne -CH₂-CH(R₁₃)-S- ou -CH₂-CH(R₉)-CH₂- pour lesquels R₁₃ et R₉ représentent un radical -CH₂OH peuvent être obtenus par réduction d'un dérivé de formule: 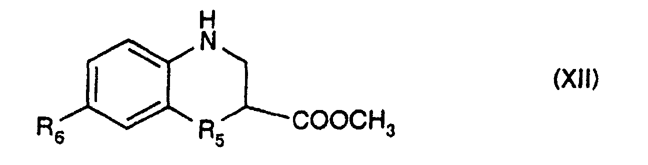 dans laquelle R₆ a les mêmes significations que dans la formule (I) et R₅ représente un atome de soufre ou un radical -CH₂-.

Cette réaction s'effectue de préférence au moyen du complexe borane-diméthylsulfure, au sein d'un solvant inerte tel que le toluène ou le tétrahydrofuranne, à la température d'ébullition du milieu réactionnel.

Les dérivés de formule (XII) peuvent être obtenus selon les schémas réactionnels suivants : 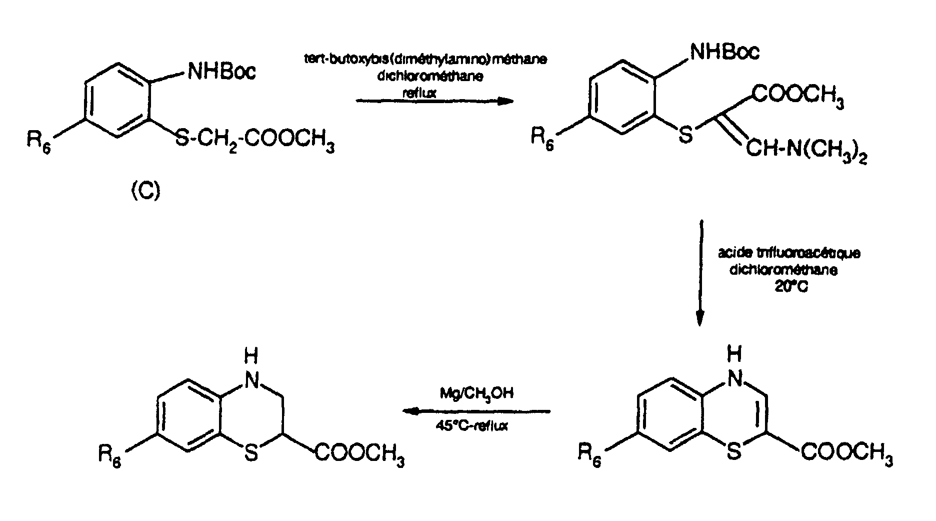 dans ces formules R₆ a les mêmes significations que dans la formule (I) et Boc représente un radical tert-butoxycarbonyle. 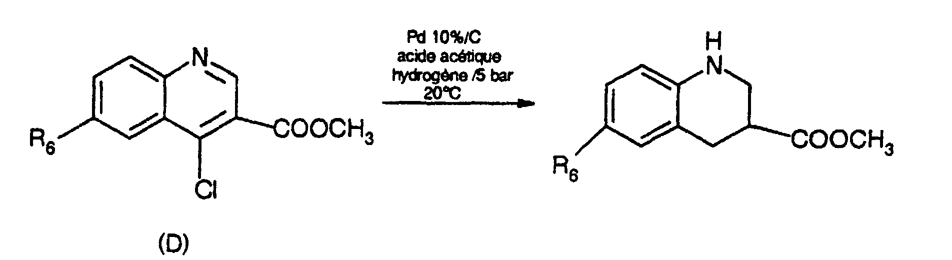 dans ces formules R₆ a les mêmes significations que dans la formule (I).

Les dérivés de formule (c) peuvent être obtenus comme mentionné précédemment pour les dérivés de formule (B) en utilisant un bromoacétate d'alkyle au lieu d'un 2-bromopropionate d'alkyle.

Les dérivés de formule (D) peuvent être préparés par application ou adaptation de la méthode décrite dans J. Med. Chem., 22 (7), 816-823 (1979).

Il est entendu pour l'homme de métier que, pour la mise en oeuvre des procédés selon l'invention décrits précédemment, il peut être nécessaire d'introduire des groupes protecteurs des fonctions amino afin d'éviter des réactions secondaires. Notamment, on opère selon les méthodes décrites par T.W. Greene, Protective Groups in Organic Synthesis, A. Wiley Interscience Publication (1981), ou par Mc Omie, Protective Groups in Organic Chemistry, Plenum Press (1973). Les fonctions amino peuvent par exemple être protégées par des radicaux phtalimido, méthoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués ou sous forme de carbamates de tert-butyle ou de méthyle puis régénérées au moyen d'acide trifluoroacétique ou d'acide chlorhydrique dans le tétrahydrofuranne ou de carbamates de benzyle puis régénérées par hydrogénation après avoir mis en oeuvre le procédé selon l'invention.

Les mélanges réactionnels obtenus par les divers procédés décrits précédemment sont traités suivant des méthodes classiques physiques (évaporation, extraction, distillation, chromatographie, cristallisation par exemple) ou chimiques (formation de sels par exemple).

Les énantiomères des composés de formule (I) contenant au moins un site asymétrique peuvent être obtenus par synthèse à partir des précurseurs chiraux ou par dédoublement des racémiques par exemple par chromatographie sur colonne chirale selon W. H. PIRKLE et coll., asymetric synthesis, vol 1, Academic Press (1983).

Les composés de formule (I) sous forme de base libre peuvent être éventuellement transformés en sels d'addition avec un acide minéral ou organique, par action d'un tel acide au sein d'un solvant organique tel qu'un alcool, une cétone, un éther ou un solvant chloré.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate.

Les composés de formule (I) présentent des propriétés pharmacologiques intéressantes. Ces composés sont des anticonvulsivants et interfèrent avec la transmission glutamatergique et sont donc utiles pour traiter ou prévenir toutes les ischémies (telles l'ischémie focale ou globale) consécutives à des accidents vasculaires cérébraux tels que le stroke thromboembolique et hémorragique, un arrêt cardiaque, une hypotension artérielle, une intervention chirurgicale cardiaque, vasculaire ou pulmonaire ou une hypoglycémie sévére. Ils sont également utiles dans le traitement des effets dus à une anoxie, qu'elle soit périnatale ou consécutive à une noyade, une haute pression ou à des lésions cérébro-spinales. Ces composés peuvent également être utilisés pour traiter ou prévenir l'évolution de maladies neurodégénératives, de la chorée d'HUNTINGTON, de la maladie d'ALZHEIMER et autres démences, de la sclérose latérale amyotrophique ou d'autres maladies du motoneurone, de l'atrophie olivo-pontocérébelleuse et de la maladie de PARKINSON. Ces composés peuvent aussi être utilisés vis-à-vis des manifestations épileptogènes et/ou convulsives, pour le traitement des traumatismes cérébraux ou spinaux, des traumatismes liés à la dégénérescence de l'oreille interne (R. PUJOL et coll., Neuroreport, 3, 299-302 (1992) ou de la rétine (J.L. MONSINGER et coll., Exp. Neurol., 113, 10-17 (1991), du tinnitus, de l'anxiété (KEHNE et coll., Eur. J. Pharmacol., 193, 283 (1991)), de la dépression (TRULLAS et coll.,Eur. J. Pharmacol., 185, 1 (1990)), de la schizophrénie (REYNOLDS, TIPS, 13, 116 (1992)), du syndrome de TOURETTE, des encéphalopathies hépatiques, des troubles du sommeil, des désordres du déficit attentionnel, des troubles des conditions hormonales (excès de la sécrétion de HG ou HL, sécrétion de corticostérone), en tant qu'analgésiques (DICKENSON et coll., Neurosc. Letters, 121, 263 (1991)), antiinflammatoires (SLUTA et coll., Neurosci. Letters, 149, 99-102 (1993)) antianorexiques (SORRELS et coll., Brain Res., 572, 265 (1992)), antimigraineux, antiémétiques et pour traiter les empoisonnements par des neurotoxines ou d'autres substances agonistes du récepteur NMDA ou AMPA, ainsi que les troubles neurologiques associés aux maladies virales telles que les méningites et encéphalites virales, le SIDA (LIPTON et coll., Neuron, 7, 111 (1991)), la rage, la rougeole et le tétanos (BAGETTA et coll., Br. J. Pharmacol., 101, 776 (1990)). Ces composés sont aussi utiles pour la prévention, la tolérance et la dépendance des symptômes d'abstinence aux drogues, à l'alcool et de l'inhibition de l'accoutumance et de la dépendance aux opiacés, barbituriques, amphétamine et benzodiazépines. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

L'activité de ces produits comme anticonvulsivant a été déterminée chez la souris selon la méthode de l'électrochoc supra maximal. Des souris blanches CD1 sont traitées intraveineusement avec les composés à tester en milieu salin, 10 minutes avant d'être soumis à un choc électrique (75mA; durée 0,04 seconde) au moyen d'électrodes occulaires. Normalement ce choc produit une convulsion tonique chez la souris non traitée, caractérisée par une extension des membres. Si la convulsion tonique ne survient pas, l'animal est considéré protégé. Dans ce test, les composés de formule (I) présentent une DE50 égale ou inférieure à 6 mg/kg.

L'activité de ces produits comme antiglutamate a été déterminée sur les convulsions induites par le glutamate selon une technique inspirée de celle de I. P. LAPIN, J. Neural. Transmission, 54, 229-238 (1982); l'injection du glutamate par voie intracérébroventriculaire étant effectuée selon une technique inspirée de celle de R. CHERMAT et P. SIMON, J. Pharmacol. (Paris), 6, 489-492 (1975). Leur DE₅₀ est inférieure à 10 mg/kg.

Les composés de formule (I) présentent une toxicité faible. Leur DL₅₀ est supérieure à 15 mg/kg par voie IV chez la souris.

Pour l'emploi médicinal, il peut être fait usage des composés de formule (I) tels quels ou à l'état de sels pharmaceutiquement acceptables, c'est-à-dire non toxiques aux doses d'utilisation.

Sont particulièrement intéressants, les composés pour lesquels
R₁ représente un atome de soufre ou de sélénium
R₂ représente un atome d'hydrogène ou un radical alkyle,
-R₃-R₄-R₅- représente une chaîne de formule -CH₂-CH₂-CH₂-, -CH(R₇)-CH₂-CH₂-, -CH₂-CF₂-CH(OH)-, -CH₂-CH₂-CH(R₈)-, -CH₂-CH₂-CO, -CH₂-CH₂-S-, -CH₂-CH₂-SO-, -CH₂-CH₂-SO₂-,
R₆ représente un radical polyfluoroalkyle, polyfluoroalcoxy ou polyfluoroalkylthio,
R₇ représente un radical alkyle, -CH₂OH ou -CH₂NR₁₁R₁₂
R₈ représente un radical -NR₁₁R₁₂, -SO₂-alk-, -SO-alk- ou phényle,
R₁₁ représente un atome d'hydrogène ou un radical alkyle ou acyle,
R₁₂ représente un atome d'hydrogène ou un radical alkyle,
ou bien R₁₁ et R₁₂ forment avec l'atome d'azote auquel ils sont rattachés un hétérocycle à 5 ou 6 chaînons, saturé ou insaturé et comportant éventuellement un autre hétéroatome choisi parmi azote, oxygène ou soufre.

Parmi les composés particulièrement intéressants on peut citer les composés suivants :
-2-imino-8-trifluorométhoxy-5,6-dihydro-2H,4H-thiazolo[5,4,3-ij]quinoléine,
-2-imino-8-trifluorométhyl-5,6-dihydro-2H,4H-thiazolo[5,4,3-ij]quinoléine,
- (R,S)-2-imino-4-méthyl-8-trifluorométhyl-5,6-dihydro-2H,4H-thiazolo[5,4,3-ij]quinoléine et ses énantiomères,
-2-imino-8-trifluorométhoxy-2H,4H-thiazolo[5,4,3-ij]quinoléine-6-one,
- 2-imino-8-trifluorométhoxy-4,5-dihydro-2H-thiazolo-[3,4,5-de][1,4]benzothiazine-6,6-dioxyde,
- (R,S)-2-imino-8-trifluorométhoxy-4,5-dihydro-2H-thiazolo-[3,4,5-de][1,4]benzothiazine-6-oxyde et ses énantiomères,
- (R,S)-2-imino-8-trifluorométhyl-4,5-dihydro-2H-thiazolo[3,4,5-de][1,4]benzothiazine-6-oxyde et ses énantiomères,
- 2-imino-8-trifluorométhyl-4,5-dihydro-2H-thiazolo[3,4,5-de][1,4]benzothiazine,
- (R,S)-2-imino-6-phényl-8-trifluorométhyl-5,6-dihydro-2H,4H-thiazolo[5,4,3-ij]quinoléine et ses énantioméres,
- 2-imino-8-trifluorométhoxy-4,5-dihydro-2H-thiazolo[3,4,5-d,e]-[1,4]-benzoxazine,
- (R,S)-2-imino-8-trifluorométhyl-5,6-dihydro-2H,4H-thiazolo[5,4,3-ij]quinoléine-4-méthanol et ses énantiomères,
- (R,S)-5,5-difluoro-6-hydroxy-2-imino-8-trifluorométhyl-5,6-dihydro-2H,4H-thiazolo[5,4,3-ij]quinoléine et ses énantiomères,
- (R,S)-éthylméthyl(2-imino-8-trifluorométhyl-5,6-dihydro-2H,4H-thiazolo[5,4,3-ij]quinol-4-ylméthyl)amine et ses énantiomères,
- 2-imino-8-trifluorométhyl-5,6-dihydro-2H,4H-sélénazolo[5,4,3-ij]quinoléine
- (R,S)-2-imino-6-éthylsulfinyl-8-triflucrométhyl-5,6-dihydro-2H,4H-thiazolo[5,4,3-ij]quinoléine et ses énantiomères,
- (R,S)-2-imino-6-éthylsulfonyl-8-trifluorométhyl-5,6-dihydro-2H,4H-thiazolo[5,4,3-ij]quinoléine et ses énantiomères
et leurs sels.

Les exemples suivants illustrent l'invention sans la limiter.

### EXEMPLE 1

On ajoute goutte à goutte en 10 minutes, à une température voisine de 20°C, 1,6 g de brome dilué dans 5 ml d'acide acétique à une solution de 3,9 g de thiocyanate de potassium et de 2,1 g de 6-trifluorométhoxy-1,2,3,4-tétrahydroquinoléine dans 20 ml d'acide acétique. Le mélange réactionnel est agité pendant 20 heures à la même température, versé sur de la glace, alcalinisé par une solution d'ammoniaque à 20% et extrait par trois fois 100 ml d'acétate d'éthyle. Les phases organiques sont réunies, lavées par 100 ml d'eau distillée, séchées sur sulfate de magnésium et concentrées à sec sous pression réduite (2 kPa) à 40°C. Le produit brut est chromatographié sur colonne de gel de silice en éluant avec de l'acétate d'éthyle et conduit à 0,6 g d'un solide blanc. Ce dernier est redissous dans le minimum d'éthanol, puis additionné d'un excès d'isopropanol chlorhydrique (environ 5 N). On obtient ainsi, après recristallisation dans l'éthanol, 0,52 g de chlorhydrate de 2-imino-8-trifluorométhoxy-5,6-dihydro-2H,4H-thiazolo[5,4,3-ij]quinoléine sous forme d'un solide blanc fondant au-dessus de 260°C avec décomposition [RMN Spectre ¹H dans DMSO-d6, T=300K, δ en ppm (250 Mhz) : 2,15 (2H, m, CH₂), 2,95 (2H, t, J=6Hz, CH₂-aryl), 4,15 (2H, t, J=6Hz, NCH₂), 7,45 (1H, s, CH arom.), 8,00 (1H, s, CH arom.), 10,50 (2H, s large, NH,HCl)].

La 6-trifluorométhoxy-1,2,3,4-tétrahydroquinoléine peut être préparée de la manière suivante : 2,13 g de 6-trifluorométhoxyquinoléine avec 0,4 g d'oxyde de platine dans 25 ml de méthanol sont hydrogénés à une température voisine de 20°C sous une pression de 5 à 3 bar pendant 1,5 heure. Après filtration du milieu réactionnel, la phase organique est concentrée sous pression réduite (2 kPa) à 40°C, pour conduire à une huile légèrement brune. Celle-ci est dissoute dans un excès d'isopropanol chlorhydrique (environ 5 N), puis à nouveau concentrée sous vide pour fournir 1 g de chlorhydrate de 6-trifluorométhoxy-1,2,3,4-tétrahydroquinoléine sous forme d'un solide blanc fondant à 145°C avec décomposition. Par traitement du chlorhydrate avec une solution diluée d'hydroxyde de sodium et extraction à l'acétate d'éthyle on obtient la base sous la forme d'une huile.

La 6-trifluorométhoxyquinoléine peut être préparée de la manière suivante : on chauffe à une température voisine de 150°C pendant 1 heure 20 minutes un mélange de 17,7 g de 4-trifluorométhoxyaniline, 33,7 g de 3-nitrobenzènesulfonate de sodium, 6 g de sulfate de fer heptahydraté, 10 g d'acide borique, 250 ml de glycérol et 60 ml d'acide sulfurique concentré. Le mélange réactionnel est ensuite versé sur de la glace, alcalinisé par une solution concentrée d'hydroxyde de sodium, puis extrait par trois fois 300 ml de dichlorométhane. La phase organique est lavée par 100 ml d'eau, séchée sur sulfate de magnésium puis concentrée sous pression réduite (2 kPa). Le résidu d'évaporation est chromatographié sur colonne de gel de silice en éluant avec un mélange de cyclohexane et d'acétate d'éthyle (70-30 en volumes) pour fournir 15 g de 6-trifluorométhoxyquinoléine, sous forme d'une huile claire [RMN Spectre ¹H dans CDCl3, T=300K, δ en ppm (200 MHz) : 7,40 (1H, dd, J=3 et 7Hz, CH arom.), 7,55 (1H, d, J=8Hz, CH arom.), 7,60 (1H, s, CH arom.), 8,10 (1H, dd, J=7 et 1Hz, CH arom.), 8,12 (1H, d, J=8Hz, CH arom.), 8,90 (1H, dd, J=3 et 1Hz, CH arom.)].

### EXEMPLE 2

On opère comme à l'exemple 1 mais à partir de 3,5 g de brome dilué dans 10 ml d'acide acétique, 5 g de thiocyanate de potassium et 4,4 g de 6-trifluorométhyl-1,2,3,4-tétrahydroquinoléine dans 40 ml d'acide acétique. On obtient ainsi 2,8 g de chlorhydrate de 2-imino-8-trifluorométhyl-5,6-dihydro-2H,4H-thiazolo[5,4,3-ij]quinoléine sous forme d'un solide blanc fondant au-dessus de 260°C avec décomposition [RMN Spectre ¹H dans DMSO-d6, T=300K, δ en ppm (300 Mhz) : 2,20 (2H, m, CH₂), 3,00 (2H, t, J=6Hz, CH₂-aryl), 4,15 (2H, t, J=6Hz, NCH₂), 7,75 (1H, s, CH arom.), 8,30 (1H, s, CH arom.), 10,80 (2H, s large, NH,HCl).].

La 6-trifluorométhyl-1,2,3,4-tétrahydroquinoléine peut être préparée comme décrit dans J. Chem. Soc. Perkin Trans., 1, (9), 1933-9 (1980).

### EXEMPLE 3

On opère comme à l'exemple 1 mais à partir de 1,6 g de brome dans 10 ml d'acide acétique, 2,3 g de thiocyanate de potassium et 2,1 g de (R,S)-2-méthyl-6-trifluorométhyl-1,2,3,4-tétrahydroquinoléine dans 25 ml d'acide acétique. Le résidu obtenu est chromatographié sur colonne de gel de silice en éluant avec un mélange de cyclohexane et d'acétate d'éthyle (50-50 en volumes) et conduit à une huile qui est dissoute dans l'éthanol et auquel on additionne une solution d'isopropanol chlorhydrique (environ 5 N) en léger excès. La solution est concentrée sous pression réduite et les cristaux de chlorhydrate obtenus sont recristallisés dans l'éthanol contenant des traces d'acétate d'éthyle. On obtient ainsi 0,9 g de chlorhydrate de (R,S)-2-imino-4-méthyl-8-trifluorométhyl-5,6-dihydro-2H,4H-thiazolo[5,4,3-ij]quinoléine fondant vers 180°C avec décomposition [RMN Spectre ¹H dans DMSO-d6, T=300K, δ en ppm (300 MHz) : 1,35 (3H, d, J=6Hz, CH₃), 2,20 (2H, m, CH₂), 3,05 (2H, m, CH₂-aryl), 4,95 (1H, m, NCH), 7,80 (1H, s, CH arom.), 8,30 (1H, s, CH arom.), 10,90 (2H, s large, NH,HCI)].

La (R,S)-2-méthyl-6-trifluorométhyl-1,2,3,4-tétrahydroquinoléine peut être préparée en opérant comme dans l'exemple 1 mais à partir de 2,1 g de 2-méthyl-6-trifluorométhylquinoléine et 0,2 g d'oxyde de platine dans 15 ml de tétrahydrofuranne. L'hydrogénation est effectuée sous une pression de 10 à 5 bar pendant 30 minutes On obtient 2,1 g de (R,S)-2-méthyl-6-trifluorométhyl-1,2,3,4-tétrahydroquinoléine sous forme d'une huile claire [RMN Spectre ¹H dans DMSO-d6, T=300K, δ en ppm (300 MHz) : 1,20 (3H, d, J=6Hz, CH₃), 1,35 et 1,90 (1H chacun, m, CH₂), 2,75 (2H, m, CH₂-aryl), 3,35 (1H,m, NCH), 6,35 (1H, s, NH), 6,55 (1H, d, J=8Hz, CH arom.), 7,15 (2H, m, 2 CH arom.)].

La 2-méthyl-6-trifluorométhylquinoléine peut être préparée comme décrit dans J. Pharm. Sci., 68(3), 336-8 (1979).

### EXEMPLE 4

On opère comme à l'exemple 1, mais à partir de 0,67 g de brome dans 2 ml d'acide acétique, 0,94 g de thiocyanate de potassium et 0,9 g de l'isomère lévogyre de la (R,S)-2-méthyl-6-trifluorométhyl-1,2,3,4-tétrahydroquinoléine dans 10 ml d'acide acétique. On obtient ainsi 0,8 g du chlorhydrate de l'isomère dextrogyre de la (R,S)-2-imino-4-méthyl-8-trifluorométhyl-5,6-dihydro-2H,4H-thiazolo[5,4,3-ij]quinoléine se décomposant au dessus de 200°C; [α]_{D}²⁰=+39,0 ±0,7° (c=0,5; éthanol); [Analyse C12H12CIF3N2S, % calculé C: 46,68, H: 3,92, Cl : 11,48, F: 18,46, N : 9,07, S : 10,39, % trouvé C : 46,3, H : 4,3, Cl : 11,4, F : 18,1, N : 9,1, S : 10,2].

L'isomère lévogyre de la (R,S)-2-méthyl-6-trifluorométhyl-1,2,3,4-tétrahydroquinoléine peut être préparé de la manière suivante : 11 g de (R,S)-2-méthyl-6-trifluorométhyl-1,2,3,4-tétrahydroquinoléine sont traités par 17,5 g de l'acide 2,3-di-O-benzoyl-L-tartrique dans le minimum d'acétate d'éthyle vers 50°C, puis la solution abandonnée à température voisine de 20°C fournit un solide. Celui-ci est recristallisé une fois dans l'acétate d'éthyle et ensuite deux fois dans le méthanol aqueux à 60% pour donner 2,9 g de cristaux blancs fondant vers 142°C; [α]_{D}²⁰ = -104,1 ± 1,5° (c = 0,5; éthanol). Le sel ainsi obtenu est trituré dans 30 ml de solution 1 N d'hydroxyde de sodium et la base libérée est extraite par deux fois 50 ml d'éther éthylique. La phase organique est lavée par 10 ml d'eau distillée, puis séchée sur sulfate de magnésium et concentrée sous pression réduite pour fournir 0,9 g de l'isomère lévogyre de la (R,S)-2-méthyl-6-trifluorométhyl-1,2,3,4-tétrahydroquinoléine sous forme d'une huile claire; [α]_{D}²⁰ = -62,2 ±1,0° (c=0,5; éthanol).

### EXEMPLE 5

On opère comme à l'exemple 1, mais à partir de 0,81 g de brome dans 2 ml d'acide acétique, 1,14 g de thiocyanate de potassium et 1,1 g de l'isomère dextrogyre de la 2-méthyl-6-trifluorométhyl-1,2,3,4-tétrahydroquinoléine dans 10 ml d'acide acétique. On obtient ainsi 1,0 g du chlorhydrate de l'isomère lévogyre de la (R,S)-2-imino-4-méthyl-8-trifluorométhyl-5,6-dihydro-2H,4H-thiazolo[5,4,3-ij]quinoléine se décomposant au dessus de 200°C; [α]_{D}²⁰=-39,4 ± 0,7° (c=0,5; éthanol) [Analyse C12H12ClF3N2S, % calculé C : 46,68, H : 3,92, Cl : 11,48, F : 18,46, N : 9,07, S : 10,39, % trouvé C : 46,5, H: 3,9, Cl : 11,7, F : 18,2, N : 9,0, S : 10,5].

L'isomère dextrogyre de la (R,S)-2-méthyl-6-trifluorométhyl-1,2,3,4-tétrahydroquinoléine peut être préparé de la manière suivante : les filtrats de cristallisation du 2,3-di-O-benzoyl-L-tartrate de l'isomère lévogyre de (R,S)-2-méthyl-6-trifluorométhyl-1,2,3,4-tétrahydroquinoléine de l'exemple 4 sont concentrés sous pression réduite. Par traitement du résidu d'évaporation obtenu par une solution diluée d'hydroxyde de sodium et extraction à l'éther éthylique, on obtient 5,7 g de base, sous forme d'une huile. Ceux-ci sont traités par 10,5 g de l'acide 2,3-di-O-benzoyl-D-tartrique dans le minimum d'acétate d'éthyle vers 50°C, puis la solution abandonnée à température voisine de 20°C fournit un solide. Celui-ci est recristallisé une fois dans l'acétate d'éthyle et ensuite deux fois dans le méthanol aqueux à 60% pour donner 3,3 g de cristaux blancs fondant vers 145°C; [α]_{D}²⁰ = +94.6 ± 1,4° (c=0,5; éthanol). Le sel ainsi obtenu est trituré dans 30 ml de solution 1 N d'hydroxyde de sodium et la base libérée est extraite par deux fois 50 ml d'éther éthylique. La phase organique est lavée par 10 ml d'eau distillée, puis séchée sur sulfate de magnésium et concentrée sous pression réduite pour fournir 1,1 g de l'isomère dextrogyre de la (R,S)-2-méthyl-6-trifluorométhyl-1,2,3,4-tétrahydroquinoléine sous forme d'une huile claire; [α]_{D}²⁰ = +62,9 ± 1,2° (c=0,5; éthanol).

### EXEMPLE 6

On ajoute goutte à goutte en 10 minutes, à une température voisine de 20°C, 1,2 g de brome dilué dans 5 ml d'acide acétique à une solution de 1,7 g de thiocyanate de potassium et 2,2 g de (R,S)-2-méthylsulfonylméthyl-6-trifluorométhoxy-1,2,3,4-tétrahydroquinoléine dans 20 ml d'acide acétique. Le mélange réactionnel est agité pendant 20 heures à la même température,versé sur de la glace, alcalinisé par une solution d'ammoniaque à 20% et extrait par deux fois 150 ml d'acétate d'éthyle. Les phases organiques sont réunies, lavées par 100 ml d'eau distillée, séchées sur sulfate de magnésium et concentrées à sec sous pression réduite (2 kPa) à 40°C. Le solide obtenu est purifié par trituration dans l'éther éthylique, puis recristallisé dans l'acétate d'éthyle. On obtient ainsi 1,2 g de (R,S)-2-imino-4-méthylsulfonylméthyl-8-trifluorométhoxy-5,6-dihydro-2H,4H-thiazolo[5,4,3-ij]quinoléine sous forme de cristaux jaunes fondant à 195°C [RMN Spectre ¹H dans DMSO-d6, T=300K, δ en ppm (300 MHz) : 1,95 et 2,50 (1H chacun, m, CH₂), 2,80 et 3,00 (1H chacun, m, CH₂-aryl), 3,15 (3H, s, CH₃), 3,50 (2H, m, SCH₂), 4,90 (1H, m, NCH), 7,10 (1H, s, CH arom.), 7,45 (1H, s, CH arom.), 8,60 (1H, s, NH)].

La (R,S)-2-méthylsulfonylméthyl-6-trifluorométhoxy-1,2,3,4-tétrahydroquinoléine peut être préparée en opérant comme dans l'exemple 1, mais à partir de 2,3 g de 2-méthylsulfonylméthyl-6-trifluorométhoxyquinoléine, 0,4 g d'oxyde de platine dans 25 ml d'un mélange méthanol-tétrahydrofuranne (50-50 en volumes). On obtient ainsi 2,2 g de (R,S)-2-méthylsulfonylméthyl-6-trifluorométhoxy-1,2,3,4-tétrahydroquinoléine sous forme d'un solide blanc fondant à 103°C.

La 2-méthylsulfonylméthyl-6-trifluorométhoxyquinoléine peut être préparée de la manière suivante : à une solution de 4,6 g de 6-trifluorométhoxyquinoléine-1-oxyde dans 3,8 ml d'anhydride acétique on ajoute la solution de 2,7 g de 1-(méthylsulfonyl)propan-2-one dans 10 ml d'éther diméthylique de l'éthylèneglycol, agite à température voisine de 20°C pendant 20 heures, puis vers 60°C pendant 20 heures. Le milieu réactionnel est ensuite additionné de 5 ml de méthanol et porté à reflux pendant 1 heure. Il est alors versé sur de la glace puis alcalinisé par une solution concentrée d'hydroxyde de sodium. Le précipité brun qui apparait est extrait par 2 fois 150 ml d'acétate d'éthyle. La phase organique est séchée sur sulfate de magnésium et concentrée sous pression réduite (2 kPa) à 40°C, fournissant une huile brune qui cristallise rapidement. Les cristaux sont triturés dans 10 ml d'éthanol et on obtient ainsi 2,3 g de 2-méthylsulfonylméthyl-6-trifluorométhoxyquinoléine sous forme d'un solide blanc fondant à 143°C.

Le 6-trifluorométhoxyquinoléine-1-oxyde peut être préparé de la manière suivante : à une solution de 8,9 g de 6-trifluorométhoxyquinoléine dans 200 ml de dichlorométhane, maintenue à température voisine de 20°C, est ajouté de l'acide 3-chloro-perbenzoïque en deux fois (7,2 g au départ et 3 g après 20 heures). Le milieu réactionnel est agité encore 4 heures après la deuxième addition puis concentré sous pression réduite (2 kPa) à 20°C. Le résidu obtenu est purifié par chromatographie sur colonne de gel de silice en éluant par de l'acétate d'éthyle puis par un mélange d'acétate d'éthyle et de méthanol (80-20 en volumes). On obtient ainsi 8,8 g de 1-oxyde de 6-trifluorométhoxyquinoléine sous forme d'une huile brun-clair, qui cristallise en un solide fondant à 72°C.

### EXEMPLE 7

On opère comme à l'exemple 1 mais à partir de 0,37 g de brome dilué dans 2 ml d'acide acétique, 0,52 g de thiocyanate de potassium et 0,6 g de (R,S)-6-trifluorométhoxy-1,2,3,4-tétrahydroquinoléine-2-méthanol dans 7 ml d'acide acétique. Après recristallisation dans l'éthanol contenant des traces d'éther de pétrole on obtient 0,27 g de chlorhydrate de (R,S)-2-imino-8-trifluorométhoxy-5,6-dihydro-2H,4H-thiazolo[5,4,3-ij]quinoléine-4-méthanol, fondant à 132°C avec décomposition [RMN Spectre ¹H dans DMSO-d6, T=300K, δ en ppm (300 MHz) : 2,00 et 2,35 (1H chacun, m, CH₂), 2,90 et 3,05 (1H chacun, m, CH₂-aryl), 3,65 et 3,75 (1H chacun, m, OCH₂), 4,78 (1H, m, NCH), 5,30 (1H, s large, OH), 7,40 (1H, s, CH arom.), 7,95 (1H, s, CH arom.)].

Le (R,S)-6-trifluorométhoxy-1,2,3,4-tétrahydroquinoléine-2-méthanol peut être préparé comme à l'exemple 1, mais à partir de 1,5 g de 2-hydroxyméthyl-6-trifluorométhoxyquinoléine et 0,3 g d'oxyde de platine dans 15 ml de méthanol. On obtient 1,4 g de (R,S)-6-trifluorométhoxy-1,2,3,4-tétrahydroquinoléine-2-méthanol sous forme d'une huile claire, qui cristallise en un solide fondant à 77°C.

Le 6-trifluorométhoxyquinoléine-2-méthanol peut être préparé de la manière suivante : on chauffe à l'ébullition pendant 3 heures une solution de 9,2 g de 2-méthyl-6-trifluorométhoxyquinoléine-1-oxyde dans 35 ml d'anhydride acétique, puis concentre sous pression réduite. L'huile obtenue est dissoute dans 50 ml de dioxanne, puis sont ajoutés 30 ml d'eau distillée et une solution concentrée d'hydroxyde de sodium (3 ml en plus de la quantité nécessaire à la neutralisation). Le milieu réactionnel est porté 1 heure à reflux, puis concentré à sec. Le résidu obtenu est extrait par 400 ml d'acétate d'éthyle, la phase organique est lavée par 50 ml d'eau distillée puis séchée sur sulfate de magnésium et concentrée sous pression réduite (2 kPa) à 40°C. Le résidu obtenu est chromatographié sur colonne de gel de silice en éluant avec un mélange de cyclohexane et d'acétate d'éthyle (50-50 en volumes). On obtient ainsi 5,7 g de 6-trifluorométhoxyquinoléine-2-méthanol sous forme d'un solide blanchâtre fondant à 75°C.

Le 2-méthyl-6-trifluorométhoxyquinoléine-1-oxyde peut être préparé de la manière suivante : à la solution maintenue à température voisine de 20°C de 2,8 g de 2-méthyl-6-trifluorométhoxyquinoléine dans 50 ml de dichlorométhane est ajouté de l'acide 3-chloroperbenzoïque en deux fois (2,8 g au départ et 0,3 g après 1 heure). Le milieu réactionnel est agité encore 19 heures après la deuxième addition, puis est lavé avec une solution diluée d'hydrogénocarbonate de sodium. La phase organique est ensuite séchée sur sulfate de magnésium et concentrée sous pression réduite (2 kPa) à 20°C. Après trituration du résidu obtenu dans l'éther de pétrole, on obtient 2,5 g de 2-méthyl-6-trifluorométhoxyquinoléine-1-oxyde sous forme de cristaux blanchâtres fondant à 105°C.

La 2-méthyl-6-trifluorométhoxyquinoléine peut être préparée de la manière suivante : on porte à reflux une suspension de 70,8 g de 4-trifluorométhoxyaniline, 100 ml d'acide chlorhydrique concentré, 99 g de 2,3,5,6-tétrachloro-1,4-benzoquinone dans 100 ml de n-butanol et on ajoute goutte à goutte en 3 heures une solution de 34 g de crotonaldéhyde dans 40 ml de n-butanol, puis poursuit l'ébullition 20 minutes et laisse revenir à température voisine de 20°C. Les produits neutres sont extraits par quatre fois 400 ml d'éther éthylique et éliminés, puis la phase aqueuse est alcalinisée par 120 ml de solution concentrée d'hydroxyde de sodium. L'huile apparue est extraite par trois fois 500 ml d'éther éthylique, la phase organique est ensuite lavée par 100 ml d'eau distillée, puis séchée sur sulfate de magnésium et concentrée sous pression réduite (2 kPa) à 40°C. Le résidu obtenu est chromatographié sur colonne de gel de silice en éluant avec un mélange de cyclohexane et d'acétate d'éthyle (50-50 en volumes), puis est distillé à 100°C sous 0,7 kPa pour conduire à 52,5 g de 2-méthyl-6-trifluorométhoxyquinoléine sous forme d'un solide jaune-verdâtre fondant vers 43°C [RMN Spectre ¹H dans DMSO-d6, T=300K, δ en ppm (250 MHz) : 2,70 (3H, s, CH₃), 7,55 (1H, d, J=8Hz, CH en 3), 7,72 (1H, dd, J=8 et 2Hz, CH en 7), 8,01 (1H, s, CH en 5), 8,08 (1H, d, J=8Hz, CH en 8), 8,40 (1H, d, J=8Hz, CH en 4)].

### EXEMPLE 8

On opère comme à l'exemple 1 mais à partir de 2,2 g de brome dans 10 ml d'acide acétique, 3,09 g de thiocyanate de potassium et 3,2 g 6-trifluorométhoxy-2,3-dihydro-(1H)-quinoléine-4-one dans 35 ml d'acide acétique. La phase organique est extraite par 170 ml d'une solution aqueuse d'acide chlorhydrique environ 0,1 N. La phase aqueuse chlorhydrique est alcalinisée par 4 ml d'une solution de soude 10 N et extraite par trois fois 100ml d'acétate d'éthyle. Les extraits organiques sont réunis, lavés par 100 ml d'eau distillée, séchés sur sulfate de magnésium et concentrés à sec sous pression réduite (2 kPa) à 40°C. Le produit brut obtenu est chromatographié sur gel de silice en éluant avec un mélange d'acétate d'éthyle et d'éther de pétrole (80-20 en volumes). Le solide jaune isolé (0,3 g) est lavé par une solution froide d'éther de pétrole et d'éther isopropylique (60-40 en volumes), puis est séché sous pression réduite (70 Pa) à 40°C. On obtient ainsi 0,25 g de 2-imino-8-trifluorométhoxy-2H,4H-thiazolo[5,4,3-ij]quinoléine-6-one sous forme d'une poudre jaune fondant à 192°C [Analyse C11H7F3N2O2S, % calculé C : 45,84, H : 2,45, F : 19,77, N : 9,72, O : 11,1, S : 11,12, % trouvé C : 45,5, H : 2,1, F : 19,4, N : 9,3, S : 11,1].

La 6-trifluorométhoxy-2,3-dihydro-(1H)-quinoléine-4-one peut être préparée de la manière suivante : à une solution de 3,4 g de 4-oxo-6-trifluorométhoxy-3,4-dihydro-(2H)-quinoléine-1-carboxylate de *tert*-butyle dans 60 ml de dichlorométhane sont ajoutés 10 ml d'acide trifluoroacétique. Après 3 heures à température voisine de 20°C le milieu réactionnel est concentré à sec sous pression réduite. Le résidu est traité par une solution diluée d'hydrogénocarbonate de sodium et extrait par de l'éther éthylique. La phase organique est lavée par de l'eau distillée, séchée sur sulfate de magnésium et concentrée à sec sous pression réduite (2,2 kPa) à 50°C. On obtient 2,1 g d'un solide ocre qui est chromatographié sur gel de silice en éluant avec un mélange de dichlorométhane et d'éther de pétrole (70-30 en volumes). On obtient ainsi 1,75 g de 6-trifluorométhoxy-2,3-dihydro-(1H)-quinoléine-4-one sous forme d'un solide jaune vif fondant à 118°C.

Le 4-oxo-6-trifluorométhoxy-3,4-dihydro-(2H)-quinoléine-1-carboxylate de *tert*-butyle peut être préparé de la manière suivante : à une solution agitée de 11,6 g de 6-trifluorométhoxy-3,4-dihydro-(2H)-quinoléine-1-carboxylate de *tert*-butyle dans 120 ml de dichlorométhane sont additionnés une première fois 18,5 ml d'une solution aqueuse à 70% d'hydroperoxyde de *tert*-butyle et 0,095 g d'anhydride chromique. Après 4 heures à température voisine de 20°C on ajoute une nouvelle fois 18,5 ml d'une solution aqueuse à 70% d'hydroperoxyde de *tert*-butyle et 0,095 g d'anhydride chromique. Pour compléter la réaction on rajoute après 18 heures 10 ml d'une solution aqueuse à 70% d'hydroperoxyde de tertiobutyle et 60 mg d'anhydride chromique puis abandonne le mélange réactionnel pendant 24 heures. On ajoute alors à froid une solution de dithionite de sodium jusqu'à fin de dégagement gazeux. La phase organique est lavée par de l'eau distillée et par une solution aqueuse saturée de chlorure de sodium, séchée sur sulfate de magnésium puis concentrée à sec sous pression réduite (2 kPa) à 40°C. L'huile rouge obtenue est filtrée sur gel de silice en éluant avec de l'éther de pétrole. On obtient ainsi 8,45 g de 4-oxo-6-trifluorométhoxy-3,4-dihydro-(2H)-quinoléine-1-carboxylate de *tert*-butyle sous forme d'une poudre jaune fondant à 66°C.

Le 6-trifluorométhoxy-3,4-dihydro-2H-quinoléine-1-carboxylate de *tert*-butyle peut être préparé de la manière suivante : on porte et maintient à l'ébullition pendant 16 heures une solution de 11 g de 6-trifluorométhoxy-1,2,3,4-tétrahydroquinoléine et 11,8 g de dicarbonate de di-tert-butyle dans 110 ml de tétrahydrofuranne. Pour compléter la réaction, on rajoute 1,9 g de dicarbonate de di-tert-butyle et poursuit l'ébullition pendant 4 heures puis concentre sous pression réduite. Le résidu obtenu est extrait par 300 ml d'éther éthylique, la phase organique est lavée par 50 ml d'eau distillée, séchée sur sulfate de magnésium et concentrée sous pression réduite (2kPa) à 20°C. Après trituration du résidu dans l'éther de pétrole, on obtient 9,2 g de 6-trifluorométhoxy-3,4-dihydro-2H-quinoléine-1-carboxylate de *tert*-butyle sous forme de cristaux blancs fondant à 68°C.

### EXEMPLE 9

A une solution, sous argon et refroidie à 5°C, de 5,3 g d'acide 3-chloroperbenzoïque (pureté 80%) dans 100 ml de dichlorométhane, on ajoute 3,35 g de 2-imino-8-trifluorométhoxy-4,5-dihydro-2H-thiazolo[3,4,5-de][1,4]benzothiazine et on laisse agiter 16 heures à une température voisine de 20°C. On ajoute ensuite 100 ml d'une solution aqueuse 1 M d'hydrogénocarbonate de sodium et on agite 1 heure à la même température. Après séparation des deux phases, on extrait la phase aqueuse deux fois par 50 ml de dichlorométhane et les extraits organiques réunis sont séchés sur sulfate de magnésium et concentrés à sec sous pression réduite (2 kPa). L'huile jaune obtenue est chromatographiée sous pression d'azote (150 kPa) sur 20 g de gel de silice 20-45 µm contenus dans une colonne de 2 cm de diamètre, en éluant avec de l'acétate d'éthyle. Le produit obtenu (730 mg) est dissous dans 50 ml d'éthanol absolu, puis on ajoute 100 µl d'acide méthanesulfonique. Après 1 heure 45 minutes d'agitation, le précipité est séparé par filtration, lavé à l'éthanol, puis à l'éther éthylique et séché sous pression réduite (2 kPa) à 20°C. On obtient ainsi 750 mg de méthanesulfonate de 6,6-dioxyde de 2-imino-8-trifluorométhoxy-4,5-dihydro-2H-thiazolo-[3,4,5-de][1,4]benzothiazine, sous forme d'un solide blanc fondant à une température supérieure à 260°C [Analyse C11H11F3N2O6S3, % calculé C : 31,43, H : 2,64, F : 13,56, N : 6,66, S : 22,88, % trouvé C: 31,39, H : 2,29, F : 13,28, N : 6,59, S : 22,60].

### EXEMPLE 10

A une solution, sous argon et refroidie à -10°C, de 4,5 g d'acide 3-chloroperbenzoïque (pureté 80%) dans 150 ml de dichlorométhane, on ajoute 5 g de 2-imino-8-trifluorométhoxy-4,5-dihydro-2H-thiazolo[3,4,5-de][1,4]benzothiazine et on agite pendant 1 heure à une température voisine de 20°C. Le mélange est filtré et concentré à sec sous pression réduite (2 kPa). Le solide jaune obtenu est chromatographié sous pression d'azote (150 kPa) sur 60 g de gel de silice 20-45 µm contenus dans une colonne de 2,5 cm de diamètre, en éluant avec un mélange d'acétate d'éthyle et de méthanol (80-20 en volumes). Le produit obtenu (3,3 g) est dissous dans 200 ml d'éthanol absolu, puis on ajoute 0,8 ml d'acide méthanesulfonique. Après 5 heures d'agitation à 20°C, la solution est concentrée à sec sous pression réduite (2 kPa). Le produit obtenu est mis en suspension dans de l'éther éthylique, séparé par filtration, lavé avec de l'éther éthylique et séché sous pression réduite (2 kPa) à 20°C. On obtient ainsi 3,9 g de méthanesulfonate de (R,S)-2-imino-8-trifluorométhoxy-4,5-dihydro-2H-thiazolo-[3,4,5-de][1,4]benzothiazine-6-oxyde, sous forme d'un solide jaune pâle fondant à 268°C [Analyse C11H11F3N2O5S3, % calculé C : 32,67, H : 2,74, F : 14,09, N : 6,93, O : 19,78, S : 23,79, % trouvé C : 32,87, H : 2,39, F : 14,46, N : 6,91, S : 23,49].

### EXEMPLE 11

On opère comme dans l'exemple 1 mais en utilisant 0,33 ml de brome dilué dans 5 ml d'acide acétique, 1,5 g de 7-trifluorométhoxy-3,4-dihydro-2H-[1,4]benzothiazine et 1,3 g de thiocyanate de potassium dans 20 ml d'acide acétique. On obtient ainsi 1 g de chlorhydrate de 2-imino-8-trifluorométhoxy-4,5-dihydro-2H-thiazolo[3,4,5-de][1,4]benzothiazine, sous forme d'une poudre jaune fondant à 245°C [Analyse C10H8ClF3N2OS2, % calculé C : 36,53, H : 2,45, Cl : 10,78, F : 17,34, N : 8,52, O : 4,87, S : 19,51, % trouvé C : 36,59, H : 2,18, Cl : 10,48, F : 16,94, N : 8,63, S : 19,15].

La 7-trifluorométhoxy-3,4-dihydro-2H-[1,4]benzothiazine peut être préparée de la manière suivante : à 19 ml d'une solution environ 0,5 M de tétrahydroaluminate de lithium dans le tétrahydrofuranne, maintenue sous argon à 5°C, on ajoute goutte à goutte en 15 minutes, une solution de 2 g de 7-trifluorométhoxy-1H-[1,4]benzothiazine-3-one dans 15 ml de tétrahydrofuranne anhydre. Le mélange réactionnel est agité ensuite 2 heures à 20°C. L'insoluble est séparé par filtrations et lavé avec du tétrahydrofuranne. Le filtrat dilué avec de l'acétate d'éthyle est lavé à l'eau distillée, séché sur sulfate de magnésium et concentré à sec sous pression réduite (2KPa). On obtient ainsi 1,7 g de 7-trifluorométhoxy-3,4-dihydro-2H-[1,4]benzothiazine sous forme d'une huile ambrée [RMN Spectre ¹H (CDCl₃), T=300K, δ en ppm (300 MHz) : 3,05 (2H, m, SCH₂); 3,65 (2H, m, NCH₂); 4,05 (1H, s large, NH); 6,40 (1H, d, J=8Hz, CH arom.); 6,75 (1H, d, J=8Hz, CH arom.); 6,90 (1H, s, CH arom.)].

La 7-trifluorométhoxy-1H-[1,4]benzothiazine-3-one peut être préparée de la manière suivante : à une suspension de 23 g de 2-amino-6-trifluorométhoxybenzothiazole dans 230 ml d'eau distillée, on ajoute 140 g de potasse en pastilles par portions d'environ 10 g. Le mélange est ensuite agité pendant 16 heures à une température voisine de 20°C, puis pendant 5 heures au reflux. Après refroidissement vers 20°C, on ajoute 30 g de bromoacétate de méthyle puis 30 ml d'eau distillée et le milieu est agité pendant 16 heures à la même température, puis filtré. Le filtrat est ensuite acidifié avec de l'acide chlorhydrique concentré à une température voisine de 5°C. Le précipité apparu est séparé par filtration, lavé à l'eau distillée et séché sous courant d'air vers 20°C. On obtient ainsi 20 g de 7-trifluorométhoxy-1H-[1,4]benzothiazine-3-one sous forme d'un solide blanchâtre fondant à 185°C.

Le 2-amino-6-trifluorométhoxybenzothiazole peut être préparé par la méthode décrite par L.M. YAGUPOL'SKII et coll., Zh. Obshch. Khim., 33 (7), 2301 (1963).

### EXEMPLE 12

A une solution, maintenue à -10°C, de 500 mg de 2-imino-8-trifluorométhyl-4,5-dihydro-2H-thiazolo[3,4,5-de][1,4]benzothiazine dans 15 ml de dichlorométhane, on ajoute 475 mg au total d'acide 3-chloroperbenzoïque (pureté 80%) en trois fois sur une période de 5 heures. Le mélange est ensuite agité pendant 1 heure à une température voisine de 20°C et concentré à sec sous pression réduite (2 kPa). Le produit obtenu est chromatographié sous pression d'azote (150 kPa) sur 25 g de gel de silice 20-45 µm contenus dans une colonne de 2 cm de diamètre, en éluant d'abord avec de l'acétate d'éthyle puis avec un mélange d'acétate d'éthyle et de méthanol (80-20 en volumes). Le produit obtenu (350 mg) est dissous dans 5 ml d'éthanol absolu auxquels on ajoute 95 µl d'acide méthanesulfonique. Après 16 heures d'agitation à 20°C, le précipité apparu est recristallisé dans l'éthanol. On obtient ainsi 366 mg de méthanesulfonate de (R,S)-2-imino-8-trifluorométhyl-4,5-dihydro-2H-thiazolo[3,4,5-de][1,4]benzothiazine-6-oxyde sous forme d'un solide blanc cassé fondant à 236°C [Analyse C11H11F3N2O4S3, % calculé C : 34,02, H: 2,85, F : 14,67, N : 7,21, O : 16,48, S : 24,77, % trouvé C : 33,7, H : 2,7, F : 14,3, N : 7,00, S : 24,9].

### EXEMPLE 13

On opère comme à l'exemple 1 mais à partir de 2,2 g de brome dans 10 ml d'acide acétique, 3 g de 7-trifluorométhyl-3,4-dihydro-2H-[1,4]benzothiazine et 2,9 g de thiocyanate de potassium dans 30 ml d'acide acétique. Le produit obtenu (3,74 g) est chromatographié sous pression d'azote (150 kPa) sur 50 g de gel de silice 20-45 µm contenus dans une colonne de 3 cm de diamètre, en éluant avec un mélange d'acétate d'éthyle et de cyclohexane (50-50 en volumes). Le produit obtenu est mis en suspension dans de l'éther de pétrole, séparé par filtration, lavé par de l'éther de pétrole et séché sous pression réduite. On dissout 0,3 g du produit obtenu (sur 1,8 g obtenu au total) dans 5 ml d'éthanol auxquels on ajoute 0,07 ml d'acide méthanesulfonique. Après 1 heure d'agitation à 20°C, le produit est recristallisé dans l'éthanol absolu. On obtient ainsi 0,29 g de méthanesulfonate de 2-imino-8-trifluorométhyl-4,5-dihydro-2H-thiazolo[3,4,5-de][1,4]benzothiazine sous forme d'un solide blanc fondant à 262°C [Analyse C11H11F3N2O3S3, % calculé C : 35,48, H : 2,98, F : 15,30, N : 7,52, O : 12,89, S : 25,83, % trouvé C : 35,4, H : 2,9, F : 15,1, N : 7,5, S : 26,1].

La 7-trifluorométhyl-3,4-dihydro-2H-[1,4]benzothiazine peut être préparée de la manière suivante : on opère comme à l'exemple 14, mais en utilisant 16 ml d'une solution de tétrahydroaluminate de lithium (environ 0,3 M) dans le tétrahydrofuranne, 930 mg de 7-trifluorométhyl-1H-[1,4]benzothiazine-3-one dans 10 ml de tétrahydrofuranne anhydre. Le mélange réactionnel est agité ensuite 16 heures à 20°C, additionné lentement de 2 ml d'eau distillée, et agité 1 heure à une température voisine de 20°C. L'insoluble est séparé par filtration, lavé avec de l'acétate d'éthyle et éliminé. Le filtrat et le lavage sont réunis, lavés à l'eau distillée, séchés sur du sulfate de magnésium et concentrés à sec sous pression réduite (2 kPa). On obtient ainsi 410 mg de 7-trifluorométhyl-3,4-dihydro-2H-[1,4]benzothiazine, sous forme d'une huile jaune [RMN Spectre ¹H dans DMSO-d6, T=300K, δ en ppm (300 MHz) : 3,00 (2H, m, SCH₂); 3,60 (2H, m, NCH₂); 6,65 (1H, d, J=8Hz, CH arom.); 6,85 (1H, s, NH); 7,05 (1H, d, J=8Hz, CH arom.); 7,08 (1H, s, CH arom.)].

La 7-trifluorométhyl-1H-[1,4]benzothiazine-3-one peut être préparée de la manière suivante : on agite pendant 2 heures à une température voisine de 20°C, un mélange de 7,9 g de (5-trifluorométhyl-2-*tert*-butoxycarbonylamino phénylsulfanyl) acétate de méthyle et de 12 ml d'acide trifluoroacétique dans 15 ml de dichlorométhane. Le mélange est ensuite concentré à sec sous pression réduite (2 kPa). Le produit obtenu est mis en suspension dans de l'éther isopropylique, filtré, rincé avec le même solvant, essoré, séché sous pression réduite (2 kPa) à 20°C. On obtient ainsi 3,5 g de 7-trifluorométhyl-1H-[1,4]benzothiazine-3-one sous forme d'un solide beige clair fondant à 168°C.

Le (5-trifluorométhyl-2-*tert*-butoxycarbonylaminophényl)sulfanyl acétate de méthyle peut être préparé de la manière suivante : à une solution de 20 g de 4-trifluorométhylphénylcarbamate de *tert*-butyle dans 300 ml de tétrahydrofuranne anhydre, maintenue sous une atmosphère d'argon à une température inférieure à -20°C, on ajoute goutte à goutte, en 30 minutes, 102 ml d'une solution (environ 1,5 M) de *tert*-butyllithium dans le pentane. Le mélange est agité pendant 3 heures 15 minutes à la même température, puis on ajoute 2,5 g de soufre à -30°C. Le mélange est agité pendant 1,5 heure en laissant remonter progressivement la température à 20°C, additionné de 10,8 g de bromoacétate de méthyle, et agité pendant 16 heures à une température voisine de 20°C. Après addition d'eau distillée, le mélange est extrait deux fois avec de l'acétate d'éthyle. Les extraits organiques sont réunis, séchés sur sulfate de magnésium, et concentrés à sec sous pression réduite (2 kPa). Le produit obtenu (29,5 g) est chromatographié sous pression d'azote (150 kPa) sur 400 g de gel de silice 20-45 µm contenus dans une colonne de 6 cm de diamètre, en éluant avec un mélange de cyclohexane et d'acétate d'éthyle (90-10 en volumes). On obtient ainsi 6,2 g de (5-trifluorométhyl-2-*tert*-butoxycarbonylaminophényl)sultanyl acétate de méthyle sous forme d'une huile ambrée [RMN Spectre ¹H dans DMSO-d6, T=300K, δ en ppm (200 MHz) : 1,50 (9H, s, C(CH₃)₃ ; 3,60 (3H, s, OCH₃); 3,90 (2H, s, SCH₂CO); 7,65 (1H, dd, J=2 et 8Hz, CH arom.); 7,84 (1H, d, J=2Hz, CH arom.); 7,87 (1H, d, J=8Hz, CH arom.); 8,70 (1H, s, NH)].

Le 4-trifluorométhylphénylcarbamate de *tert*-butyle peut être préparé de la manière suivante : on coule, en 10 minutes et à 0°C, une solution de 163 g de di-*tert*-butyldicarbonate dans 100 ml de tétrahydrofuranne anhydre sur une solution de 96 g de 4-trifluorométhylaniline dans 750 ml de tétrahydrofuranne anhydre. Le milieu réactionnel est agité à 80°C pendant 3 heures, puis concentré à sec. On obtient un produit qui est redissous dans 300 ml d'acétate d'éthyle. La solution est lavée trois fois par de l'eau distillée, séchée sur sulfate de magnésium et concentrée à sec. On obtient ainsi 136 g de 4-trifluorométhylphénylcarbamate de *tert*-butyle sous forme d'un solide blanc fondant à 121°C.

### EXEMPLE 14

Une solution de 0,96 g de brome dans 10 ml d'acide acétique est ajoutée goutte à goutte, en 10 minutes à une température inférieure à 30°C, à une solution de 1,7 g de (R,S)-4-phényl-6-trifluorométhyl-1,2,3,4-tétrahydroquinoléine et de 1,3 g de thiocyanate de potassium dans 40 ml d'acide acétique. Le mélange est agité pendant 2 heures à une température voisine de 20°C, versé sur 16 g de glace, additionné de 18 ml d'ammoniaque environ à 28% et de 25 ml d'acétate d'éthyle puis filtré. Après décantation, la phase aqueuse est extraite 2 fois avec 50 ml au total d'acétate d'éthyle et les extraits organiques sont réunis, séchés sur du sulfate de magnésium et concentrés à sec sous pression réduite (2,2 kPa) à 60°C. Le produit obtenu (4,3 g) est chromatographié sous pression d'azote (150 kPa) sur 835 g de gel de silice neutre 20-45 µm contenus dans une colonne de 6,8 cm de diamètre en éluant avec un mélange de dichlorométhane et de méthanol (98-2 en volumes). Le produit obtenu (1,1 g) est dissous dans 3 ml d'éthanol et, après addition de 5 ml d'éther chlorhydrique 3,8N et conservation pendant 1 heure à une température voisine de 5°C, l'insoluble est séparé par filtration, lavé 2 fois avec 20 ml au total d'éther éthylique et séché sous pression réduite (0,13 kPa) à 60°C. On obtient ainsi 0,8 g de chlorhydrate de (R,S)-2-imino-6-phényl-8-trifluorométhyl-5,6-dihydro-2H,4H-thiazolo[5,4,3-ij]quinoléine sous la forme d'un solide jaune fondant au-dessus de 260°C avec décomposition [Spectre ¹H dans DMSO-d6, T=300K, δ en ppm (300 MHz) : 2,40 (2H, m, CH₂), 4,05 et 4,20 (1H chacun, m, NCH₂), 4,45 (1H, t, J=6Hz, NCH), 7,20 (3H, m, 3 CH arom.), 7,40 (3H, m, 3 CH arom.), 8,30 (1H, s, CH arom.), 10,50 (2H, s large, NH,HCI)].

La (R,S)-4-phényl-6-trifluorométhyl-1,2,3,4-tétrahydroquinoléine peut être préparée de la manière suivante : 4,7 g de tétrachlorure d'étain sont ajoutés goutte à goutte en 5 minutes à -78°C à une suspension de 6,6 g de N-paratoluènesulfonylméthyl-4-trifluorométhylaniline dans 100 ml de dichlorométhane. Le mélange est agité pendant 10 minutes et une solution de 2,1 g de styrène dans 50 ml de dichlorométhane est ajoutée goutte à goutte en 30 minutes à là même température. Le mélange est agité pendant 1 heure à -78°C et pendant 15 heures à une température voisine de 20°C puis il est neutralisé avec 100 ml d'une solution aqueuse saturée d'hydrogénocarbonate de sodium. Après addition de 50 ml d'eau distillée et de 250 ml de dichlorométhane, la phase aqueuse est extraite 2 fois avec 100 ml au total de dichlorométhane. Les extraits organiques sont réunis, lavés 2 fois avec 100 ml d'eau distillée, séchés sur du sulfate de magnésium et concentrés à sec sous pression réduite (2,2 kPa) à 60°C. Le produit obtenu (5,6 g) est chromatographié sous pression d'azote (150 kPa) sur 1390 g de gel de silice neutre 20-45 µm contenus dans une colonne de 6,8 cm de diamètre en éluant avec un mélange de cyclohexane et d'acétate d'éthyle (85-15 en volumes). On obtient ainsi 3,3 g de (R,S)-4-phényl-6-trifluorométhyl-1,2,3,4-tétrahydroquinoléine sous la forme d'une huile jaune [RMN Spectre ¹H dans DMSO-d6, T=300K, δ en ppm (300 MHz) : 2,00 (2H, m, CH₂), 3,05 et 3,25 (1H chacun, m, NCH₂), 4,15 (1H, t, J=6Hz, CH-Ph), 6,65 (2H, m, CH arom. et NH), 6,85 (1H, s, CH arom.), 7,10 (2H, d, J=7Hz, 2CH arom.), 7,25 (2H, t, J=7Hz, 2 CH arom), 7,30 (2H, t, J=7Hz, 2 CH arom.)].

La N-para-toluènesulfonylméthyl-4-trifluorométhylaniline peut être préparée de la manière suivante : 26,75 g d'une solution aqueuse à 37% de formaldéhyde sont ajoutés à une solution de 53,4 g de para-toluènesulfinate de sodium dans 600 ml d'eau distillée. Le mélange est agité et une solution de 48,3 g de trifluorométhylaniline dans 900 ml d'une solution aqueuse 0,033 N d'acide chlorhydrique est ajoutée goutte à goutte en 20 minutes à une température inférieure à 30°C. Le mélange est agité pendant 15 heures à une température voisine de 20°C et l'insoluble apparu est séparé par filtration, lavé 2 fois avec 500 ml au total d'eau distillée. Le produit humide obtenu (161,2 g) est dissous dans 900 ml de méthanol bouillant et, après refroidissement et conservation pendant une heure à une température voisine de 5°C, les cristaux apparus sont séparés par filtration, lavés 2 fois avec 600 ml au total de méthanol refroidi à 5°C et séchés sous pression réduite (0,13 kPa) à 50°C. On obtient ainsi 75,9 g de N-para-toluènesulfonylméthyl-4-trifluorométhylaniline fondant à135°C.

### EXEMPLE 15

Une solution de 0,62 g de brome dans 3 ml d'acide acétique est ajoutée goutte à goutte, en 10 minutes à une température inférieure à 30°C, à une solution de 1,06 g de (R,S)-4-(N-méthyl-acétamido)-6-trifluorométhyl-1,2,3,4-tétrahydroquinoléine et de 0,83 g de thiocyanate de potassium dans 15 ml d'acide acétique. Le mélange est agité pendant 16 heures à une température voisine de 20°C, versé sur 20 g de glace et 20 ml d'eau distillée et extrait 2 fois avec 60 ml au total d'acétate d'éthyle. Les extraits organiques sont éliminés et la phase aqueuse est alcalinisée avec 20 ml d'ammoniaque à 28% puis extraite 3 fois avec 45 ml au total d'acétate d'éthyle. Les extraits organiques sont réunis, séchés sur du sulfate de magnésium et concentrés à sec sous pression réduite (2,2 kPa) à 60°C. Le produit obtenu (0,44 g) est dissous dans 1 ml d'éthanol et, après addition de 5 ml d'éther chlorhydrique 1,5 N, le mélange est conservé pendant 1 heure à une température voisine de 5°C. L'insoluble apparu est séparé par filtration, lavé 2 fois avec 10 ml au total d'éther éthylique et séché sous pression réduite (0,13 kPa) à 60°C. On obtient ainsi 0,4 g de chlorhydrate de (R,S)-N-(2-imino-8-trifluorométhyl-5,6-dihydro-2H,4H-thiazolo[5,4,3-ij]quinol-6-yl)-N-méthyl-acétamide sous la forme d'un solide crème fondant à 200°C avec décomposition.

La (R,S)-4-(N-méthyl-acétamido)-6-trifluorométhyl-1,2,3,4-tétrahydro-quinoléine peut être préparée de la manière suivante : 3,25 g de tétrachlorure d'étain sont ajoutés goutte à goutte en 5 minutes à -78°C à une suspension de 1,65 g de N-para-toluènesulfonylméthyl-4-trifluorométhyl-aniline dans 25 ml de dichlorométhane. Le mélange est agité pendant 10 minutes et une solution de 0,5 g de N-méthyl-N-vinyl acétamide dans 15 ml de dichlorométhane est ajoutée goutte à goutte en 10 minutes à là même température. Le mélange est agité pendant 1 heure à -78°C et pendant 15 heures à une température voisine de 20°C. L'insoluble apparu est séparé par filtration, lavé 2 fois avec 20 ml au total de dichlorométhane, mis en suspension dans 20 ml d'eau distillée et le mélange agité est neutralisé avec 60 ml d'une solution aqueuse saturée d'hydrogénocarbonate de sodium et extrait 3 fois avec 70 ml au total de dichlorométhane. Les extraits organiques sont réunis, lavés 2 fois avec 20 ml au total d'eau distillée, séchés sur du sulfate de magnésium et concentrés à sec sous pression réduite (2,2 kPa) à 60°C. On obtient ainsi 1,06 g de (R,S)-4-(N-méthyl-acétamido)-6-trifluorométhyl-1,2,3,4-tétrahydroquinoléine sous la forme d'une laque jaune [RMN Spectre ¹H dans DMSO-d6, T=393K, δ en ppm (400 MHz) : 2,00 (2H, m, CH₂), 2,15 (3H, s, COCH₃), 2,70 (3H, s, NCH₃), 3,40 (2H, m, NCH₂), 5,50 (1H, s large, NCH), 6,70 (1H, d, J=8Hz, CH arom.), 7,00 (1H, d, J=2Hz, CH arom.), 7,25 (1H, dd, J=8 et 2Hz, CH arom.)].

### EXEMPLE 16

Une solution de 0,86 g de brome dans 3,5 ml d'acide acétique est ajoutée goutte à goutte, en 10 minutes à une température inférieure à 30°C, à une solution de 1,25 g de (R,S)-6-trifluorométhyl-1,2,3,4-tétrahydroquinoléine-4-méthanol et de 1,15 g de thiocyanate de potassium dans 20 ml d'acide acétique. Le mélange est agité pendant 16 heures à une température voisine de 20°C, versé sur 30 g de glace et 30 ml d'eau distillée et extrait avec 40 ml de dichlorométhane. L' extrait organique est éliminé et la phase aqueuse est alcalinisée avec 30 ml d'ammoniaque à 28% puis extraite 3 fois avec 60 ml au total de dichlorométhane. Les extraits organiques sont réunis, séchés sur du sulfate de magnésium et concentrés à sec sous pression réduite (2,2 kPa) à 60°C. Le produit obtenu (1,09 g) est dissous dans 10 ml de méthanol et, après addition de 15 ml d'éther chlorhydrique 1,2 N, le mélange est conservé pendant 1 heure à une température voisine de 5°C. L'insoluble apparu est séparé par filtration, lavé 2 fois avec 10 ml au total d'éther éthylique et séché sous pression réduite (0,13 kPa) à 60°C. 0,56 g du produit obtenu (sur le 0,9 g obtenu au total) est mis en suspension dans 56 ml d'eau distillée à 50°C et, après addition de noir décolorant et filtration, le filtrat est concentré à sec sous pression réduite (0,13 kPa) à 60°C. Le produit obtenu (0,46 g) est mis en suspension dans 5 ml d'acétone, filtré, lavé 2 fois avec 6 ml au total d'acétone et séché sous pression réduite (0,13 kPa) à 60°C. On obtient ainsi 0,41 g de chlorhydrate de (R,S)-(2-imino-8-trifluorométhyl-5,6-dihydro-2H,4H-thiazolo[5,4,3-ij]quinol-6-yl)méthanol sous la forme d'un solide écru fondant au-dessus de 260°C avec décomposition [Spectre dans DMSO-d6, T=300K, δ en ppm (250 MHz) : 2,20 (2H, m, CH₂), 3,20 (1H, t, J=6Hz, CH), 3,70 (2H, m, OCH₂), 4,10 et 4,30 (1H chacun, m, NCH₂), 5,20 (1H, t, J=6 Hz, OH), 7,85 (1H, s, CH arom.), 8,30 (1H, s, CH arom), 10,80 (2H, s, NH,HCl)].

Le (R,S)-6-trifluorométhyl-1,2,3,4-tétrahydroquinoléine-4-méthanol peut être préparé de la manière suivante : 16,25 g de tétrachlorure d'étain sont ajoutés goutte à goutte en 5 minutes à -78°C à une suspension de 8,2 g de N-paratoluènesulfonylméthyl-4-trifluorométhylaniline dans 125 ml de dichlorométhane. Le mélange est agité pendant 10 minutes et une solution de 3,6 g d'alcool allylique dans 75 ml de dichlorométhane est ajoutée goutte à goutte en 15 minutes à la même température. Le mélange est agité pendant 2 heures à -78°C et pendant 80 heures à une température voisine de 20°C puis neutralisé avec 500 ml d'une solution aqueuse saturée d'hydrogéno-carbonate de sodium. Après addition de 125 ml d'eau distillée, de 125 ml de dichlorométhane, filtration et décantation, la phase aqueuse est extraite 2 fois avec 250 ml au total de dichlorométhane. Les extraits organiques sont réunis, lavés 2 fois avec 250 ml au total d'eau distillée, séchés sur du sulfate de magnésium et concentrés à sec sous pression réduite (2 kPa) à 60°C. Le produit obtenu (5,6 g) est chromatographié sous pression d'azote (150 kPa) sur 380 g de gel de silice neutre 20-45 µm contenus dans une colonne de 6,4 cm de diamètre en éluant avec un mélange de cyclohexane et d'acétate d'éthyle (50-50 en volumes). On obtient ainsi 1,25 g de (R,S)-6-trifluorométhyl-1,2,3,4-tétrahydroquinoléine-4-méthanol sous la forme d'une huile jaune [RMN Spectre ¹H dans DMSO-d6, T=300K, δ en ppm (300 MHz) : 1,65 et 1,95 (1H chacun, m, CH₂), 2,80 (1H, m, CH), 3,20 (2H, m, NCH₂), 3,50 (2H, m, OCH₂), 4,80 (1H, t, J=6Hz, OH), 6,50 (1H, s, NH), 6,55 (1H, d, J=8Hz, CH arom.), 7,15 (1H, dd, J=8 et 2Hz, CH arom.), 7,25 (1H, s, CH arom.)].

### EXEMPLE 17

Une solution de 3,38 g de (R,S)-N-(2-imino-8-trifluorométhyl-5,6-dihydro-2H,4H-thiazolo[5,4,3-ij]quinol-6-yl)-N-méthyl acétamide dans 340 ml d'une solution aqueuse 2 N d'acide chlorhydrique est chauffée à l'ébullition pendant 5 heures, conservée pendant 75 heures à une température voisine de 20°C et concentrée à sec sous pression réduite (2,2 kPa) à 100°C. Une suspension de 1 g de produit obtenu (sur les 3,56 g obtenus au total) dans 60 ml d'acide acétique est chauffée à l'ébullition pendant 15 minutes, refroidie et conservée pendant 1 heure à une température voisine de 20°C. L'insoluble est séparé par filtration, lavé 2 fois avec 6 ml au total d'acide acétique, 2 fois avec 10 ml au total d'éther éthylique et séché sous pression réduite (0,13 kPa) à 60°C. On obtient ainsi 0,74 g de dichlorhydrate de (R,S)-N-méthyl(2-imino-8-trifluorométhyl-5,6-dihydro-2H,4H-thiazolo[5,4,3-ij]quinol-6-yl)amine sous la forme d'un solide blanc fondant au-dessus de 260°C avec décomposition [Spectre dans DMSO-d6, T=300K, δ en ppm (250 MHz) : 2,45 et 2,80 (1H chacun, m, CH₂), 2,65 (3H, s, NCH₃), 4,40 (2H, m, NCH₂), 4,80 (1H, m, NCH), 8,20 (1H, s, CH arom.), 8,55 (1H, s, CH arom), 10,10 (2H, s, NH,HCI), 11,20 (2H, s large, NH,HCI)].

### EXEMPLE 18

Une solution de 0,4 g de brome dans 5 ml d'acide acétique est ajoutée goutte à goutte, en 5 minutes à une température voisine de 25°C, à une solution de 0,68 g de (R,S)-4-(1-pyrrolidinyl)-6-trifluorométhyl-1,2,3,4-tétrahydroquinoléine et de 0,53 g de thiocyanate de potassium dans 15 ml d'acide acétique. Le mélange est agité pendant 4 heures à une température voisine de 20°C, versé sur un mélange de 40 g de glace et de 40 ml d'eau distillée, alcalinisé avec 35 ml d'ammoniaque à 28% et extrait 3 fois avec 150 ml au total de dichlorométhane. Les extraits organiques sont réunis, lavés avec 50 ml d'eau distillée, séchés sur du sulfate de magnésium et concentrés à sec sous pression réduite (2 kPa) à 30°C. Le produit obtenu (0,68 g) est dissous dans 10 ml d'éthanol et la solution, additionnée de noir décolorant est agitée pendant 5 minutes et filtrée. Le filtrat, additionné de 5 ml d'éther chlorhydrique anhydre, est conservé pendant 1 heure à une température voisine de 5°C puis le solide apparu est séparé par filtration, lavé 2 fois avec 10 ml au total d'éther anhydre et séché sous pression réduite (0,13 kPa) à 60°C. On obtient ainsi 0,29 g de (R,S)-2-imino-6-(1-pyrrolidinyl)-8-trifluorométhyl-5,6-dihydro-2H,4H-thiazolo[5,4,3-ij]quinoléine sous la forme d'une poudre jaune pâle fondant à 223°C avec décomposition.

La (R,S)-4-(1-pyrrolidinyl)-6-trifluorométhyl-1,2,3,4-tétrahydroquinoléine peut être préparée de la manière suivante : 11 ml d'une solution à 2 mol par litre dans le tétrahydrofuranne du complexe borane-sulfure de méthyle est ajoutée goutte à goutte en 5 minutes à une température voisine de 20°C à une solution maintenue sous atmosphère d'argon de 1,42 g de (R,S)-4-(2-oxopyrrolidin-1-yl)-6-trifluorométhyl-1,2,3,4-tétrahydroquinoléine dans 10 ml de tétrahydrofuranne anhydre. Le mélange est agité pendant 20 heures à la même température, additionné lentement de 20 ml d'eau distillée, agité pendant 15 minutes et extrait 4 fois avec 50 ml au total de dichlorométhane. Les extraits organiques sont réunis, lavés 2 fois avec 20 ml au total d'eau distillée, séchés sur du sulfate de magnésium et concentrés à sec sous pression réduite (2 kPa) à 30°C. Le produit obtenu (1,25 g) est dissous dans 25 ml de dichlorométhane et la solution est extraite 3 fois avec 30 ml au total d'une solution aqueuse d'acide chlorhydrique 1 N. Les extraits aqueux sont réunis, alcalinisés avec 40 ml d'une solution aqueuse saturée d'hydrogénocarbonate de sodium et extraits 3 fois avec 60 ml au total de dichlorométhane. Les extraits organiques sont réunis, lavés avec 20 ml d'eau distillée, séchés sur du sulfate de magnésium et concentrés à sec sous pression réduite (2 kPa) à 30°C. On obtient ainsi 0,56 g de (R,S)-4-(1-pyrrolidinyl)-6-trifluorométhyl-1,2,3,4-tétrahydroquinoléine sous la forme d'un solide blanc fondant à 99°C.

La (R,S)-2-imino-6-(2-oxopyrrolidin-1-yl)-8-trifluorométhyl-5,6-dihydro-2H,4H-thiazolo[5,4,3-ij]quinoléine peut être préparée de la manière suivante : une solution de 0,64 g de brome dans 5 ml d'acide acétique est ajoutée goutte à goutte, en 10 minutes à une température voisine de 25°C, à une solution de 1 g de (R,S)-4-(2-oxopyrrolidin-1-yl)-6-trifluorométhyl-1,2,3,4-tétrahydroquinoléine et de 0,85 g de thiocyanate de potassium dans 25 ml d'acide acétique. Le mélange est agité pendant 3 heures à une température voisine de 20°C, versé sur un mélange de 60 g de glace et de 60 g d'eau distillée et extrait avec 50 ml de dichlorométhane. Après décantation, la phase organique est éliminée et la phase aqueuse est alcalinisée avec 75 ml d'ammoniaque à 28% et extraite 4 fois avec 200 ml au total de dichlorométhane. Les extraits organiques sont réunis, lavés avec 50 ml d'eau distillée, séchés sur du sulfate de magnésium et concentrés à sec sous pression réduite (2 kPa) à 30°C. Le produit obtenu (0,9 g) est dissous dans 20 ml d'éther éthylique anhydre et le mélange est conservé pendant 15 minutes à une température voisine de 20°C. Le solide apparu est séparé par filtration, lavé avec 5 ml d'éther anhydre et séché sous pression réduite (0,13 kPa) à 60°C. On obtient ainsi 0,7 g de (R,S)-2-imino-6-(2-oxopyrrolidin-1-yl)-8-trifluorométhyl-5,6-dihydro-2H,4H-thiazolo[5,4,3-ij]quinoléine fondant à 171°C.

La (R,S)-4-(2-oxopyrrolidin-1-yl)-6-trifluorométhyl-1,2,3,4-tétrahydroquinoléine peut être préparée de la manière suivante : 13 g de tétrachlorure d'étain sont ajoutés goutte à goutte en 5 minutes à -78°C à une suspension de 6,6 g de N-para-toluènesulfonylméthyl-4-trifluorométhylaniline dans 100 ml de dichlorométhane. Le mélange est agité pendant 10 minutes et une solution de 2,2 g de 1-vinyl-2-pyrrolidinone dans 60 ml de dichlorométhane est ajoutée goutte à goutte en 10 minutes à la même température. Le mélange est agité pendant 2 heures à -78°C et pendant 1 heure à une température voisine de 20°C. L'insoluble apparu est séparé par filtration, lavé 2 fois avec 100 ml au total de dichlorométhane, mis en suspension dans 400 ml d'eau distillée et 400 ml de dichlorométhane et le mélange agité est neutralisé avec 400 ml d'une solution aqueuse saturée d'hydrogénocarbonate de sodium et filtré. Après décantation, la phase aqueuse est extraite 3 fois avec 600 ml au total de dichlorométhane et les extraits organiques sont réunis, lavés avec 200 ml d'eau distillée, séchés sur du sulfate de magnésium et concentrés à sec sous pression réduite (2 kPa) à 60°C. On obtient ainsi 4,9 g de (R,S)-4-(2-oxopyrrolidin-1-yl)-6-trifluorométhyl-1,2,3,4-tétrahydroquinoléine sous la forme d'une poudre blanche fondant à 139°C.

### EXEMPLE 19

Une solution de 2,1 g de brome dans 10 ml d'acide acétique est ajoutée goutte à goutte, en 15 minutes à une température voisine de 25°C, à une solution de 2,93 g de 7-trifluorométhoxy-2,3-dihydro-4H-[1,4]benzoxazine et de 2,83 g de thiocyanate de potassium dans 40 ml d'acide acétique. Le mélange est agité pendant 2 heures à une température voisine de 20°C, versé sur 40 g de glace, alcalinisé avec 40 ml d'ammoniaque à 28% et extrait 3 fois avec 150 ml au total d'acétate d'éthyle. Les extraits organiques sont réunis, séchés sur du sulfate de magnésium et concentrés à sec sous pression réduite (2 kPa) à 60°C. Le produit obtenu (3,1 g) est chromatographié sous pression d'azote (150 kPa) sur 30 g de gel de silice neutre 20-45 µm contenus dans une colonne de 2,5 cm de diamètre en éluant avec un mélange d'acétate d'éthyle et de méthanol (95-5 en volumes). Le produit obtenu (0,8 g) est dissous dans 5 ml d'éthanol et, après addition de 2 ml d'éthanol chlorhydrique 6,5 N et conservation pendant 1 heure à une température voisine de 5°C, le solide apparu est séparé par filtration, lavé avec 2 ml d'éthanol et séché sous pression réduite (0,13 kPa) à 60°C. On obtient ainsi 0,7 g de chlorhydrate de 2-imino-8-trifluorométhoxy-4,5-dihydro-2H-thiazolo[3,4,5-d,e]-[1,4]-benzoxazine sous la forme d'un solide jaune fondant au-dessus de 260°C avec décomposition [Spectre dans DMSO-d6, T=300K, δ en ppm (400 MHz) : 4,40 (2H, t, J=6Hz, NCH₂), 4,60 (2H, t, J=6Hz, OCH₂), 7,20 (1H, s, CH arom.), 7,70 (1H, s, CH arom), 10,80 (2H, s large, NH,HCl)].

La 7-trifluorométhoxy-2,3-dihydro-4H[1,4]benzoxazine peut être préparée de la manière suivante : à 18 ml d'une solution 0,5 M de tétrahydroaluminate de lithium dans le tétrahydrofuranne maintenue sous atmosphère inerte sont ajoutés goutte à goutte en 35 minutes à une température voisine de 5°C, une solution de 3,49 g de 7-trifluorométhoxy-4H-[1,4]benzoxazine-3-one dans 20 ml de tétrahydofuranne anhydre. Le mélange est agité pendant 1 heure à une température voisine de 20°C puis 15 ml d'eau distillée sont ajoutés lentement en maintenant la température inférieure à 15°C. Le mélange est filtré et le filtrat est extrait 3 fois avec 150 ml au total d'acétate d'éthyle. Les extraits organiques sont réunis, lavés 3 fois avec 150 ml au total d'eau distillée, séchés sur du sulfate de magnésium et concentrés à sec sous pression réduite (2,2 kPa) à 60°C. On obtient ainsi 2,98 g de 7-trifluorométhoxy-2,3-dihydro-4H-[1,4]benzoxazine sous la forme d'un solide jaune [RMN Spectre ¹H dans CDCl₃, T=300K, δ en ppm (300 MHz) : 3,30 (2H, t, J=6Hz, NCH₂), 3,70 (1H, s large, NH), 4,20 (2H, t, J=6Hz, OCH₂), 6,45 (1H, d, J=8Hz, CH arom.), 6,55 (1H, dd, J=8 et 2 Hz, CH arom.), 6,60 (1H, d, J=2Hz, CH arom.)].

La 7-trifluorométhoxy-4H-[1,4]benzoxazine-3-one peut être préparée de la manière suivante : une solution de 5,6 g de chlorure de chloroacétyle dans 85 ml de chloroforme est ajoutée goutte à goutte en 30 minutes à une température voisine de 5°C à une solution de 9,85 g de 2-amino-5-trifluorométhoxyphénol et de 9,8 g de chlorure de triéthylbenzylammonium dans 170 ml de chloroforme contenant en suspension 17,3 g d'hydrogénocarbonate de sodium. Le mélange est agité pendant 1 heure à la même température, pendant 5 heures à l'ébullition, refroidi à une température voisine de 20°C et concentré à sec sous pression réduite (2,2 kPa) à 60°C. Le produit obtenu est mis en suspension dans 170 ml d'eau distillée puis l'insoluble est séparé par filtration, lavé 3 fois avec 255 ml au total d'eau distillée et séché à l'air. Le produit obtenu (8,78 g) est chromatographié sous pression d'azote (150 kPa) sur 200 g de gel de silice neutre 20-45 µm contenus dans une colonne de 5,5 cm de diamètre en éluant avec du dichlorométhane. On obtient ainsi 3,49 g de 7-trifluorométhoxy-4H-[1,4]benzoxazine-3-one sous la forme d'un solide jaune fondant vers 150°C.

Le 2-amino-5-trifluorométhoxyphénol peut être préparé de la manière suivante : 12 g de N-(2-hydroxy-4-trifluorométhoxyphényl)carbamate de tert-butyle sont ajoutés en 15 minutes à une température voisine de 25°C à 225 ml d'une solution 6,5 N de dioxanne chlorhydrique. Le mélange est agité pendant 3 heures à la même température et concentré à sec sous pression réduite (2,2 kPa) à 60°C. On obtient ainsi 9,85 g de chlorhydrate de 2-amino-5-trifluorométhoxyphénol sous la forme d'un solide beige se sublimant à 170°C [RMN Spectre ¹H dans DMSO-d6, T=300K, δ en ppm (300 MHz) : 6,90 (1H, dd, J=8 et 2Hz, CH arom.), 7,05 (1H, s, CH arom.), 7,45 (1H, d, J=8Hz, CH arom.), 9,50 (3H, s large, NH2-HCl), 11,20 (1H, s, OH)].

Le N-(2-hydroxy-4-trifluorométhoxyphényl)carbamate de tert-butyle peut être préparé de la manière suivante : 69 ml d'une solution 1,5M de tert-butyllithium dans le pentane sont ajoutés goutte à goutte en 1 heure à -70°C à une solution maintenue sous atmosphère inerte de 12 g de N-(4-trifluorométhoxyphényl)carbamate de tert-butyle dans 130 ml de tétrahydrofuranne anhydre. Le mélange est agité pendant 2 heures à -20°C, refroidi à -70°C et 13,9 g de borate de triméthyle sont ajoutés goutte à goutte en 15 minutes. Le mélange est agité pendant 1 heure et 30 minute à -20°C et 7,8 ml d'acide acétique sont ajoutés goutte à goutte en 15 minutes à -5°C. Le mélange est agité pendant 5 minutes et 28,6 ml d'eau oxygénée à 16% sont ajoutés goutte à goutte en 15 minutes à 0°C. Le mélange est agité en laissant remonter la température à 20°C puis 65 ml d'eau distillée et 170 ml d'éther éthyliques sont ajoutés. Après décantation, la phase éthérée est lavée avec une solution aqueuse de bisulfite de sodium, avec de l'eau distillée et avec une solution saturée de chlorure de sodium, séchée sur du sulfate de magnésium et concentrée à sec sous pression réduite (2,2 kPa) à 60°C. On obtient ainsi 12 g de N-(2-hydroxy-4-trifluorométhoxyphényl)carbamate de tert-butyle sous la forme d'un solide jaune fondant à 102°C.

### EXEMPLE 20

On opère comme à l'exemple 1 mais à partir de 3,8 g de brome dilué dans 21 ml d'acide acétique, 5,3 g de thiocyanate de potassium et 5,5 g de (R,S)-6-trifluorométhyl-1,2,3,4-tétrahydroquinoléine-2-méthanol dans 71 ml d'acide acétique. On obtient ainsi 0,6 g de chlorhydrate de (R,S)-2-imino-8-trifluorométhyl-5,6-dihydro-2H,4H-thiazolo[5,4,3-ij]quinoléine-4-méthanol, fondant à 265°C avec décomposition [Analyse C12H12ClF3N2OS, % calculé C : 44,38, H : 3,72, Cl : 10,92, F : 17,55, N : 8,63, O : 4,93, S : 9,87, trouvé C : 44,6, H : 4,1, Cl : 11,0, F : 17,1, N : 8,5, S : 9,5].

Le (R,S)-6-trifluorométhyl-1,2,3,4-tétrahydroquinoléine-2-méthanol peut être préparé comme à l'exemple 1, mais à partir de 6,4 g de 6-trifluorométhylquinoléine-2-méthanol et 0,5 g d'oxyde de platine dans 60 ml de méthanol. On obtient 5,5 g de (R,S)-6-trifluorométhyl-1,2,3,4-tétrahydroquinoléine-2-méthanol sous forme d'une huile claire [RMN Spectre dans DMSO-d6, T=300K, d en ppm (300 MHz) : 1,55 et 1,85 (1H chacun, m, CH₂), 2,75 (2H, t, J=6Hz, CH₂), 3,40 (1H, m, NCH), 3,48 (2H, t large, CH₂O), 4,90 (1H, t, J=5Hz, OH), 6,40 (1H, s, NH), 6,70 (1H, d, J=8Hz, CH arom.), 7,20 (2H, m, CH arom.)].

Le 6-trifluorométhylquinoléine-2-méthanol peut être préparé comme à l'exemple 7, mais à partir de 45,5 g de 2-méthyl-6-trifluorométhylquinoléine-1-oxyde dans 200 ml d'anhydride acétique. On obtient ainsi 6,4 g de 6-trifluorométhylquinoléine-2-méthanol sous forme d'un solide brunâtre fondant à 85°C.

Le 2-méthyl-6-trifluorométhylquinoléine-1-oxyde peut être préparé comme à l'exemple 6 mais à partir de 10,5 g de 2-méthyl-6-trifluorométhylquinoléine et de 41 g d'acide 3-chloroperbenzoïque dans 200 ml de dichlorométhane. On obtient ainsi 11,6 g de 2-méthyl-6-trifluorométhylquinoléine-1-oxyde sous forme d'un solide pâteux [RMN Spectre dans CDCl₃, T=300K, δ en ppm (300 MHz) : 2,75 (3H, s, CH₃), 7,45 (1H, d, J=8Hz, CH arom.), 7,75 (1H, d, J=8Hz, CH arom.), 7,90 (1H, dd, J=8 et 2Hz, CH arom.), 8,15 (1H, s, CH arom.), 8,90 (1H, d, J=8Hz, CH arom.)].

### EXEMPLE 21

On opère comme à l'exemple 9, mais à partir de 1 g de 2-imino-8-trifluorométhyl-4,5-dihydro-2H-thiazolo-[3,4,5-de][1,4]benzothiazine et de 4,2 g d'acide 3-chloroperbenzoïque (pureté 80%) dans 20 ml de dichlorométhane. On obtient ainsi 720 mg de méthanesulfonate de 2-imino-8-trifluorométhyl-4,5-dihydro-2H-thiazolo-[3,4,5-de][1,4]benzothiazine-6,6-dioxyde sous forme d'une poudre blanche, fondant à une température supérieure à 260°C [Analyse C11H11F3N2O5S3, % calculé C : 32,67, H : 2,74, F : 14,09, N : 6,93, S : 23,79, % trouvé C : 32,75, H : 2,58, F : 14,17, N : 6,94, S : 24,02].

### EXEMPLE 22

On ajoute goutte à goutte en 10 minutes, à une température voisine de 20°C et sous argon, 2,07 g de brome difué dans 6,75 ml d'acide acétique à une solution de 3,6 g de thiocyanate de potassium et de 3,43 g de 7-trifluorométhyl-3,4-dihydro-2H-benzo[b][1,4]sélénazine dans 52 ml d'acide acétique. Le mélange réactionnel est agité pendant 3 heures à la même température, versé sur de la glace, alcalinisé par une solution d'ammoniaque à 20% et extrait par 100 ml d'acétate d'éthyle. La phase organique est lavée par 50 ml d'eau distillée, séchée sur sulfate de magnésium et concentrée à sec sous pression réduite (4 kPa) à 40°C. Le produit brut est chromatographié sur colonne de gel de silice en éluant avec un mélange de cyclohexane et d'acétate d'éthyle (50-50 en volumes) pour donner 2,2 g d'un solide jaune pâle. Ce dernier, après décoloration au moyen de charbon végétal dans l'éther éthylique au reflux fournit 2,7 g de 2-imino-8-trifluorométhyl-4,5-dihydro-2H-thiazolo[5,4,3-ed]-1,4-benzosélénazine sous forme de cristaux blancs fondant à 130°C [RMN Spectre ¹H dans DMSO-d6, T=300K, δ en ppm (300 Mhz) : 3,30 (2H, t, J=6Hz, SeCH₂), 4,30 (2H, t, J=6Hz, NCH₂), 7,60 (1H, s, CH arom.), 7,75 (1H, s, CH arom.), 8,80 (1H, s, NH)].

La 7-trifluorométhyl-3,4-dihydro-2H-benzo[b][1,4]sélénazine peut être préparée de la manière suivante : à une suspension de 10,5 g de 7-trifluorométhyl-3,4-dihydro-2H-benzo[b][1,4]sélénazin-3-one dans 255 ml de toluène on ajoute goutte à goutte, sous argon et à une température voisine de 20°C, 35,5 ml d'une solution 2 N dans le tétrahydrofurane du complexe borane-méthylsulfure. Le milieu réactionnel est ensuite porté et maintenu à l'ébullition pendant 1 heure 45 minutes. Après refroidissement vers 20°C, il est hydrolysé avec 600 ml d'une solution saturée d'hydrogénocarbonate de sodium, puis extrait par 3 fois 100 ml d'acétate d'éthyle. Les phases organiques réunies sont lavées par 100 ml d'eau distillée, séchées sur sulfate de magnésium et concentrées à sec sous pression réduite (4 kPa) à 40°C. Après trituration du résidu dans l'éther de pétrole on obtient 9,65 g de 7-trifluorométhyl-3,4-dihydro-2H-benzo[b][1,4]sélénazine sous forme de cristaux blanchâtres fondant à 55°C.

La 7-trifluorométhyl-3,4-dihydro-2H-benzo[b][1,4]sélénazin-3-one peut être préparée de la manière suivante : à une solution de 17 g de 2-(2-*tert*-butoxycarbonylamino-5-trifluorométhylphénylsélanyl)acétate d'éthyle dans 150 ml de dichlorométhane sont ajoutés 15 ml d'acide trifluoroacétique. Après 29 heures à une température voisine de 20°C le milieu réactionnel est traité par 250 ml d'une solution saturée d'hydrogénocarbonate de sodium et extrait par 250 ml d'acétate d'éthyle. La phase organique est lavée par de l'eau distillée, séchée sur sulfate de magnésium et concentrée à sec sous pression réduite (4 kPa) à 40°C. Après trituration du résidu dans un mélange d'éther éthylique et d'éther de pétrole on obtient 10,5 g de 7-trifluorométhyl-3,4-dihydro-2H-benzo[b][1,4]sélénazin-3-one sous forme de cristaux blancs fondant à 207°C.

Le 2-(2-*tert*-butoxycarbonylamino-5-trifluorométhylphénylsélanyl)acétate d'éthyle peut être préparé de la manière suivante : à une solution de 14,47 g de N-[2-(2-*tert*-butoxycarbonylamino-5-trifluorométhylphényldisélanyl)-4-trifluorométhylphényl]carbamate de *tert*-butyle dans un mélange de 100 ml d'éthanol et de 150 ml de tétrahydrofurane, on ajoute petit à petit, à une température voisine de 20°C et sous argon, 1 g de tétrahydroborate de sodium. Après 10 minutes on coule goutte à goutte la solution de 7,5 g de bromoacétate d'éthyle dans 5 ml de tétrahydrofurane. On ajoute à nouveau 0,6 g de tétrahydroborate de sodium en plusieurs fois durant 1 heure. Le milieu réactionnel est ensuite versé sur 150 ml d'une solution environ 4 N de chlorure d'ammonium et extrait par 250 ml d'éther éthylique. La phase organique est concentrée jusqu'à apparition de 2 phases et le résidu est extrait à nouveau par 200 ml d'éther éthylique. La phase organique est lavée à l'eau distillée, séchée sur sulfate de magnésium et concentrée à sec sous pression réduite (4 kPa) à 40°C. Le produit brut est redissous, à température voisine de 20°C et sous argon, dans 100 ml de diméthylformamide. On ajoute petit à petit 0,5 g de tétrahydroborate de sodium, puis 3 g de bromoacétate d'éthyle. Après environ 20 minutes on traite par 200 ml d'une solution environ 4 N de chlorure d'ammonium et extrait par 2 fois 100 ml d'acétate d'éthyle. Les phases organiques réunies sont lavées par 100 ml d'eau distillée, séchées sur sulfate de magnésium et concentrées à sec sous pression réduite (4 kPa) à 40°C. Après trituration du résidu dans l'éther de pétrole on obtient 10,85 g de 2-(2-*tert*-butoxycarbonylamino-5-trifluorométhylphénylsélanyl) acétate d'éthyle sous forme de cristaux blancs fondant à 69°C.

Le N-[2-(2-*tert*-butoxycarbonylamino-5-trifluorométhylphényldisélanyl)-4-trifluorométhylphényl]carbamate de *tert*-butyle peut être préparé de la manière suivante : à une solution de 42 g de 4-trifluorométhylphénylcarbamate de *tert*-butyle dans 300 ml de tétrahydrofurane anhydre, maintenue sous une atmosphère d'argon à une température inférieure à -20°C, on ajoute goutte à goutte en 30 minutes 200 ml d'une solution (environ 1,7 M) de *tert*-butyllithium dans le pentane. Le mélange est agité pendant 2 heures à la même température, puis on ajoute 13,5 g de poudre de sélénium à - 30°C. Le mélange est agité pendant 35 minutes vers - 15°C, puis est additionné de 100 ml d'une solution saturée de chlorure d'ammonium et laissé remonter vers 20°C. Il est ensuite agité à cette température, au contact de l'air, pendant 18 heures, puis extrait par 600 ml d'éther éthylique. La phase organique est séchée sur sulfate de magnésium et concentrée à sec sous pression réduite (4 kPa) à 40°C. Le produit obtenu est filtré sur gel de silice en éluant avec un mélange de cyclohexane et d'acétate d'éthyle (65-35 en volumes). Après trituration dans l'éther de pétrole on obtient 39,1 g de N-[2-(2-*tert*-butoxycarbonylamino-5-trifluorométhylphényldisélanyl)-4-trifluorométhylphényl]carbamate de *tert*-butyle sous forme de cristaux jaunes fondant à 150°C.

### EXEMPLE 23

On ajoute goutte à goutte en 10 minutes, à une température voisine de 20°C et sous argon, 5,05 g de brome dilué dans 10 ml d'acide acétique à une solution de 9,3 g de thiocyanate de potassium et de 4 g de 3,3-difluoro-6-trifluorométhyl-1,2,3,4-tétrahydro-4-quinoléinol dans 180 ml d'acide acétique. Le mélange réactionnel est agité pendant 18 heures à la même température, versé sur de la glace, alcalinisé par une solution d'ammoniaque à 20% et extrait par 200 ml d'acétate d'éthyle. La phase organique est séchée sur sulfate de magnésium et concentrée à sec sous pression réduite (4 kPa) à 40°C. Le produit brut est chromatographié sur colonne de gel de silice en éluant avec un mélange de cyclohexane et d'acétate d'éthyle (65-35 puis 35-65 en volumes) pour donner une huile jaune. Celle-ci est redissoute dans l'éther éthylique et additionnée d'une solution d'éther chlorhydrique. Le solide obtenu est dissous dans l'isopropanol, puis reprécipité par addition d'acétate d'isopropyle. On obtient 0,32 g de chlorhydrate de (R,S)-5,5-difluoro-6-hydroxy-2-imino-8-trifluorométhyl-5,6-dihydro-2H,4H-thiazolo[5,4,3-ij]quinoléine sous forme de solide blanc fondant au dessus de 200°C avec décomposition [RMN Spectre ¹H dans DMSO-d6, T=300K, δ en ppm (300 Mhz) : 4,75 (2H, m, NCH₂), 5,30 (1H, dd, J=9 et 3Hz, OCH), 7,15 (1H, s, OH), 8,00 (1H, s, CH arom.), 8,50 (1H, s, CH arom.), 11,20 (2H, s, NH,HCl)].

Le 3,3-difluoro-6-trifluorométhyl-1,2,3,4-tétrahydro-4-quinoléinol peut être préparé de la manière suivante : à une suspension de 10,76 g de 3,3-difluoro-4-hydroxy-6-trifluorométhyl-1,2,3,4-tétrahydro-2-quinoléinone dans 250 ml de toluène on ajoute goutte à goutte, sous argon et à une température voisine de 20°C, 30 ml de complexe borane-méthylsulfure. Le milieu réactionnel est ensuite porté et maintenu à l'ébullition pendant 1 heure et 10 minutes. Après refroidissement vers 20°C, il est hydrolysé avec 100 ml d'une solution saturée d'hydrogénocarbonate de sodium, puis la phase organique est séchée sur sulfate de magnésium et concentrée à sec sous pression réduite (4 kPa) à 40°C. Le résidu est traité par une solution diluée d'acide chlorhydrique et extrait par de l'éther éthylique. La phase organique est séchée sur sulfate de magnésium et concentrée à sec sous pression réduite (4 kPa) à 40°C. Après trituration du résidu dans l'éther de pétrole on obtient 7,1 g de 3,3-difluoro-6-trifluorométhyl-1,2,3,4-tétrahydro-4-quinoléinol sous forme de cristaux roses fondant à 110°C.

La 3,3-difluoro-4-hydroxy-6-trifluorométhyl-1,2,3,4-tétrahydrohydro-2-quinoléinone peut être préparée de la manière suivante : à une solution de 11,85 g de 3-(2*-tert*-butoxycarbonylamino-5-trifluorométhylphényl)-2,2-difluoro-3-hydroxypropanoate d'éthyle dans 65 ml de dioxane on ajoute 35 ml de dioxane chlorhydrique environ 6 N. Après 18 heures à une température voisine de 20°C le milieu réactionnel est concentré sous vide, puis le résidu est traité par 50 ml d'une solution saturée d'hydrogénocarbonate de sodium et extrait par 200 ml d'acétate d'éthyle. La phase organique est séchée sur sulfate de magnésium et concentrée à sec sous pression réduite (4 kPa) à 40°C. Après trituration du résidu dans l'éther de pétrole on obtient 6,8 g de 3,3-difluoro-4-hydroxy-6-trifluorométhyl-1,2,3,4-tétrahydro-2-quinoléinone sous forme d'un solide crème fondant à 171°C.

Le 3-(2-*tert*-butoxycarbonylamino-5-trifluorométhy(phényl)-2,2-difluoro-3-hydroxypropanoate d'éthyle peut être préparé de la manière suivante : à une solution de 1,45 g de 2-formyl-4-trifluorométhylphénylaminocarboxylate de *tert*-butyle dans 10 ml de dioxane sont ajoutés 1,22 g de bromodifluoroacétate d'éthyle, 0,75 g de poudre de zinc et 0,3 g d'iode. Le milieu réactionnel est ensuite soniqué pendant 3 heures à une température passant de 30 à 50°C. Après refroidissement vers 0°C et addition de 100 ml d'acétate d'éthyle, le mélange est filtré, puis on ajoute une solution saturée de chlorure de sodium. La phase organique est séchée sur sulfate de magnésium et concentrée à sec sous pression réduite (4 kPa) à 40°C. Le produit brut est chromatographié sur colonne de gel de silice en éluant avec un mélange de cyclohexane et d'acétate d'éthyle (70-30 en volumes) pour donner 0,85 g de 3-(2-*tert*-butoxycarbonylamino-5-trifluorométhylphényl)-2,2-difluoro-3-hydroxypropanoate d'éthyle sous forme d'une huile claire [RMN Spectre ¹H dans DMSO-d6, T=300K, δ en ppm (250 Mhz) : 1,25 (3H, t, J=6Hz, CH₃), 1,50 (9H, s, (CH₃)₃), 4,28 (2H, q, J=6Hz, OCH₂), 5,65 (1H, m, OCH), 7,42 (1H, d, J=4Hz, OH), 7,70 (1H, d, J=8Hz, CH arom.), 7.76 (1H, s, CH arom.), 7,94 (1H, d, J=8Hz, CH arom.), 9.18 (1H, s, NH)].

Le 2-formyl-4-trifluorométhylphénylaminocarboxylate de *tert*-butyle peut être préparé par la méthode décrite dans C.A. 107: 39815x.

### EXEMPLE 24

On opère comme à l'exemple 23 mais à partir de 1,55 g de brome dans 2 ml d'acide acétique, 2,8 g de thiocyanate de potassium et 2,3 g de 3,3-difluoro-6-trifluorométhyl-1,2,3,4-tétrahydroquinoléine dans 30 ml d'acide acétique. Le produit brut est chromatographié sur colonne de gel de silice en éluant avec un mélange de cyclohexane et d'acétate d'éthyle (50-50 en volumes) pour donner environ 0,35 g d'un solide blanchâtre fondant vers 85°C. Celui-ci est redissous dans l'éther éthylique et additionné de 0,1 ml d'acide méthanesulfonique. Après trituration du solide obtenu dans l'éther éthylique on obtient 0,35 g de méthanesulfonate de 5,5-difluoro-2-imino-8-trifluorométhyl-5,6-dihydro-2H,4H-thiazolo[5,4,3-ij]quinoléine sous forme de solide blanc fondant au dessus de 235°C avec décomposition [RMN Spectre ¹H dans DMSO-d6, T=300K, δ en ppm (300 Mhz) : 2,35 (3H, s, CH₃SO₃H), 3,80 (2H, t, J=16Hz, CH₂), 4,65 (2H, t, J=13Hz, CH₂), 7,90 (1H, s, CH arom.), 8,40 (1H, s, CH arom.), 10,70 (1H, s, NH)].

La 3,3-difluoro-6-trifluorométhyl-1,2,3,4-tétrahydroquinoléine peut être préparée de la manière suivante : à une solution de 0,7 g de 3,3-difluoro-6-trifluorométhyl-1,2,3,4-tétrahydro-4-quinoléinol dans 15 ml de dichlorométhane on ajoute, sous argon et à une température voisine de 20°C, 1 ml de triéthylsilane et 3 ml d'acide trifluoroacétique. Le milieu réactionnel est agité pendant 4 heures à la même température, versé sur de la glace, alcalinisé par une solution d'ammoniaque à 20% et extrait 2 fois par un mélange d'acétate d'éthyle et d'éther éthylique. Les extraits organiques sont réunis, lavés par une solution diluée de chlorure de sodium, séchés sur sulfate de magnésium et concentrés à sec sous pression réduite (4 kPa) à 40°C. Le produit brut est chromatographié sur colonne de gel de silice en éluant avec un mélange de cyclohexane et d'acétate d'éthyle (80-20 en volumes) pour donner 0,18 g de 3,3-difluoro-6-trifluorométhyl-1,2,3,4-tétrahydroquinoléine sous forme d'une huile incolore [RMN Spectre ¹H dans DMSO-d6, T=300K, δ en ppm (250 Mhz) : 3,30 (2H, t, J=15Hz, CH₂), 3,55 (2H, t, J=12Hz, NCH₂), 6,70 (1H, d, J=8Hz, CH arom.), 6,83 (1H, s, NH), 7,30 (1H, d, J=8Hz, CH arom.), 7,32 (1H, s, CH arom.)].

### EXEMPLE 25

On opère comme à l'exemple 23 mais à partir de 2,93 g de brome dans 5 ml d'acide acétique, 5,3 g de thiocyanate de potassium et 3,7 g de 6-trifluorométhyl-1,2,3,4-tétrahydroquinoxaline dans 60 ml d'acide acétique. Le produit brut est chromatographié sur colonne de gel de silice en éluant avec un mélange d'acétate d'éthyle et de méthanol (95-5 en volumes) pour donner environ 1,8 g d'une huile jaune. Celle-ci est mise en solution dans l'éther éthylique et additionnée d'éther chlorhydrique. Après trituration du solide obtenu dans l'éther éthylique, celui-ci est isolé par filtration, puis redissous dans l'eau. La solution est alcalinisée par l'ammoniaque à 20% et extraite par 2 fois 100 ml d'acétate d'éthyle. Les extraits organiques sont réunis, lavés à l'eau distillée, séchés sur sulfate de magnésium et concentrés à sec sous pression réduite (4 kPa) à 40°C. Le résidu huileux obtenu est chromatographié sur colonne de gel de silice, en éluant avec un mélange de tétrahydrofurane, de cyclohexane et d'éthanol (59,7-40-0,3 en volumes). On obtient ainsi 0,75 g de 2-imino-8-trifluorométhyl-5,6-dihydro-2H,4H-thiazolo[3,4,5-de]quinoxaline sous forme d'un solide blanc fondant à 112°C [ RMN Spectre ¹H dans DMSO-d6, T=300K, δ en ppm (250 Mhz) : 3,40 (2H, d, J=5Hz, NCH₂), 3,80 (2H, d, J=5Hz, NCH₂), 6,43 (1H, s, NH), 6,78 (1H, s, CH arom.), 7,12 (1H, s, CH arom.), 8,35 (1H, s, NH)].

La 6-trifluorométhyl-1,2,3,4-tétrahydroquinoxaline peut être préparée par la méthode décrite par Rao et col., J. Heterocycl. Chem., (1973), 10(2), 213-5.

### EXEMPLE 26

On opère comme à l'exemple 23 mais à partir de 0,45 g de brome dans 2 ml d'acide acétique, 0,7 g de 2-hydroxyméthyl-7-trifluorométhyl-3,4-dihydro-2H-[1,4]benzothiazine et 0,6 g de thiocyanate de potassium dans 10 ml d'acide acétique. Le produit obtenu est mis en suspension dans de l'éther isopropylique et de l'éther éthylique, séparé par filtration, lavé avec de l'éther isopropylique et séché sous pression réduite. On dissout 0,188 g du produit ainsi obtenu (sur 0,470 g obtenu au total) dans 15 ml d'éthanol auxquels on ajoute 0,044 ml d'acide méthanesulfonique. Après 1 heure d'agitation à 20°C, la solution est concentrée à sec sous pression réduite (2 kPa) et le produit est repris par 10 ml d'acétone, séparé par filtration, lavé avec deux fois 5 ml d'acétone, séché 16 heures sous hotte ventilée. On obtient ainsi 0,215 g de méthanesulfonate de 5-hydroxyméthyl-2-imino-8-trifluorométhyl-4,5-dihydro-2H-thiazolo[3,4,5-de][1,4]benzothiazine sous forme d'un solide blanc fondant à 216°C [Analyse C12H13F3N2O4S3, % calculé C : 35,82, H : 3,26, F : 14,16, N : 6,96, O : 15,90, S : 23,90, % trouvé C: 35,91, H : 3,13, F : 14,12, N : 6,73, S : 24,11].

La 2-hydroxyméthyl-7-trifluorométhyl-3,4-dihydro-2H-[1,4]benzothiazine peut être préparée de la manière suivante : à une solution de 1,7 g de 2-méthoxycarbonyl-7-trifluorométhyl-3,4-dihydro-2H-[1,4]benzothiazine dans 50 ml de toluène et environ 10 ml de tétrahydrofurane, maintenue sous argon, on ajoute rapidement 6 ml d'une solution environ 2 M de complexe borane-diméthylsulfure dans le tétrahydrofurane. Le mélange réactionnel est porté 1 heure au reflux, puis après retour à 20°C, est hydrolysé avec 70 ml d'une solution aqueuse saturée en hydrogénocarbonate de sodium et 40 ml d'acétate d'éthyle. L'insoluble est éliminé par filtration et le filtrat décanté, la phase organique est séchée sur sulfate de magnésium et concentrée à sec sous pression réduite (2 kPa). L'huile jaune obtenue est chromatographiée sur 35 g de gel de silice 20-45 µm contenus dans une colonne de 2 cm de diamètre, en éluant avec un mélange d'acétate d'éthyle et de cyclohexane (75-25 en volumes). On obtient ainsi 0,72 g de 2-hydroxyméthyl-7-trifluorométhyl-3,4-dihydro-2H-[1,4]benzothiazine sous forme d'un solide jaune fondant à 80°C.

La 2-méthoxycarbonyl-7-trifluorométhyl-3,4-dihydro-2H-[1,4]benzothiazine peut être préparée de la manière suivante : on porte à environ 45°C, un mélange de 2,55 g de 2-méthoxycarbonyl-7-trifluorométhyl-4H-[1,4]benzothiazine, de 9 g de magnésium en tournures dans 100 ml de méthanol. Après démarrage de la réaction, la température monte au reflux et s'y maintient d'elle même. Après retour vers 20°C, on dilue le milieu réactionnel avec 50 ml de méthanol et on agite 16 heures à environ 20°C. Le milieu refroidi vers 0°C est hydrolysé par 154 ml d'acide chlorhydrique 4 N, agité 1 heure puis filtré sur un lit de célite 545. La célite est successivement lavée par 500 ml de dichlorométhane et 40 ml d'acétone. La phase organique est ensuite lavée par 200 ml d'eau distillée, séchée sur sulfate de magnésium et concentrée à sec sous pression réduite (2 kPa). On obtient ainsi 1,75 g de 2-méthoxycarbonyl-7-trifluorométhyl-3,4-dihydro-2H-[1,4]benzothiazine, sous forme d'une huile brune utilisée telle quelle.

La 2-méthoxycarbonyl-7-trifluorométhyl-4H-[1,4]benzothiazine peut être préparée de la manière suivante : on agite pendant 24 heures à environ 20°C, un mélange de 7,8 g de 2-(2-*tert*-butoxycarbonylamino-5-trifluorométhyl-phénylsulfanyl)-3-diméthylamino acrylate de méthyle, 10,3 g d'acide trifluoroacétique et 80 ml de dichlorométhane anhydre. Après concentration à sec sous pression réduite, on reprend le résidu avec 200 ml de solution aqueuse saturée en hydrogénocarbonate de sodium et on extrait avec deux fois 200 ml d'acétate d'éthyle. Les extraits organiques réunis sont séchés sur sulfate de magnésium, concentrés à sec sous pression réduite (2 kPa) et le produit obtenu est remis en suspension dans de l'éther isopropylique. Le solide est séparé par filtration, lavé avec de l'éther isopropylique, séché sous pression réduite. On obtient ainsi 2,57 g de 2-méthoxycarbonyl-7-trifluorométhyl-4H-[1,4]benzothiazine sous forme d'un solide orange fondant à 238°C.

Le 2-(2*-tert*-butoxycarbonylamino-5-trifluorométhyl-phénylsulfanyl)-3-diméthylamino acrylate de méthyle peut être préparé de la manière suivante : on chauffe au reflux pendant 7 heures un mélange, maintenu sous argon, de 10,95 g de (5-trifluorométhyl -2-*tert*-butoxycarbonylaminophényl) sulfanyl acétate de méthyle, de 15,5 g de *tert*-butoxy-bis(diméthylamino)méthane et 300 ml de dichlorométhane anhydre. Après retour à environ 20°C et 16 heures d'agitation, le milieu est concentré à sec sous pression réduite et le produit obtenu est remis en suspension dans 20 ml d'éther isopropylique. Le solide est séparé par filtration, lavé trois fois avec de l'éther isopropylique, séché sous pression réduite. On obtient ainsi 3,4 g de 2-(2*-tert*-butoxycarbonylamino-5-trifluorométhyl-phénylsulfanyl)-3-diméthylamino acrylate de méthyle, sous forme d'un solide crème fondant à 139°C. Le filtrat du solide précédemment obtenu est concentré à sec sous pression réduite (2 kPa) et le produit obtenu est chromatographié sous pression d'azote (150 kPa) sur 75 g de gel de silice 20-45 µm contenus dans une colonne de 2,5 cm de diamètre, en éluant avec un mélange de cyclohexane et d'acétate d'éthyle (80-20 en volumes). On récupère ainsi 4,45 g de 2-(2*-tert*-butoxycarbonylamino-5-trifluorométhyl-phénylsulfanyl)-3-diméthylamino acrylate de méthyle, sous forme d'un solide crème fondant à 140°C.

### EXEMPLE 27

On opère comme à l'exemple 23 mais à partir de 6,55 g de brome dans 20 ml d'acide acétique, 9,6 g de (R,S)-2-méthyl-7-trifluorométhyl-3,4-dihydro-2H-[1,4]benzothiazine et 8,9 g de thiocyanate de potassium dans 100 ml d'acide acétique. Le produit obtenu est chromatographié sous pression d'azote (150 kPa) sur 120 g de gel de silice 20-45 µm contenus dans une colonne de 3 cm de diamètre, en éluant avec un mélange d'acétate d'éthyle et de cyclohexane (50-50 en volumes). Le produit obtenu (9,9 g) est dissous dans 150 ml d'éthanol absolu auxquels on ajoute 2,7 ml d'acide méthanesulfonique. Après 5 heures 30 mn d'agitation à environ 20°C, le produit est séparé par filtration, lavé avec de l'éthanol absolu puis de l'éther isopropylique, séché sous pression réduite. On obtient ainsi 10,7 g de méthanesulfonate de (R,S)-2-imino-5-méthyl-8-trifluorométhyl-4,5-dihydro-2H-thiazolo[3,4,5-de][1,4]benzothiazine, sous forme de cristaux écrus fondant à une température supérieure à 260°C [Analyse C12H13F3N2O3S3, % calculé C : 37,30, H : 3,39, F : 14,75, N : 7,25, O : 12,42, S : 24,89, % trouvé C : 37,66, H : 3,24, F : 14,55, N : 7,30, S : 25,30].

La (R,S)-2-méthyl-7-trifluorométhyl-3,4-dihydro-2H-[1,4]benzothiazine peut se préparer de la manière suivante ; à un mélange de 11,5 g de (R,S)-2-méthyl-7-trifluorométhyl-2,4-dihydro-[1,4]benzothiazin-3-one dans 320 ml de toluène, on ajoute, en 10 minutes, 44 ml d'une solution environ 2 M de complexe borane-diméthylsulfure dans le tétrahydrofurane. Le milieu réactionnel est porté 2 heures au reflux puis, après retour à environ 20°C, est jeté sur 400 ml de solution aqueuse saturée en hydrogénocarbonate de sodium et 200 ml d'eau distillée. Le produit est extrait deux fois par 150 ml d'acétate d'éthyle. Les extraits organiques réunis sont séchés sur sulfate de magnésium, concentrés à sec sous pression réduite et le produit ainsi obtenu est remis en suspension dans de l'éther de pétrole, puis séparé par filtration, lavé avec de l'éther de pétrole, séché sous pression réduite. On obtient ainsi 9,60 g de (R,S)-2-méthyl-7-trifluorométhyl-3,4-dihydro-2H-[1,4]benzothiazine, sous forme d'un solide crème fondant à 112°C.

La (R,S)-2-méthyl-7-trifluorométhyl-2,4-dihydro-[1,4]benzothiazin-3-one peut être préparée de la manière suivante : on agite, à environ 20°C pendant 16 heures, un mélange de 30,1 g de (R,S)-2-(2-*tert*-butoxycarbonylamino-5-trifluorométhyl-phénylsulfanyl)-propionate d'éthyle, de 43 g d'acide trifluoroacétique et 90 ml de dichlorométhane. Le solvant est ensuite éliminé par concentration sous pression réduite et remplacé par 150 ml de tétrahydrofurane. Le mélange est porté 24 heures au reflux puis concentré à sec sous pression réduite (2 kPa) et le produit obtenu est remis en suspension dans de l'éther isopropylique, séparé par filtration, lavé avec de l'éther isopropylique, séché sous pression réduite. On obtient ainsi 10,6 g de (R,S)-2-méthyl-7-trifluorométhyl-2,4-dihydro-[1,4]benzothiazin-3-one, sous forme d'un solide marron fondant à 200°C.

Le (R,S)-2-(2-*tert*-butoxycarbonylamino-5-trifluorométhyl-phénylsulfanyl)-propionate d'éthyle peut être préparé de la manière suivante : à une solution de 20 g de 4-trifluorométhylphénylcarbamate de *tert*-butyle dans 260 ml de tétrahydrofurane anhydre, maintenue sous atmosphère d'argon à une température inférieure à -70°C, on ajoute goutte à goute, en 50 minutes, 100 ml d'une solution (environ 1,7 M) de *tert*-butyllithium dans le pentane. Le mélange est agité pendant 3 heures 30 minutes à une température maintenue aux environs de -20°C, puis refroidi vers -70°C avant d'ajouter 2,5 g de soufre et de laisser remonter vers -20°C en y maintenant le mélange pendant 1 heure. La température est de nouveau ramenée vers -50°C et on ajoute en une fois 13,95 g de (R,S)-2-bromopropionate d'éthyle. La température est ramenée aux environs de 20°C et on agite ainsi pendant 48 heures. Après addition d'eau distillée, le mélange est extrait deux fois avec de l'acétate d'éthyle. Les extraits organiques sont réunis, séchés sur sulfate de magnésium et concentrés à sec sous pression réduite (2 kPa). On obtient ainsi 30,1 g de (R,S)-2-(2-*tert*-butoxycarbonylamino-5-trifluorométhyl-phénylsulfanyl)-propionate d'éthyle, sous forme d'une huile ambrée qui est utilisée sans autre purification [RMN Spectre ¹H dans DMSO-d6, T=300K, δ en ppm (300 Mhz) : 1,08 (3H, t, J=6Hz, CH₃), 1,39 (3H, d, J=6Hz, CH₃), 1,50 (9H, s, (CH₃)₃), 4,00 (3H, m, SCH et OCH₂), 7,72 (1H, d, J=8Hz, CH arom.), 7,67 (1H, s, CH arom.), 8,05 (1H, d, J=8Hz, CH arom.), 8,52 (1H, s, NH)].

### EXEMPLE 28

On ajoute goutte à goutte, à une température voisine de 20°C et sous argon, 0,87 g de brome dilué dans 5 ml d'acide acétique à une solution de 1,2 g de thiocyanate de potassium et de 1,4 g de (R,S)-diméthyl(6-trifluorométhyl-1,2,3,4-tétrahydroquinol-2-ylméthyl)amine dans 16 ml d'acide acétique. Le mélange réactionnel est agité pendant 18 heures à la même température, versé sur de la glace, alcalinisé par une solution d'ammoniaque à 32% et extrait 2 fois avec au total 75 ml d'acétate d'éthyle. Les extraits organiques sont réunis, séchés sur du sulfate de magnésium et concentrés à sec sous pression réduite (4 kPa) à 40°C. Le produit brut est chromatographié sur colonne de gel de silice en éluant avec un mélange de dichlorométhane et de méthanol (99-1 puis 98-2 en volumes) pour donner une huile brune. Celle-ci est redissoute dans l'éther éthylique, décolorée au moyen de charbon activé puis additionnée d'une solution d'isopropanol chlorhydrique. On obtient 1 g de dichlorhydrate de (R,S)-diméthyl(2-imino-8-trifluorométhyl-5,6-dihydro-2H,4H-thiazolo[5,4,3-ij]quinol-4-ylméthyl)amine sous forme de solide crème fondant vers 210°C avec décomposition [Analyse C14H18Cl2F3N3S, % calculé C : 43,31, H : 4,67, Cl : 18,26, F : 14,68, N : 10,82, S : 8,26, % trouvé C : 43,0, H : 5,0, Cl : 18,0, F : 14,2, N : 10,5, S : 7,8].

La (R,S)-diméthyl(6-trifluorométhyl-1,2,3,4-tétrahydroquinol-2-ylméthyl)amine peut être préparée de la manière suivante : à une suspension de 1,4 g de diméthyl(6-trifluorométhylquinol-2-ylméthyl)amine et de 0,23 g de chlorure de nickel(II) hexahydraté dans 21 ml de méthanol, on ajoute sous argon et à une température voisine de 20°C, 0,84 g de tétrahydroborate de sodium en 30 minutes. Après 18 heures et 21 heures d'agitation à cette température on ajoute par 2 fois 0,12 g de chlorure de nickel(II) hexahydraté et 0,42 g de tétrahydroborate de sodium. Le milieu réactionnel est agité encore 18 heures après la dernière addition puis concentré sous pression réduite (4 kPa) à 40°C. Le résidu est repris par 30 ml d'une solution d'acide chlorhydrique à 10%, puis alcalinisé au moyen d'une solution d'ammmoniaque à 32% et extrait 2 fois par au total 150 ml d'acétate d'éthyle. Les extraits organiques sont réunis, séchés sur du sulfate de magnésium et concentrés à sec sous pression réduite (4 kPa) à 40°C. Le produit brut est chromatographié sur colonne de gel de silice en éluant avec un mélange de dichlorométhane et de méthanol (99-1 puis 98-2 en volumes). On obtient ainsi 1,5 g de (R,S)-diméthyl(6-trifluorométhyl-1,2,3,4-tétrahydroquinol-2-ylméthyl)amine sous forme d'une huile brune [RMN Spectre ¹H dans CDCl₃, T=300K, δ en ppm (250 Mhz) : 1,50 et 1,90 (1H chacun, m, CH₂), entre 2,20 et 2,50 (8H, m, CH₂ et N(CH₃)₂), 2,80 (2H, m, NCH₂), 3,40 (1H, m, NCH), 5,00 (1H, s, NH), 6,50 (1H, d, J=8Hz, CH arom.), 7,20 (2H, m, 2 CH arom.)].

La diméthyl(6-trifluorométhylquinol-2-ylméthyl)amine peut être préparée de la manière suivante : on agite, à une température voisine de 20°C et pendant 72 heures, une solution, dans 20 ml d'éthanol, de 1,9 g de 2-chlorométhyl-6-trifluorométhylquinoléine et de 27,8 ml d'une solution éthanolique à 36 g par litre de diméthylamine. Le milieu réactionnel est concentré sous pression réduite (4 kPa) à 40°C. Le résidu est repris par 30 ml d'une solution d'hydroxyde de sodium 0,25 N, puis extrait 2 fois par au total 45 ml d'acétate d'éthyle. Les extraits organiques sont réunis, séchés sur du sulfate de magnésium et concentrés à sec sous pression réduite (4 kPa) à 40°C. Le produit brut est chromatographié sur colonne de gel de silice en éluant avec un mélange de cyclohexane et d'acétate d'éthyle (50-50 en volumes). On obtient ainsi 1,4 g de diméthyl(6-trifluorométhylquinol-2-ylméthyl)amine sous forme d'une huile brune [RMN Spectre ¹H dans CDCl₃, T=300K, δ en ppm (250 Mhz) : 2,30 (6H, s, N(CH₃)₂), 3,70 (2H, s, NCH₂), 7,65 (1H, d, J=8Hz, CH arom.), 7,80 (1H, dd, J=8 et 2 Hz, CH arom.), 8,05 (1H, s, CH arom.), 8,15 (2H, m, CH arom.)].

La 2-chlorométhyl-6-trifluorométhylquinoléine peut être préparée de la manière suivante : on porte et maintient 24 heures à l'ébullition sous argon une suspension de 52,75 g de 2-méthyl-6-trifluorométhylquinoléine, de 33,2 g de N-chlorosuccinimide et de 1,1 g de peroxyde de benzoyle dans 500 ml de tétrachlorométhane. Après refroidissement vers 20°C, l'insoluble est éliminé par filtration, lavé par 20 ml de tétrachlorométhane et les filtrats réunis sont concentrés sous pression réduite (4 kPa) à 40°C. Le produit brut est chromatographié sur colonne de gel de silice en éluant avec un mélange de cyclohexane et d'acétate d'éthyle (97,5-2,5 en volumes). On obtient ainsi 24,1 g de 2-chlorométhyl-6-trifluorométhylquinoléine sous forme d'un solide orangé fondant à 66°C.

### EXEMPLE 29

On opère comme à l'exemple 28 mais à partir de 1,47 g de brome dans 4,7 ml d'acide acétique, 2,05 g de thiocyanate de potassium et 2,5 g de (R,S)-éthylméthyl(6-trifluorométhyl-1,2,3,4-tétrahydroquinol-2-ylméthyl)amine dans 25 ml d'acide acétique et en laissant réagir pendant 70 heures. Le produit brut est chromatographié sur colonne de gel de silice en éluant avec un mélange de dichlorométhane et de méthanol (98,75-1,25 en volumes) pour donner une huile orangée. Celle-ci est redissoute dans 30 ml d'éther éthylique, décolorée au moyen de charbon activé puis additionnée d'une solution d'isopropanol chlorhydrique. On obtient 1,4 g de dichlorhydrate de (R,S)-éthylméthyl(2-imino-8-trifluorométhyl-5,6-dihydro-2H,4H-thiazolo[5,4,3-ij]quinol-4-ylméthyl)amine sous forme de solide crème fondant vers 280°C avec décomposition [RMN Spectre ¹H dans DMSO-d6, T=300K, δ en ppm (300 Mhz) : 1,30 (3H, t, J=6Hz, CH₃), 2,20 et2,75 (1H chacun, m, CH₂), 2,95 (3H,s, NCH₃), entre 3,00 et 3,60 (6H, m, CH₂ et 2 NCH₂), 5,45 (1H, m, NCH), 7,85 (1H, s, CH arom.), 8,30 (1H, s, CH arom.)].

La (R,S)-éthylméthyl(6-trifluorométhyl-1,2,3,4-tétrahydroquinol-2-ylméthyl)amine peut être préparée en opérant comme dans l'exemple 28, mais à partir de 3,4 g d'éthylméthyl(6-trifluorométhylquinol-2-yl)amine, de 3 fois 0,52 g de chlorure de nickel(II) hexahydraté dans 70 ml de méthanol et de 3 fois 1,9 g de tétrahydroborate de sodium, puis en hydrolysant par 100 g de glace. Le produit brut est chromatographié sur colonne de gel de silice en éluant avec un mélange de dichlorométhane et de méthanol (97,5-2,5 en volumes). On obtient ainsi 2,5 g de (R,S)-éthylméthyl(6-trifluorométhyl-1,2,3,4-tétrahydroquinol-2-ylméthyl)amine sous forme d'une huile orangée [RMN Spectre ¹H dans CDCl₃, T=300K, δ en ppm (300 Mhz) : 1,05 (3H, t, J=6Hz, CH₃), 1,45 et 1,85 (1H chacun, m, CH₂), 2,25 (3H,s, NCH₃), entre 2,25 et 2,90 (6H, m, CH₂ et 2 NCH₂), 3,40 (1H, m, NCH), 5,00 (1H, s, NH), 6,50 (1H, d, J=8Hz, CH arom.), 7,15 (2H, m, 2 CH arom.)].

L'éthylméthyl(6-trifluorométhylquinol-2-yl)amine peut être préparée de la manière suivante : on agite pendant 24 heures, sous azote et à une température voisine de 20°C, une solution de 3,5 g de 2-chlorométhyl-6-trifluorométhylquinoléine et de 2,67 g d'éthylméthylamine dans 35 ml d'éthanol. Le mélange réactionnel est additionné de 70 ml d'eau distillée, puis extrait deux fois par 105 ml au total d'acétate d'éthyle et les extraits organiques réunis sont séchés sur sulfate de magnésium et concentrés à sec sous pression réduite (4 kPa) à 40°C. Le résidu obtenu est chromatographié sur colonne de gel de silice en éluant avec un mélange d'acétate d'éthyle et de cyclohexane (75-25 en volumes). On obtient ainsi 3,4 g d'éthylméthyl(6-trifluorométhylquinol-2-yl)amine sous forme d'une huile brune [RMN Spectre ¹H dans CDCl₃, T=300K, δ en ppm (250 Mhz) : 1,15 (3H, t, J=6Hz, CH₃), 2,30 (3H,s, NCH₃), 2,55 (2H, q, J=6Hz, NCH₂), 3,85 (2H, s, NCH₂), 7,75 (1H, d, J=8Hz, CH arom.), 7,87 (1H, dd, J=8 et 2Hz, CH arom.), 8,10 (1H, s, CH arom.), 8,20 (2H, m, 2 CH arom.)].

### EXEMPLE 30

On opère comme à l'exemple 28 mais à partir de 1,13 g de brome dans 3 ml d'acide acétique, 1,6 g de thiocyanate de potassium et 2,8 g de (R,S)-2-[4-(4-fluorophényl)-pipérazin-1-ylméthyl]-6-trifluorométhyl-1,2,3,4-tétrahydroquinoléine dans 13 ml d'acide acétique. Le produit brut est chromatographié sur colonne de gel de silice en éluant avec un mélange de dichlorométhane et de méthanol (99-1 en volumes). On obtient 1,4 g de (R,S)-4-[4-(4-fluorophényl)-pipérazin-1-ylméthyl]-2-imino-8-trifluorométhyl-5,6-dihydro-2H,4H-thiazolo[5,4,3-ij]quinoléine sous forme de solide jaune fondant à 60°C [Analyse C22H22F4N4S, % calculé C : 58,66, H : 4,92, F : 16,87, N : 12,44, S : 7,12, % trouvé C : 58,70, H : 4,98, F : 16,61, N : 12,43, S : 6,88].

La (R,S)-2-[4-(4-fluorophényl)-pipérazin-1-ylméthyl]-6-trifluorométhyl-1,2,3,4-tétrahydroquinoléine peut être préparée en opérant comme dans l'exemple 28, mais à partir de 4,4 g de 2-[4-(4-fluorophényl)-pipérazin-1-ylméthyl]-6-trifluorométhylquinoléine, de 9,4 g de chlorure de nickel(II) hexahydraté dans 50 ml de méthanol et de 3,4 g de tétrahydroborate de sodium. Le produit brut est chromatographié sur colonne de gel de silice en éluant avec du dichlorométhane. On obtient ainsi 2,8 g de (R,S)-2-[4-(4-fluorophényl)-pipérazin-1-ylméthyl]-6-trifluorométhyl-1,2,3,4-tétrahydroquinoléine sous forme de cristaux crèmes fondant à 157°C.

La 2-[4-(4-fluorophényl)-pipérazin-1-ylméthyl]-6-trifluorométhylquinoléine peut être préparée de la manière suivante : on agite pendant 70 heures, à une température voisine de 20°C, une solution de 3 g de 2-chlorométhyl-6-trifluorométhylquinoléine et de 6,6 g de 1-(4-fluorophényl)-pipérazine dans 80 ml d'éthanol. Le mélange réactionnel est concentré à sec sous pression réduite (4 kPa) à 40°C. Le résidu est traité par 50 ml d'eau distillée et 10 ml d'ammoniaque à 32%, puis extrait deux fois par 150 ml au total d'acétate d'éthyle. Les extraits organiques réunis sont séchés sur sulfate de magnésium et concentrés à sec sous pression réduite (4 kPa) à 40°C. Le résidu obtenu est chromatographié sur colonne de gel de silice en éluant avec un mélange de dichlorométhane et de méthanol (98-2 en volumes). On obtient ainsi 4,4 g de 2-[4-(4-fluorophényl)-pipérazin-1-ylméthyl]-6-trifluorométhylquinoléine sous forme d'un solide brun fondant à 113°C.

### EXEMPLE 31

On opère comme à l'exemple 28 mais à partir de 0,85 g de brome dans 5 ml d'acide acétique, 1,18 g de thiocyanate de potassium et 1,5 g de (R,S)-2-(pyrrolidin-1-ylméthyl)-6-trifluorométhyl-1,2,3,4-tétrahydroquinoléine dans 16 ml d'acide acétique. Le produit brut est chromatographié sur colonne de gel de silice en éluant avec un mélange de dichlorométhane et de méthanol (99-1 en volumes) pour donner une huile jaune. Celle-ci est redissoute dans l'éther éthylique, décolorée au moyen de charbon activé puis additionnée d'une solution d'isopropanol chlorhydrique. On obtient 1,1 g de dichlorhydrate de (R,S)-2-imino-4-(pyrrolidin-1-ylméthyl)-8-trifluorométhyl-5,6-dihydro-2H,4H-thiazolo[5,4,3-ij]quinoléine sous forme de solide crème fondant vers 270°C avec décomposition [Analyse C16H20Cl2F3N3S, % calculé C : 46,38, H : 4,87, Cl : 17,11, F : 13,76, N : 10,14, S : 7,74, % trouvé C : 46,4, H : 4,6, Cl : 16,8, F : 13,6, N : 10,1, S : 8,1].

La (R,S)-2-(pyrrolidin-1-ylméthyl)-6-trifluorométhyl-1,2,3,4-tétrahydroquinoléine peut être préparée en opérant comme dans l'exemple 28, mais à partir de 2,5 g de 2-(pyrrolidin-1-ylméthyl)-6-trifluorométhylquinoléine, de 3 fois 0,74 g de chlorure de nickel(II) hexahydraté dans 40 ml de méthanol et de 3 fois 2,7 g de tétrahydroborate de sodium. Le produit brut est chromatographié sur colonne de gel de silice en éluant avec un mélange de dichlorométhane et de méthanol (99-1 en volumes). On obtient ainsi 1,6 g de (R,S)-2-(pyrrolidin-1-ylméthyl)-6-trifluorométhyl-1,2,3,4-tétrahydroquinoléine sous forme d'une huile jaune [RMN Spectre ¹H dans DMSO-d6, T=300K, δ en ppm (300 Mhz) : 1,50 et 1,95 (1H chacun, m, CH₂), 1,80 (4H, m, 2 CH₂), entre 2,40 et 2,65 (6H, m, 3 NCH₂), 2,75 (2H, m, CH₂), 3,45 (1H, m, NCH), 6,20 (1H, s, NH), 6,70 (1H, d, J=8Hz, CH arom.), 7,20 (2H, m, 2 CH arom.)].

La 2-(pyrrolidin-1-ylméthyl)-6-trifluorométhylquinoléine peut être préparée en opérant comme dans l'exemple 29, mais à partir de 3 g de 2-chlorométhyl-6-trifluorométhylquinoléine et de 2,6 g de pyrrolidine dans 30 ml d'éthanol. Le produit brut obtenu est chromatographié sur colonne de gel de silice en éluant avec un mélange de dichlorométhane et de méthanol (99-1 en volumes) . On obtient ainsi 2,5 g de 2-(pyrrolidin-1-ylméthyl)-6-trifluorométhylquinoléine sous la forme d'une huile orangée [RMN Spectre ¹H dans CDCl₃, T=300K, δ en ppm (300 Mhz) : 2,15 (4H, m, 2 CH₂), 3,30 (4H, m, 2 NCH₂), 4,50 (2H, s, NCH₂), 7,90 (1H, d, J=8Hz, CH arom.), 8,15 (2H, m, 2 CH arom.), 8,25 (1H, d, J=8Hz, CH arom.), 8,35 (1H, d, J=8Hz, CH arom.)].

### EXEMPLE 32

On opère comme à l'exemple 28 mais à partir de 0,64 g de brome dans 3,7 ml d'acide acétique, 0,89 g de thiocyanate de potassium et 1,2 g de (R,S)-2-(morpholin-4-ylméthyf)-6-trifluorométhyl-1,2,3,4-tétrahydroquinoléine dans 12 ml d'acide acétique. Le produit brut est chromatographié sur colonne de gel de silice en éluant avec un mélange de dichlorométhane et de méthanol (99-1 en volumes) pour donner une huile brune. Celle-ci est redissoute dans l'éther éthylique, décolorée au moyen de charbon activé puis additionnée d'une solution d'isopropanol chlorhydrique. On obtient 0,8 g de dichlorhydrate de (R,S)-2-imino-4-(morpholin-4-ylméthyl)-8-trifluorométhyl-5,6-dihydro-2H,4H-thiazolo[5,4,3-ij]quinoléine sous forme de solide crème fondant vers 263°C avec décomposition [Analyse C16H20Cl2F3N3OS, % calculé C : 44,66, H : 4,68, Cl : 16,48, F : 13,24, N : 9,76, O : 3,72, S : 7,45, % trouvé C : 45,1, H : 4,8, Cl : 15,9, F : 12,9, N : 9,7, S : 7,1].

La (R,S)-2-(morpholin-4-ylméthyl)-6-trifluorométhyl-1,2,3,4-tétrahydroquinoléine peut être préparée en opérant comme dans l'exemple 28, mais à partir de 3,5 g de 2-(morpholin-4-ylméthyl)-6-trifluorométhylquinoléine, de 0,98 g de chlorure de nickel(II) hexahydraté dans 50 ml de méthanol et de 3,6 g de tétrahydroborate de sodium. Le produit brut est chromatographié sur colonne de gel de silice en éluant avec un mélange de dichlorométhane et de méthanol (99-1 en volumes). On obtient ainsi 0,8 g de (R,S)-2-(morpholin-4-ylméthyl)-6-trifluorométhyl-1,2,3,4-tétrahydroquinoléine sous forme d'une huile brune [RMN Spectre ¹H dans DMSO-d6, T=300K, δ en ppm (300 Mhz) : 1,50 et 1,90 (1H chacun, m, CH₂), entre 2,30 et 2,50 (6H, m, 3 NCH₂), 2,70 (2H, m, CH₂), 3,50 (1H, m, NCH), 3,65 (4H, m, 2 OCH₂), 6,10 (1H, s, NH), 6,65 (1H, d, J=8Hz, CH arom.), 7,15 (2H, m, 2 CH arom.)].

La 2-(morpholin-4-ylméthyl)-6-trifluorométhylquinoléine peut être préparée en opérant comme dans l'exemple 29, mais à partir de 3 g de 2-chlorométhyl-6-trifluorométhylquinoléine et de 3,18 g de morpholine dans 30 ml d'éthanol. Le produit brut obtenu est chromatographié sur colonne de gel de silice en éluant avec un mélange de dichlorométhane et de méthanol (97,5-2,5 en volumes) . On obtient ainsi 3,5 g de 2-(morpholin-4-ylméthyl)-6-trifluorométhylquinoléine sous la forme d'une huile orangée [RMN Spectre ¹H dans CDCl₃, T=300K, δ en ppm (300 Mhz) : 2,55 (4H, m, 2 NCH₂), 3,75 (4H, m, 2 OCH₂), 3,85 (2H, s, NCH₂), 7,75 (1H, d, J=8Hz, CH arom.), 7,85 (1H, dd, J=8 et 2 Hz, CH arom.), entre 8,10 et 8,25 (3H, m, 3 CH arom.)].

### EXEMPLE 33

On porte et maintient 74 heures à l'ébullition une solution de 0,3 g de (R,S)-2-éthylsulfonylméthyl-8-thiocyanato-6-trifluorométhyl-1,2,3,4-tétrahydroquinoléine dans 10 ml d'éthanol. Le milieu réactionnel est concentré sous pression réduite (4 kPa) à 40°C. Le résidu est chromatographié sur colonne de gel de silice en éluant avec du dichlorométhane pour donner une meringue jaune. Celle-ci est redissoute dans 20 ml de dichlorométhane et la solution est décolorée au moyen de charbon activé puis concentrée sous vide. On obtient 0,2 g de (R,S)-4-éthylsulfonylméthyl-2-imino-8-trifluorométhyl-5,6-dihydro-2H,4H-thiazolo[5,4,3-ij]quinoléine sous forme d'une meringue jaunâtre fondant à 140°C [Analyse C14H15F3N2O2S2, % calculé C : 46,14, H : 4,15, F : 15,64, N : 7,69, O : 8,78, S : 17,60 % trouvé C : 46,00, H : 3,84, F : 15,20, N : 7,51, S : 17,71].

La (R,S)-2-éthylsulfonylméthyl-8-thiocyanato-6-trifluorométhyl-1,2,3,4-tétrahydroquinoléine peut être préparée de la manière suivante : on ajoute goutte à goutte, à une température voisine de 20°C et sous argon, 0,208 g de brome dilué dans 0,65 ml d'acide acétique à une solution de 0,29 g de thiocyanate de potassium et de 0,4 g de (R,S)-2-éthylsulfonylméthyl-6-trifluorométhyl-1,2,3,4-tétrahydroquinoléine dans 4 ml d'acide acétique. Le mélange réactionnel est agité pendant 18 heures à la même température, versé sur de la glace et alcalinisé par une solution d'ammoniaque à 32%. L'insoluble est éliminé par filtration, puis le filtrat est extrait 2 fois avec au total 75 ml d'acétate d'éthyle. Les extraits organiques sont réunis, séchés sur du sulfate de magnésium et concentrés à sec sous pression réduite (4 kPa) à 40°C. Le produit brut est chromatographié sur colonne de gel de silice en éluant avec du dichlorométhane. On obtient 0,2 g de (R,S)-2-éthylsulfonylméthyl-8-thiocyanato-6-trifluorométhyl-1,2,3,4-tétrahydroquinoléine sous forme de cristaux jaune pâle fondant à 137°C.

La (R,S)-2-éthylsulfonylméthyl-6-trifluorométhyl-1,2,3,4-tétrahydroquinoléine peut être préparée de la manière suivante : 1,3 g de 2-éthylsulfonylméthyl-6-trifluorométhylquinoléine-1-oxyde avec 0,22 g d'oxyde de platine dans 14 ml d'un mélange méthanol-tétrahydrofurane (50-50 en volumes) sont hydrogénés à une température voisine de 20°C sous une pression de 5 bar pendant 1,5 heure. Après filtration du milieu réactionnel, la phase organique est concentrée sous pression réduite (4 kPa) à 40°C. Le produit brut est chromatographié d'abord sur colonne de gel de silice en éluant avec un mélange de dichlorométhane et de méthanol (98,75-1,25 en volumes), puis sur colonne de gel de silice en éluant avec du dichlorométhane. On obtient ainsi 0,7 g de (R,S)-2-éthylsulfonylméthyl-6-trifluorométhyl-1,2,3,4-tétrahydroquinoléine sous forme de cristaux jaunes fondant à 85°C.

Le 2-éthylsulfonylméthyl-6-trifluorométhylquinoléine-1-oxyde peut être préparé de la manière suivante : à une solution, sous argon et refroidie à 5°C, de 2 g de 2-éthylsulfanylméthyl-6-trifluorométhylquinoléine dans 80 ml de dichlorométhane, on ajoute petit à petit 6,2 g d'acide 3-chloroperbenzoïque (pureté 80%) et on laisse agiter 16 heures à une température voisine de 20°C. On ajoute ensuite 100 ml d'une solution aqueuse saturée d'hydrogénocarbonate de sodium ainsi que 30 ml de dichlorométhane et poursuit l'agitation jusqu'à fin du dégagement gazeux. Après séparation des deux phases, on extrait la phase aqueuse par 50 ml de dichlorométhane et les extraits organiques réunis sont séchés sur sulfate de magnésium et concentrés à sec sous pression réduite (4 kPa) à 20°C. Le solide obtenu est chromatographié sur colonne de gel de silice en éluant avec un mélange de dichlorométhane et de méthanol (97,5-2,5 en volumes). On obtient ainsi 1,3 g de 2-éthylsulfonylméthyl-6-trifluorométhylquinoléine-1-oxyde sous forme de cristaux jaunes fondant à 166°C.

La 2-éthylsulfanylméthyl-6-trifluorométhylquinoléine peut être préparée de la manière suivante : à une suspension dans 5 ml de diméthylformamide, sous argon et refroidie à 5°C, de 1,2 g d'une dispersion à 60 % d'hydrure de sodium dans l'huile minérale est ajoutée en 20 minutes une solution de 1,9 g d'éthanethiol dans 20 ml de diméthylformamide. Le milieu réactionnel est ensuite agité 1 heure vers 20°C, puis après refroidissement vers 5°C, est additionné en 1 heure d'une solution de 6,7 g de 2-chlorométhyl-6-trifluorométhylquinoléine dans 50 ml de diméthylformamide. Le mélange est agité 16 heures vers 20°C, puis hydrolysé vers 5°C par 100 ml d'eau distillée. Il est ensuite extrait deux fois par 75 ml au total d'acétate d'éthyle et les extraits organiques réunis sont séchés sur sulfate de magnésium et concentrés à sec sous pression réduite (4 kPa) à 40°C. Le résidu obtenu est chromatographié sur colonne de gel de silice en éluant avec du dichlorométhane. On obtient ainsi 5,6 g de 2-éthylsulfanylméthyl-6-trifluorométhylquinoléine sous forme d'une huile brune [RMN Spectre ¹H dans CDCl₃, T=300K, δ en ppm (250 Mhz) : 1,20 (3H, t, J=6Hz, CH₃), 2,48 (2H, q, J=6Hz, SCH₂), 4,00 (2H, s, SCH₂), 7,65 (1H, d, J=8Hz, CH arom.), 7,85 (1H, dd, J=8 et 2 Hz, CH arom.), 8,10 (2H, m, 2 CH arom.), 8,20 (1H, d, J=8Hz, CH arom.)].

### EXEMPLE 34

On coule goutte à goutte, en 2 minutes, une solution de 0,40 g de brome dans 1 ml d'acide acétique sur une suspension de 0,79 g de sélénocyanate de potassium dans 10 ml d'acide acétique. Après agitation 1 heure à environ 20°C, on coule une solution de 0,5 g de 6-trifluorométhyl-1,2,3,4-tétrahydroquinoléine dans 1 ml d'acide acétique et on agite 3 heures à environ 20°C. Le milieu réactionnel est ensuite coulé sur un mélange de 20 g de glace et 20 ml d'eau distillée. On élimine un insoluble par filtration, le filtrat est alcalinisé avec 15 ml d'une solution aqueuse d'ammoniaque à 28% et extrait trois fois par 50 ml de dichlorométhane. Les extraits organiques réunis sont lavés avec 50 ml d'eau distillée, séchés sur sulfate de magnésium, filtrés et concentrés à sec sous pression réduite (2 kPa). On met en suspension 0,25 g du produit obtenu (sur 0,34 g) dans 7,5 ml au total d'éther éthylique auxquels on ajoute goutte à goutte 1 ml d'éther chlorhydrique environ 3,8 M. Après 10 minutes d'agitation à environ 20°C, on sépare le produit par filtration, on lave avec deux fois 2,5 ml d'éther éthylique et on sèche à l'air. On obtient ainsi 0,25 g de chlorhydrate de 2-imino-8-trifluorométhyl-5,6-dihydro-2H,4H-sélénazolo[5,4,3-ij]quinoléine, sous forme d'un solide jaune fondant à 172°C [Analyse C11H10ClF3N2Se, % calculé C : 38,67, H : 2,95, Cl : 10,38, F : 16,68, N : 8,20, % trouvé C : 37,82, H : 3,00, Cl : 10,47, F : 14,77, N : 7,72, H2O : 3,6].

### EXEMPLE 35

On agite, pendant 7 heures à une température voisine de 20°C, un mélange de 1 g de (R,S)-4-[4-(4-fluorophényl)pipérazin-1-ylméthyl]-2-trifluorométhylcarbonylimino-8-trifluorométhoxy-5,6-dihydro-4H-thiazolo[5,4,3-ij]quinoléine, 10 ml d'une solution aqueuse de carbonate de potassium à 10%, 10 ml d'acétone et 10 ml de méthanol. Le mélange est hydrolysé par 50 ml d'eau distillée et extrait deux fois par 50 ml et 25 ml d'acétate d'éthyle. Les extraits organiques réunis sont séchés sur sulfate de magnésium, filtrés et concentrés à sec sous pression réduite (2 kPa). Le résidu obtenu est purifié par chromatographie sur colonne de gel de silice en éluant avec un mélange de dichlorométhane et de méthanol (98,75-1,25 en volumes). On obtient ainsi 0,7 g de (R,S)-4-[4-(4-fluorophényl)pipérazin-1-ylméthyl]-2-imino-8-trifluorométhoxy-5,6-dihydro-2H,4H-thiazolo[5,4,3-ij]quinoléine, sous forme d'un solide crème fondant à 52°C [Analyse C22H22F4N4OS, % calculé C : 56,64, H : 4,75, F : 16,29, N : 12,01, O : 3,43, S : 6,87, % trouvé C : 56,7, H : 4,8, F : 16,1, N : 11,9, S : 6,8].

La (R,S)-4-[4-(4-fluorophényl)pipérazin-1-ylméthyl]-2-trifluorométhylcarbonylimino-8-trifluorométhoxy-5,6-dihydro-4H-thiazolo[5,4,3-ij]quinoléine peut être préparée de la manière suivante : à une solution de 0,54 g de (R,S)-2-trifluorométhylcarbonylimino-4-trifluorométhylsulfométhyl-8-trifluorométhoxy-5,6-dihydro-4H-thiazolo[5,4,3-ij]quinoléine dans 10 ml de dichlorométhane, maintenue sous argon et à environ 0°C, on ajoute goutte à goutte une solution de 0,36 g de 1-(4-fluorophényl)pipérazine dans 3,6 ml de dichlorométhane anhydre. On agite 15 minutes à une température voisine de 0°C puis on laisse revenir progressivement à une température voisine de 20°C en laissant agiter ainsi 16 heures. Le milieu est hydrolysé par 20 ml d'eau distillée, décanté et la phase aqueuse est extraite une fois par 15 ml de dichlorométhane. Les extraits organiques réunis sont séchés sur sulfate de magnésium, concentrés à sec sous pression réduite (2 kPa). Le résidu obtenu est purifié par chromatographie sur colonne de gel de silice en éluant avec du diclorométhane. On obtient ainsi 0,2 g de (R,S)-4-[4-(4-fluorophényl)pipérazin-1-ylméthyl]-2-trifluorométhylcarbonylimino-8-trifluorométhoxy-5,6-dihydro-4H-thiazolo[5,4,3-ij]quinoléine, sous forme d'un solide crème fondant à 163°C.

La (R,S)-2-trifluorométhylcarbonylimino-4-trifluorométhylsulfométhyl-8-trifluorométhoxy-5,6-dihydro-4H-thiazolo[5,4,3-ij]quinoléine peut être préparée de la manière suivante : sur une solution, maintenue à -15°C sous argon, de 2,5 ml de dichlorométhane et 0,1 g de pyridine, on coule goutte à goutte une suspension de 0,304 g de (R,S)-2-trifluorométhylcarbonylimino-8-trifluorométhoxy-5,6-dihydro-4H-thiazolo[5,4,3-ij]quinoléine-4-méthanol dans 2,5 ml de dichlorométhane puis une solution de 0,35 g d'anhydride triflique dans 2 ml de dichlorométhane. Le mélange est maintenu sous agitation à -15°C pendant 15 minutes puis ramené progressivement à température voisine de 0°C où il est maintenu ainsi pendant 2 heures. L'insoluble est éliminé par filtration sur un lit de célite 545 et de sulfate de magnésium, lavé par 2,5 ml de dichlorométhane et le filtrat ainsi obtenu de (R,S)-2-trifluorométhylcarbonylimino-4-trifluorométhylsulfométhyl-8-trifluorométhoxy-5,6-dihydro-4H-thiazolo[5,4,3-ij]quinoléine est directement mis en réaction dans l'étape suivante.

Le (R,S)-2-trifluorométhylcarbonylimino-8-trifluorométhoxy-5,6-dihydro-4H-thiazolo[5,4,3-ij]quinoléine-4-méthanol peut être préparée de la manière suivante : sur une suspension de 0,6 g de (R,S)-2-imino-8-trifluorométhoxy-5,6-dihydro-2H,4H-thiazolo[5,4,3-ij]quinoléine-4-méthanol dans 12 ml de dichlorométhane anhydre, maintenue sous argon et à une température voisine de 20°C, on coule goutte à goutte 1,26 g d'anhydride trifluoroacétique et on poursuit l'agitation 5 heures à la même température. Le milieu est ensuite neutralisé par 1,5 ml de triéthylamine puis hydrolysé par 25 ml d'eau distillée à une température voisine de 20°C. La phase aqueuse est extraite par 20 ml de dichlorométhane et les extraits organiques réunis sont séchés sur sulfate de magnésium, concentrés à sec sous pression réduite (2 kPa). Le résidu obtenu est purifié par chromatographie sur colonne de gel de silice en éluant avec un mélange de dichlorométhane et de méthanol (99-1 en volumes). On obtient ainsi 0,7 g de (R,S)-2-trifluorométhylcarbonylimino-8-trifluorométhoxy-5,6-dihydro-4H-thiazolo[5,4,3-ij]quinoléine-4-méthanol, sous forme d'un solide crème fondant à 156°C.

### EXEMPLE 36

On opère comme à l'exemple 23 mais à partir de 1,24 g de brome dilué dans 0,4 ml d'acide acétique, 1,73 g de thiocyanate de potassium et de 1,8 g de (R,S)-6-trifluorométhyl-1,2,3,4-tétrahydroquinoléine-3-méthanol dans 22 ml d'acide acétique. Le produit obtenu est purifié par chromatographie sur colonne de gel de silice en éluant avec un mélange de dichlorométhane et de méthanol (97,5-2,5 en volumes). On obtient ainsi 0,8 g de (R,S)-2-imino-8-trifluorométhyl-5,6-dihydro-2H,4H-thiazolo[5,4,3-ij]quinoléine-5-méthanol, sous forme d'un solide crème fondant à 177°C [Analyse C12H11F3N2OS, %calculé C : 50,0, H : 3,85, F : 19,77, N : 9,72, O : 5,55, S : 11,12 % trouvé C : 49,67, H : 3,49].

Le (R,S)-6-trifluorométhyl-1,2,3,4-tétrahydroquinoléine-3-méthanol peut être préparé de la manière suivante : sur une suspension, maintenue sous argon et à environ 0°C, de 0,38 g de tétrahydroaluminate de lithium dans 3,8 ml de tétrahydrofurane anhydre, on coule goutte à goutte une solution de 2,73 g de 6-trifluorométhyl-1,2,3,4-tétrahydroquinoléine-3-carboxylate d'éthyle dans 27 ml de tétrahydrofurane anhydre. Le mélange est agité 1 heure 30 minutes vers 5°C puis 16 heures à une température voisine de 20°C. Le milieu réactionnel est ensuite refroidi vers 5°C puis hydrolysé lentement avec 30 ml d'eau distillée, et le produit est extrait deux fois par 50 ml puis 25 ml d'acétate d'éthyle. Les extraits organiques réunis sont séchés sur sulfate de magnésium, filtrés et concentrés à sec sous pression réduite (2 kPa). Le produit obtenu (2,2 g) est purifié par chromatographie sur colonne de gel de silice en éluant avec un mélange de dichlorométhane et de méthanol (97,5-2,5 en volumes). On obtient ainsi 1,8 g de (R,S)-6-trifluorométhyl-1,2,3,4-tétrahydroquinoléine-3-méthanol, sous forme d'une huile jaune [RMN Spectre ¹H dans CDCl₃, T=300K, δ en ppm (250 Mhz) : 1,70 (1H, s, NH), 2,20 (1H, m, CH), 2,55 et 2,85 (1H chacun, respectivement dd, J=16 et 8 Hz, et dd, J=16 et 4 Hz, CH₂-aryle), 3,18 et 3,50 (1H chacun, respectivement dd, J=10 et 8 Hz, et dd, J=10 et 2 Hz, NCH₂), 3,65 (2H, m, OCH₂), 4,20 (1H, s, OH), 6,50 (1H, d, J=8Hz, CH arom.), 7,20 (2H, m, 2 CH arom.)].

Le 6-trifluorométhyl-1,2,3,4-tétrahydroquinoléine-3-carboxylate d'éthyle peut être préparé de la manière suivante : 30 g de 4-chloro-6-trifluorométhylquinoléine-3-carboxylate d'éthyle avec 2,1 g de palladium sur charbon à 10% dans 300 ml d'acide acétique sont hydrogénés à une température voisine de 20°C sous une pression de 5 bars pendant 16 heures. Le milieu réactionnel est coulé sur 400 g de glace et alcalinisé par une solution d'ammoniaque aqueuse à 28% jusqu'à un pH de 11. Le produit est extrait par 500 ml d'acétate d'éthyle et l'extrait organique est filtré sur un lit de clarcel qui est lavé par de l'eau. La phase aqueuse du filtrat est extraite par 250 ml d'acétate d'éthyle et les extraits organiques réunis sont séchés sur sulfate de magnésium, filtrés et concentrés à sec sous pression réduite (2 kPa). Le résidu obtenu est purifié par chromatographie sur colonne de gel de silice en éluant avec du dichlorométhane. On obtient ainsi 3,1 g de 6-trifluorométhyl-1,2,3,4-tétrahydroquinoléine-3-carboxylate d'éthyle sous forme de cristaux jaunes fluorescents, fondant à 69°C.

Le 4-chloro-6-trifluorométhylquinoléine-3-carboxylate d'éthyle peut être préparé selon C. J. Matson et coll., J. Med. Chem., 22, (7), 816-823 (1979).

### EXEMPLE 37

On opère comme à l'exemple 23 mais à partir de 1,1 g de brome dans 3,5 ml d'acide acétique, 1,6 g de 7-trifluorométhoxy-3,4-dihydro-1H-quinoxalin-2-one et 1,54 g de thiocyanate de potassium dans 20 ml d'acide acétique. Le produit obtenu (1,8 g) est purifié par chromatographie sur colonne de gel de silice en éluant avec un mélange de dichlorométhane et de méthanol (99-1 en volumes). On obtient ainsi 0,3 g de 2-imino-8-trifluorométhoxy-2H,4H-thiazolo[3,4,5-de]quinoxalin-5(6H)-one sous forme d'un solide jaune fondant à 255°C [Analyse C10H6F3N3O2S, % calculé C : 41,53, H : 2,09, F : 19,71, N : 14,53, O : 11,06, S : 11, 09, % trouvé C : 41,00, H : 1,7, F : 19,2, N : 14,50, S : 11,6].

la 7-trifluorométhoxy-3,4-dihydro-1H-quinoxalin-2-one peut être préparée de la manière suivante : sur une suspension de 0,92 g de N-(2-nitro-4-trifluorométhoxyphényl)glycinate d'éthyle, 1,36 g d'étain en grenaille dans 2,3 ml d'éthanol, on coule goutte à goutte, à une température voisine de 20°C, 3 ml d'acide chlorhydrique à 36%. Le milieu réactionnel est ensuite chauffé 2 heures au reflux puis, après retour à une température voisine de 20°C, dilué avec 6 ml d'eau distillée et neutralisé par du carbonate de sodium solide. Le produit est extrait par 40 ml d'acétate d'éthyle puis le tout est filtré sur un lit de clarcel, lavé par 10 ml d'acétate d'éthyle. La phase aqueuse du filtrat est ensuite extraite par 10 ml d'acétate d'éthyle et les extraits organiques réunis sont séchés sur sulfate de magnésium, filtrés et concentrés à sec sous pression réduite (2 kPa). Le produit obtenu (0,7 g) est purifié par chromatographie sur colonne de gel de silice en éluant avec un mélange de cyclohexane et d'acétate d'éthyle (75-25 en volumes). On obtient ainsi 0,3 g de 7-trifluorométhoxy-3,4-dihydro-1H-quinoxalin-2-one sous forme d'un solide jaune clair fondant à 140°C.

Le N-(2-nitro-4-trifluorométhoxyphényl)glycinate d'éthyle peut être préparé de la manière suivante : on ajoute à 17,3 g d'acide sulfurique à 95%, maintenu à 5°C, 4,7 g de N-(4-trifluorométhoxyphényl)-N-tosylglycinate d'éthyle sur lesquels on coule, au goutte à goutte en maintenant à une température comprise entre 5° et 10°C, une solution de 1,12 g d'acide nitrique à 94% dans 2 ml d'acide sulfurique à 95%. Le mélange est agité 1 heure à 10°C puis agité 16 heures à une température voisine de 20°C. Le milieu réactionnel est hydrolysé par 50 g de glace et extrait deux fois par 50 ml et 25 ml d'acétate d'éthyle. Les extraits organiques réunis sont séchés sur sulfate de magnésium, filtrés et concentrés à sec sous pression réduite (2 kPa). Le produit obtenu est purifié par chromatographie sur colonne de gel de silice en éluant avec un mélange de cyclohexane et d'acétate d'éthyle (95-5 en volumes). On obtient ainsi 0,9 g de N-(2-nitro-4-trifluorométhoxyphényl)glycinate d'éthyle sous forme d'un solide orangé fondant à 94°C.

Le N-(4-trifluorométhoxyphényl)-N-tosylglycinate d'éthyle peut être préparé de la manière suivante : sur une suspension de 0,44 g d'hydrure de sodium (à 60% dans l'huile de vaseline) dans 5 ml de tétrahydrofurane anhydre, maintenue sous argon, on coule goutte à goutte une solution de 3,31 g de N-tosyl-4-trifluorométhoxyaniline dans 33 ml de tétrahydrofurane anhydre puis on dilue le milieu avec 30 ml de tétrahydrofurane anhydre. Après 1 heure d'agitation à une température voisine de 20°C, on coule une solution de 1,84 g de bromoacétate d'éthyle dans 12 ml de tétrahydrofurane anhydre et on agite 16 heures à une température voisine de 20°C. Le milieu est hydrolysé par 50 ml d'eau distillée et extrait deux fois par 50 ml et 25 ml d'acétate d'éthyle. Les extraits organiques réunis sont séchés sur sulfate de magnésium, filtrés et concentrés à sec sous pression réduite (2 kPa). Le produit obtenu est purifié par chromatographie sur colonne de gel de silice en éluant avec un mélange de cyclohexane et d'acétate d'éthyle (87,5-12,5 en volumes). On obtient ainsi 3,7 g de N-(4-trifluorométhoxyphényl)-N-tosylglycinate d'éthyle sous forme d'une huile jaune [ RMN Spectre ¹H dans CDCl₃, T=300K, δ en ppm (300 MHz) : 1,15 (3H, t, J=6Hz, CH₃), 2,35 (3H, s, CH₃Ph), 4,10 (2H, q, J=6Hz, OCH₂), 4,30 (2H, s, NCH₂CO), 7,08 (2H, d, J=7Hz, CH arom.), 7,20 (4H, m, 4 CH arom.), 7,50 (2H, d, J=7Hz, CH arom.].

La N-tosyl-4-trifluorométhoxyaniline peut être préparé de la manière suivante : sur une solution de 30 g de 4-trifluorométhoxyaniline dans 170 ml de pyridine, maintenue sous argon à température voisine de 20°C, on ajoute par petites fractions 32,2 g de chlorure de p-toluènesulfonyle et le mélange ainsi obtenu est agité 16 heures à la même température. Le milieu réactionnel est hydrolysé avec 250 ml d'eau distillée et extrait deux fois par 250 ml et 125 ml d'acétate d'éthyle. Les extraits organiques réunis sont séchés sur sulfate de magnésium, filtrés, concentrés à sec sous pression réduite (2 kPa). Le résidu obtenu est repris deux fois avec 200 ml de toluène et concentré à chaque fois sous pression réduite puis repris par 100 ml d'éther isopropylique. Le solide est séparé par filtration, lavé par deux fois 50 ml d'éther isopropylique et séché sous pression réduite. On obtient ainsi 45 g de N-tosyl-4-trifluorométhoxyaniline sous forme d'un solide blanc fondant à 115°C.

### EXEMPLE 38

Sur une solution, maintenue sous argon et aux environs de 17°C, de 0,42 g de thiocyanate de potassium dans 5 ml d'acide acétique on coule au goutte à goutte une solution de 0,25 g de brome dans 2 ml d'acide acétique et la suspension obtenue est agitée pendant 10 minutes avant de lui additionner une solution de 0,4 g de 4-éthylsulfinyl-6-trifluorométhyl-1,2,3,4-tétrahydroquinoléine dans 1 ml d'acide acétique. Le mélange réactionnel est agité 16 heures à une température voisine de 20°C puis coulé sur une solution aqueuse froide d'ammoniaque et d'acétate d'éthyle. L'extrait organique séparé est lavé avec de l'eau distillée, puis avec une solution aqueuse saturée en chlorure de sodium, séché sur sulfate de magnésium, filtré et concentré à sec sous pression réduite (2 kPa). Le produit obtenu (0,35 g) est mis en solution dans de l'acétate d'éthyle, puis on ajoute 0,55 ml d'acide méthanesulfonique environ 1,94 M dans l'isopropanol. Le précipité est séparé par filtration, lavé avec de l'acétate d'éthyle et séché sous pression réduite à une température de 50°C. On obtient ainsi 0,3 g de méthanesulfonate de 2-imino-6-éthylsulfinyl-8-trifluorométhyl-5,6-dihydro-2H,4H-thiazolo[5,4,3-ij]quinoléine, sous forme d'un solide crème fondant à 220°C avec décomposition [Analyse C14H17F3N2O4S3, % calculé C :39,06, H : 3,98, F : 13,24, N : 6,51, O : 14,87, S : 22,34, % trouvé C : 38,37, H : 4,21, F : 12,27, N : 6,48, S : 22,22].

La 4-éthylsulfinyl-6-trifluorométhyl-1,2,3,4-tétrahydroquinoléine peut être préparé de la manière suivante : à une solution de 2 g de 4-éthylsulfinyl-6-trifluorométhyl-1,2,3,4-tétrahydroquinoléine-1-carboxylate de *tert*-butyle dans 25 ml de dichlorométhane, on ajoute 25 g de silice préalablement séchés à 50°C sous pression réduite pendant 2 heures. Le mélange est concentré à sec sous pression réduite et le résidu obtenu est maintenu à 50°C sous pression réduite pendant 72 heures. Après retour à une température voisine de 20°C, la silice est reprise avec du tétrahydrofurane et séparée par filtration. Le filtrat est concentré à sec sous pression réduite et le résidu obtenu est purifié par chromatographie sur colonne de gel de silice en éluant avec de l'acétate d'éthyle. On obtient 0,45 g de 4-éthylsulfinyl-6-trifluorométhyl-1,2,3,4-tétrahydroquinoléine, sous forme d'une huile jaune utilisée telle quelle.

Le 4-éthylsulfinyl-6-trifluorométhyl-1,2,3,4-tétrahydroquinoléine-1-carboxylate de *tert*-butyle peut être préparé de la manière suivante : à une solution, maintenue vers 5°C, de 4,9 g de 4-éthylthio-6-trifluorométhyl-1,2,3,4-tétrahydroquinoléine-1-carboxylate de *tert*-butyle dans 50 ml de dichlorométhane on ajoute en plusieurs parties 3,2 g d'acide 3-chloroperbenzoïque (pureté 80%). Le milieu réactionnel est agité pendant 20 heures à une température voisine de 20°C puis l'insoluble est éliminé par filtration et lavé avec du dichlorométhane. Le filtrat organique est lavé par une solution aqueuse saturée en hydrogénocarbonate de sodium, puis de l'eau distillée et séché sur sulfate de magnésium avant d'être concentré à sec sous pression réduite (2 kPa). Le résidu obtenu (5,11 g) est purifié par chromatographie sur colonne de gel de silice en éluant avec du dichlorométhane puis un mélange de dichlorométhane et d'acétate d'éthyle (80-20 en volumes). On obtient ainsi 2,9 g de 4-éthylsulfinyl-6-trifluorométhyl-1,2,3,4-tétrahydroquinoléine-1 -carboxylate de *tert*-butyle, sous forme d'une huile jaune [RMN Spectre ¹H du mélange 60/40 des diastéréoisomères :

Isomère majoritaire: spectre dans DMSO-d6, T=300K, δ en ppm (400 Mhz) : 1,25 (3H, t, J=6Hz, CH₃), 1,52 (9H, s, (CH₃)₃), entre 2,15 et 2,90 (4H, m, SCH₂ et CH₂), 3,60 et 4,00 (1H chacun, m, NCH₂), 4,40 (1H, t, J=5Hz, SCH), entre 7,60 et 8,00 (3H, m, 3 CH arom.).

Isomère minoritaire: spectre dans DMSO-d6, T=300K, δ en ppm (400 Mhz) : 1,27 (3H, t, J=6Hz, CH₃), 1,52 (9H, s, (CH₃)₃), entre 2,20 et 3,05 (4H, m, SCH₂ et CH₂), 3,70 et 3,85 (1H chacun, m, NCH₂), 4,33 (1H, t, J=5Hz, SCH), entre 7,55 et 8,00 (3H, m, 3 CH arom.)].

Le 4-éthylthio-6-trifluorométhyl-1,2,3,4-tétrahydroquinoléine-1-carboxylate de *tert*-butyle peut être préparé de la manière suivante : sur une suspension, maintenue sous argon et à une température voisine de 0°C, de 0,64 g d'hydrure de sodium (pureté 60% dans l'huile de vaseline) dans 35 ml de diméthylformamide anhydre, on coule goutte à goutte une solution de 1 g d'éthanethiol dans 5 ml de diméthylformamide. Après 10 minutes d'agitation vers 0°C, on coule une solution de 6 g de 4-bromo-6-trifluorométhyl-1,2,3,4-tétrahydroquinoléine-1-carboxylate de *tert*-butyle dans 10 ml de diméthylformamide et on laisse agiter le mélange 16 heures à une température voisine de 20°C. Le milieu est hydrolysé par 100 ml d'eau distillée et extrait par 100 ml d'acétate d'éthyle. L'extrait organique est lavé trois fois par 50 ml d'eau distillée puis par une solution aqueuse saturée en hydrogénocarbonate de sodium, séché sur sulfate de magnésium, filtré et concentré à sec sous pression réduite (2 kPa). On obtient 5,65 g de 4-éthylthio-6-trifluorométhyl-1,2,3,4-tétrahydroquinoléine-1-carboxylate de *tert*-butyle, sous forme d'une huile incolore [RMN Spectre ¹H dans DMSO-d6, T=300K, δ en ppm (250 Mhz) : 1,25 (3H, t, J=6Hz, CH₃), 1,52 (9H, s, (CH₃)₃), 2,15 (2H, m, CH₂), 2,65 (2H, m, SCH₂), 3,75 et 3,95 (1H chacun, m, NCH₂), 4,37 (1H, t, J=5Hz, SCH), 7,55 (1H, d, J=8Hz, CH arom.), 7,70 (1H, s, CH arom.), 7,95 (1H, d, J=8Hz, CH arom.)].

### EXEMPLE 39

Sur une solution, maintenue sous argon et aux environs de 17°C, de 0,525 g de thiocyanate de potassium dans 5 ml d'acide acétique on coule au goutte à goutte une solution de 0,59 g de brome dans 2 ml d'acide acétique et la suspension obtenue est agitée pendant 10 minutes avant de lui additionner une solution de 0,55 g de (R,S)-4-éthylsulfonyl-6-trifluorométhyl-1,2,3,4-tétrahydroquinoléine dans 1 ml d'acide acétique. Le mélange réactionnel est agité 16 heures à une température voisine de 20°C puis coulé sur une solution aqueuse froide d'ammoniaque. On extrait par l'acétate d'éthyle et le dichlorométhane. Les extraits organiques sont lavés à l'eau distillée, séchés sur sulfate de magnésium, filtrés et concentrés à sec sous pression réduite (2 kPa). Le résidu obtenu est purifié par chromatographie sur colonne de gel de silice en éluant avec un mélange d'acétate d'éthyle et d'éther de pétrole (60-40 en volumes). On obtient ainsi 0,32 g de (R,S)-2-imino-6-éthylsulfonyl-8-trifluorométhyl-5,6-dihydro-2H,4H-thiazolo[5,4,3-ij]quinoléine, sous forme d'un solide beige fondant à 186°C [Analyse C13H13F3N2O2S2, % calculé C : 44,56, H : 3,74, F : 16,27, N : 8,00, O : 9,13, S : 18,3, % trouvé C : 45,01, H : 4,05, N : 7,58, S : 17,21].

La (R,S)-4-éthylsulfonyl-6-trifluorométhyl-1,2,3,4-tétrahydroquinoléine peut être préparée de la manière suivante : on agite, à une température voisine de 20°C, pendant 4 heures, une solution de 1,9 g de (R,S)-4-éthylsulfonyl-6-trifluorométhyl-1,2,3,4-tétrahydroquinoléine-1-carboxylate de *tert*-butyle dans 20 ml de dichlorométhane, et de 7,4 g d'acide trifluoroacétique. Le mélange est concentré à sec sous pression réduite et le résidu obtenu est repris par de l'acétate d'éthyle. La phase organique est lavée par une solution aqueuse saturée en hydrogénocarbonate de sodium, trois fois par 20 ml d'eau distillée puis par une solution saturée en chlorure de sodium, séchée sur sulfate de magnésium, filtrée et concentrée à sec sous pression réduite (2 kPa). Le résidu obtenu est purifié par chromatographie sur colonne de gel de silice en éluant avec un mélange de dichlorométhane et d'acétate d'éthyle (95-5 en volumes). On obtient 0,57 g de (R,S)-4-éthylsulfonyl-6-trifluorométhyl-1,2,3,4-tétrahydroquinoléine, sous forme d'un solide blanc fondant à 150°C.

Le (R,S)-4-éthylsulfonyl-6-trifluorométhyl-1,2,3,4-tétrahydroquinoléine-1-carboxylate de *tert*-butyle peut être préparé de la manière suivante : à une solution, maintenue vers 5°C, de 3,3 g de (R,S)-4-éthylthio-6-trifluorométhyl-1,2,3,4-tétrahydroquinoléine-1-carboxylate de *tert*-butyle dans 40 ml de dichlorométhane on ajoute en plusieurs parties 7,8 g d'acide 3-chloroperbenzoïque (pureté 80%). Le milieu réactionnel est agité pendant 20 heures à une température voisine de 20°C puis neutralisé par 45 ml d'une solution aqueuse saturée en hydrogénocarbonate de sodium. L'extrait organique est lavé avec de l'eau distillée et séché sur sulfate de magnésium avant d'être concentré à sec sous pression réduite (2 kPa). Le résidu obtenu (4,83 g) est repris avec de l'éther éthylique, laissé 16 heures à une température voisine de 0°C et le produit est séparé par filtration, lavé avec de l'éther éthylique, séché sous pression réduite. On obtient ainsi 1,8 g de (R,S)-4-éthylsulfonyl-6-trifluorométhyl-1,2,3,4-tétrahydroquinoléine-1-carboxylate de *tert*-butyle sous forme d'un solide blanc fondant à 110°C.

### EXEMPLE 40

On opère comme à l'exemple 1 mais à partir de 0,98 g de brome dilué dans 4 ml d'acide acétique, 1,8 g de thiocyanate de potassium et 1,5 g de (R,S)-4-diméthylamino-6-trifluorométhyl-1,2,3,4-tétrahydroquinoléine dans 18 ml d'acide acétique. On obtient ainsi 2 g d'une huile jaune qui est chromatographiée sur colonne de gel de silice en éluant avec un mélange d'acétate d'éthyle et d'éther de pétrole (80-20 en volumes) puis avec de l'acétate d'éthyle pur. L'huile jaune obtenue (0,5 g) est reprise par 5 ml d'acétate d'éthyle, auxquels on ajoute 1,5 ml d'une solution 1,94 N d'acide méthanesulfonique dans l'isopropanol. Le précipité obtenu trituré dans 5 ml d'acétate d'éthyle conduit à 0,4 g de diméthanesulfonate de (R,S)-6-diméthylamino-2-imino-8-trifluorométhyl-5,6-dihydro-2H,4H-thiazolo[5,4,3-ij]quinoléine sous forme d'une poudre blanche fondant à 176°C.

La (R,S)-4-diméthylamino-6-trifluorométhyl-1,2,3,4-tétrahydroquinoléine peut être préparée de la manière suivante : sur une solution de 2,5 g de (R,S)-4-diméthylamino-6-trifluorométhyl-1,2,3,4-tétrahydroquinoléine-1-carboxylate de *tert*-butyle dans 25 ml de dichlorométhane sont ajoutés 5 ml d'acide trifluoroacétique. Après 18 heures à température voisine de 20°C la solution jaune obtenue est concentrée à sec sous pression réduite. Le résidu est traité par une solution diluée d'hydrogénocarbonate de sodium et extrait par de l'éther éthylique. La phase organique est lavée par de l'eau distillée puis par une solution saturée de chlorure de sodium, séchée sur sulfate de magnésium et concentrée à sec sous pression réduite (2 kPa) à 50°C. On obtient ainsi 1,6 g de (R,S)-4-diméthylamino-6-trifluorométhyl-1,2,3,4-tétrahydroquinoléine sous forme d'huile orangée utilisée telle quelle dans l'étape suivante [RMN Spectre ¹H dans CDCl₃, T=300K, δ en ppm (400 Mhz) : 1,90 et 2,05 (1H chacun, m, CH₂), 2,32 (6H, s, N(CH₃)₂), 3,35 et 3,50 (1H chacun, m, NCH₂), 3,55 (1H, m, NCH), 6,48 (1H, d, J=8Hz, CH arom.), 7,23 (1H, d, J=8Hz, CH arom.), 7,50 (1H, s, CH arom.)].

Le (R,S)-4-diméthylamino-6-trifluorométhyl-1,2,3,4-tétrahydroquinoléine-1-carboxylate de *tert*-butyle peut être préparé de la manière suivante : sur une solution de 2,5 g de (R,S)-4-bromo-6-trifluorométhyl-1,2,3,4-tétrahydroquinoléine-1-carboxylate de *tert*-butyle dans 25 ml de d'éthanol sont ajoutés 3,5 ml d'une solution 5,6 N de diméthylamine dans l'éthanol. Après 18 heures à température voisine de 20°C le mélange réactionnel est concentré à sec sous pression réduite. Le solide beige obtenu est repris à l'acétate d'éthyle et la phase organique est lavée par de l'eau distillée puis par une solution saturée de chlorure de sodium, séchée sur sulfate de magnésium et concentrée à sec sous pression réduite (2 kPa) à 50°C. On obtient ainsi 1,5 g d'huile jaune fluide, qui est chromatographiée sur une colonne de gel de silice en éluant avec un mélange d'éther de pétrole et d'acétate d'éthyle (95-5 en volumes). On obtient ainsi 1 g de (R,S)-4-diméthylamino-6-trifluorométhyl-1,2,3,4-tétrahydroquinoléine-1-carboxylate de *tert*-butyle sous forme d'un solide blanc fondant à 62°C.

Le (R,S)-4-bromo-6-trifluorométhyl-1,2,3,4-tétrahydroquinoléine-1-carboxylate de *tert*-butyle peut être préparé de la manière suivante : une solution de 24,7 g de 6-trifluorométhyl-1,2,3,4-tétrahydroquinoléine-1-carboxylate de *tert*-butyle dans 250 ml de tétrachlorure de carbone est irradiée avec une lampe de 100 W pendant 5 heures, au reflux, en présence de 14,5 g de N-bromosuccinimide et de 1 g de peroxyde de benzoyle. Le mélange réactionnel est filtré puis concentré à sec sous pression réduite, l'huile orange obtenue est concrétée dans 30 ml de pentane par trituration à une température de -15°C. On obtient ainsi 16,5 g de (R,S)-4-bromo-6-trifluorométhyl-1,2,3,4-tétrahydroquinoléine-1-carboxylate de *tert*-butyle sous forme d'un solide blanc fondant à 72°C.

### EXEMPLE 41

On opère comme à l'exemple 1 mais à partir de 0,976 g de brome dilué dans 2 ml d'acide acétique, 1,81 g de thiocyanate de potassium et 1,7 g de (R,S)-4-diéthylamino-6-trifluorométhyl-1,2,3,4-tétrahydroquinoléine dans 20 ml d'acide acétique. On obtient ainsi 1,7 g d'une huile orange qui est chromatographiée sur colonne de gel de silice en éluant avec un mélange d'acétate d'éthyle et d'éther de pétrole (80-20 en volumes). L'huile orange obtenue (0,74 g) est reprise par 15 ml d'éther éthylique auxquels on ajoute 1,1 ml d'une solution 4 N d'acide chlorhydrique dans l'isopropanol. On obtient ainsi 0,8 g de chlorhydrate de (R,S)-6-diéthylamino-2-imino-8-trifluorométhyl-5,6-dihydro-2H,4H-thiazolo[5,4,3-ij]quinoléine sous forme d'une poudre crème fondant à 225°C (avec décomposition).

La (R,S)-4-diéthylamino-6-trifluorométhyl-1,2,3,4-tétrahydroquinoléine peut être préparée comme à l'exemple 40 mais à partir de 2,2 g de (R,S)-4-diéthylamino-6-trifluorométhyl-1,2,3,4-tétrahydroquinoléine-1-carboxylate de *tert*-butyle dans 20 ml de dichlorométhane et 10 ml d'acide trifluoroacétique. On obtient ainsi 1,53 g de (R,S)-4-diéthylamino-6-trifluorométhyl-1,2,3,4-tétrahydroquinoléine sous forme d'une huile brune utilisée telle quelle dans l'étape suivante.

Le (R,S)-4-diéthylamino-6-trifluorométhyl-1,2,3,4-tétrahydroquinoléine-1-carboxylate de *tert*-butyle peut être préparé comme dans l'exemple 40 mais à partir de 6 g de (R,S)-4-bromo-6-trifluorométhyl-1,2,3,4-tétrahydroquinoléine-1-carboxylate de *tert*-butyle dans 60 ml d'éthanol et de 5,76 g de diéthylamine. On obtient ainsi 2,3 g d'une huile brune qui est utilisée telle quelle à l'étape suivante [RMN Spectre ¹H dans DMSO-d6, T=300K, δ en ppm (300 Mhz) : 1,03 (6H, t, J=6Hz, 2 CH₃), 1,65 et 1,95 (1H chacun, m, CH₂), 2,45 (4H, m, 2 NCH₂), 3,30 (2H, m, NCH₂), 3,95 (1H, m, NCH), 6,45 (1H, s, NH), 6,55 (1H, d, J=8Hz, CH arom.), 7,20 (1H, d, J=8Hz, CH arom.), 7,65 (1H, s, CH arom.)]

### EXEMPLE 42

On ajoute goutte à goutte en 10 minutes, à une température voisine de 20°C, 1,52 g de brome dilué dans 5 ml d'acide acétique à une solution de 2,8 g de thiocyanate de potassium dans 25 ml d'acide acétique et on laisse agiter pendant 1 heure, puis on coule une solution de 2,5 g de (R,S)-4-éthylméthylamino-6-trifluorométhyl-1,2,3,4-tétrahydroquinoléine dans 5 ml d'acide acétique. Le mélange réactionnel est agité pendant 20 heures à la même température, versé sur de la glace, alcalinisé par une solution d'ammoniaque à 20% et extrait par trois fois 100 ml d'acétate d'éthyle. Les phases organiques sont réunies, lavées par 100 ml d'eau distillée, séchées sur sulfate de magnésium et concentrées à sec sous pression réduite (2 kPa) à 40°C. Le produit brut est chromatographié sur colonne de gel de silice en éluant avec un mélange d'éther de pétrole et de tétrahydrofurane (70-30 en volumes) et conduit à 1,2 g d'une huile jaune. Cette dernière est redissoute dans 25 ml d'éther éthylique, puis additionnée de 1,6 ml d'isopropanol chlorhydrique (environ 4 N). On obtient ainsi 1 g de dichlorhydrate de (R,S)-2-imino-6-éthylméthylamino-8-trifluorométhyl-5,6-dihydro-2H,4H-thiazolo[5,4,3-ij]quinoléine sous forme d'un solide blanc fondant à 230°C avec décomposition.

La (R,S)-4-éthylméthylamino-6-trifluorométhyl-1,2,3,4-tétrahydroquinoléine peut être préparée comme à l'exemple 40 mais à partir de 3,5 g de (R,S)-4-éthylméthylamino-6-trifluorométhyl-1,2,3,4-tétrahydroquinoléine-1-carboxylate de *tert*-butyle, 10 ml de dichlorométhane et 10 ml d'acide trifluoroacétique. On obtient ainsi 2,5 g de (R,S)-4-éthylméthylamino-6-trifluorométhyl-1,2,3,4-tétrahydroquinoléine sous forme d'une huile brune utilisée telle quelle dans l'étape suivante [RMN Spectre ¹H dans DMSO-d6, T=300K, δ en ppm (300 Mhz) : 1,05 (3H, t, J=6Hz, CH₃), 1,70 et 1,90 (1H chacun, m, CH₂), 2,22 (3H, s, NCH₃), 2,45 (2H, m, NCH₂), 3,30 (2H, m, NCH₂), 3,80 (1H, m, NCH), 6,45 (1H, s, NH), 6,55 (1H, d, J=8Hz, CH arom.), 7,20 (1H, d, CH arom.), 7,55 (1H, s, CH arom.)].

La (R,S)-4-éthylméthylamino-6-trifluorométhyl-1,2,3,4-tétrahydroquinoléine-1-carboxylate de *tert*-butyle peut être préparée comme dans l'exemple 40 mais à partir de 7,5 g de (R,S)-4-bromo-6-trifluorométhyl-1,2,3,4-tétrahydroquinoléine-1-carboxylate de *tert*-butyle, 80 ml d'éthanol et 5,8 g de éthylméthylamine. On obtient ainsi 3,2 g d'huile orange visqueuse qui est utilisée telle quelle à l'étape suivante [RMN Spectre ¹H dans DMSO-d6, T=300K, δ en ppm (400 Mhz) : 1,01 (3H, t, J=6Hz, CH₃), 1,51 (9H, s, (CH₃)₃), 1,80 et 1,95 (1H chacun, m, CH₂), 2,16 (3H, s, NCH₃), 2,42 (2H, q, J=6Hz, NCH₂), 3,55 et 3,88 (1H chacun, m, NCH₂), 3,91 (1H, m, NCH), 7,50 (1H, d, J=8Hz, CH arom.), 7,78 (1H, s, CH arom.), 7,86 (1H, d, J=8Hz, CH arom.)].

### EXEMPLE 43

On opère comme à l'exemple 42 mais à partir de 0,83 g de brome dilué dans 20 ml d'acide acétique, 1,52 g de thiocyanate de potassium et 1,6 g de (R,S)-4-thiomorpholino-6-trifluorométhyl-1,2,3,4-tétrahydroquinoléine dans 15 ml d'acide acétique. On obtient ainsi 1,6 g d'une meringue orange, qui est chromatographiée sur colonne de gel de silice en éluant avec un mélange d'acétate d'éthyle et d'éther de pétrole (55-45 en volumes). L'huile jaune obtenue (0,7 g) est reprise par 10 ml d'acétate d'éthyle et on ajoute 2 ml d'une solution 1,94 N d'acide méthanesulfonique dans l'isopropanol. On obtient ainsi 0,76 g de diméthanesulfonate de (R,S)-2-imino-6-thiomorpholino-8-trifluorométhyl-5,6-dihydro-2H,4H-thiazolo[5,4,3-ij]quinoléine sous forme d'un solide rosé fondant aux environs de 180°C (avec décomposition).

La (R,S)-4-thiomorpholino-6-trifluorométhyl-1,2,3,4-tétrahydroquinoléine peut être préparée comme à l'exemple 40 mais à partir de 2,3 g de (R,S)-4-thiomorpholino-6-trifluorométhyl-1,2,3,4-tétrahydroquinoléine-1-carboxylate de *tert*-butyle dans 25 ml de dichlorométhane et 5 ml d'acide trifluoroacétique. On obtient ainsi 1,6 g de (R,S)-4-thiomorpholino-6-trifluorométhyl-1,2,3,4-tétrahydroquinoléine sous forme de cristaux fondant à 90°C.

Le (R,S)-4-thiomorpholino-6-trifluorométhyl-1,2,3,4-tétrahydroquinoléine-1-carboxylate de *tert*-butyle peut être préparé comme à l'exemple 40, mais à partir de 6 g de (R,S)-4-bromo-6-trifluorométhyl-1,2,3,4-tétrahydroquinoléine-1-carboxylate de *tert*-butyle, 60 ml d'éthanol et 3,3 g de thiomorpholine. On obtient ainsi 5,5 g d'huile visqueuse brune qui cristallise partiellement. Après trituration dans 120 ml de pentane bouillant on filtre l'insoluble et les liqueurs mères refroidies à -15°C laissent déposer des cristaux blancs. On obtient ainsi 1,5 g de (R,S)-4-thiomorpholino-6-trifluorométhyl-1,2,3,4-tétrahydroquinoléine-1-carboxylate de *tert*-butyle sous forme de cristaux blancs fondant à 116°C.

### EXEMPLE 44

On opère comme dans l'exemple 42, mais à partir d'une solution de 0,512 g de thiocyanate de potassium dans 5 ml d'acide acétique, 1 ml d'une solution de 0,55 g de brome dans 2 ml d'acide acétique et d'une solution de 0,55 g de (R,S)-4-trifluoroacétamido-6-trifluorométhyl-1,2,3,4-tétrahydroquinoléine dans 6 ml d'acide acétique. On obtient ainsi 0,48 g de (R,S)-2-imino-6-trifluoroacétamido-8-trifluorométhyl-5,6-dihydro-2H,4H-thiazolo[5,4,3-ij]quinoléine sous forme d'un solide beige fondant à 236°C.

La (R,S)-4-trifluoroacétamido-6-trifluorométhyl-1,2,3,4-tétrahydroquinoléine peut être préparée de la manière suivante : à une solution de 2 g de (R,S)-4-amino-6-trifluorométhyl-1,2,3,4-tétrahydroquinoléine dans 20 ml de pyridine refroidie aux environs de -30°C, est ajouté, goutte à goutte, 1,28 ml d'anhydride trifluoroacétique. Après 30 minutes le mélange réactionnel est repris par 150 ml d'eau distillée et extrait par 30 ml de dichlorométhane. La phase organique est lavée à 3 reprises par 50 ml d'eau distillée puis par 20 ml d'une solution d'acide chlorhydrique (1 N), puis à nouveau à 2 reprises par 50 ml d'eau distillée et enfin par 20 ml d'une solution saturée de chlorure de sodium. La phase organique séchée sur sulfate de magnésium et concentrée à sec sous pression réduite (2 kPa) à 50°C, livre 1,2 g d'un solide beige, qui est chromatographié sur gel de silice en éluant avec un mélange d'éther de pétrole et d'acétate d'éthyle (80-20 en volumes). On obtient ainsi 0,4 g de (R,S)-4-trifluoroacétamido-6-trifluorométhyl-1,2,3,4-tétrahydroquinoléine sous forme d'un solide blanc fondant à 150°C.

La (R,S)-4-amino-6-trifluorométhyl-1,2,3,4-tétrahydroquinoléine peut être préparée de la manière suivante : on chauffe à reflux pendant 5 heures, 5 g de (R,S)-4-phtalimido-6-trifluorométhyl-1,2,3,4-tétrahydroquinoléine en solution dans 50 ml de méthanol en présence de 1,4 ml d'hydrate d'hydrazine. On reprend ensuite à l'eau le mélange réactionnel et évapore sous pression réduite le méthanol. On extrait à l'acétate d'éthyle, lave à la soude, puis par une solution saturée de chlorure de sodium, puis extrait 3 fois avec 35 ml au total d'acide chlorhydrique (1 N). La phase aqueuse est ensuite alcalinisée par 20 ml de soude (10 N), puis on extrait à l'acétate d'éthyle, lave à l'eau distillée, sèche sur du sulfate de magnésium et concentre à sec sous pression réduite (2 kPa) à 60°C. On obtient ainsi 2,4 g de (R,S)-4-amino-6-trifluorométhyl-1,2,3,4-tétrahydroquinoléine sous forme d'une huile orange, qui est utilisée telle quelle à l'étape suivante [RMN Spectre ¹H dans DMSO-d6, T=300K, δ en ppm (300 Mhz) : 1,65 et 1,80 (1H chacun, m, CH₂), 3,25 (2H, m, NCH₂), 3,80 (1H, m, NCH), 6,50 (1H, s, NH), 6,55 (1H, d, J=8Hz, CH arom.), 7,15 (1H, d, J=8Hz, CH arom.), 7,50 (1H, s, CH arom.)].

La (R,S)-4-phtalimido-6-trifluorométhyl-1,2,3,4-tétrahydroquinoléine peut être préparée comme dans l'exemple 15, mais à partir de 16,25 g de tétrachlorure d'étain, 8,25 g de N-para-toluènesulfonylméthyl-4-trifluorométhylaniline dans 125 ml de dichlorométhane, 4,35 g de N-vinylphtalimide dans 60 ml de dichlorométhane. On obtient ainsi 8,1 g de (R,S)-4-phtalimido-6-trifluorométhyl-1,2,3,4-tétrahydroquinoléine sous forme d'un solide crème fondant à 177°C.

Les médicaments selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthyléneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des conditions qui requièrent l'administration d'un antagoniste du récepteur AMPA ou d'un antagoniste du récepteur NMDA. Ces composés sont notamment utiles pour traiter ou prévenir toutes les ischémies et en particulier l'ischèmie cérébrale, les effets dus à une anoxie, l'évolution de maladies neurodégénératives, de la chorée d'HUNTINGTON, de la maladie d'ALZHEIMER et autres démences, de la sclérose latérale amyotrophique ou d'autres maladies du motoneurone, de l'atrophie olivo-pontocérébelleuse, de la maladie de PARKINSON, vis-à-vis des manifestations épileptogènes et/ou convulsives, les traumatismes cérébraux ou spinaux, les traumatismes liés à la dégénérescence de l'oreille interne ou de la rétine, du tinnitus, de l'anxiété, de la dépression, de la schizophrénie, du syndrome de TOURETTE, des encéphalopathies hépatiques, des troubles du sommeil, des désordres du déficit attentionnel, des troubles des conditions hormonales (excès de la sécrétion de HG ou HL, sécrétion de corticostérone), en tant qu'analgésiques, antiinflammatoires, antianorexiques, antimigraineux, antiémétiques et pour traiter les empoisonnements par des neurotoxines ou d'autres substances agonistes du récepteur NMDA ou AMPA, ainsi que les troubles neurologiques associés aux maladies virales telles que les méningites et encéphalites virales, le SIDA, la rage, la rougeole et le tétanos, pour la prévention, la tolérance et la dépendance des symptômes d'abstinence aux drogues, à l'alcool et de l'inhibition de l'accoutumance et de la dépendance aux opiacés, barbituriques, amphétamine et benzodiazépines, dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

Les doses dépendent de l'effet recherché, de la durée du traitement et de la voie d'administration utilisée; elles sont généralement comprises entre 10 mg et 300 mg par jour par voie orale pour un adulte avec des doses unitaires allant de 5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

## Revendications

1. Composé de formule : 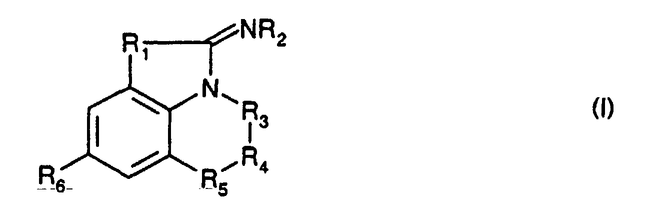 dans laquelle
R₁ représente un atome de soufre ou de sélénium,
R₂ représente un atome d'hydrogène ou un radical alkyle,
-R₃-R₄-R₅- représente une chaîne de formule -CH₂-CH₂-CH₂-, -CH(R₇)-CH₂-CH₂-, -CH₂-CH₂-CH(R₈)-, -CH₂-CH(R₉)-CH₂-, -CH₂-CH₂-S-, -CH₂-CH₂-SO-, -CH₂-CH₂-SO₂-, -CH₂-CH₂-Se-, -CH₂-CH₂-O-, -CH₂-CH₂-CO-, -CH₂-CH₂-N(R₁₀)-, -CH₂-CO-N(R₁₀)-, -CH₂-CF₂-CH₂-, -CH₂-CF₂-CH(OH)-, -CH₂-CH(R₁₃)-S-, -CH₂-CH(R₁₃)-SO- ou -CH₂-CH(R₁₃)-SO₂-,
R₆ représente un radical polyfluoroalkyle, polyfluoroalcoxy ou polyfluoroalkylthio,
R₇ représente un radical alkyle, -CH₂OH, -CH₂-SO₂-alk ou -CH₂-NR₁₁R₁₂,
R₈ représente un radical alkyle, hydroxy, -CH₂OH, -NR₁₁R₁₂, -CH₂-NR₁₁R₁₂, -S-alk, -SO-alk, -SO₂-alk, thiényle, furyle, phényle ou phényle substitué par un substituant choisi parmi les atomes d'halogène et les radicaux alkyle et alcoxy,
R₉ représente un radical alkyle ou -CH₂OH,
R₁₀ représente un atome d'hydrogène ou un radical alkyle,
R₁₁ représente un atome d'hydrogène ou un radical alkyle, -CO-alk ou -CO-CF₃,
R₁₂ représente un atome d'hydrogène ou un radical alkyle,
ou bien R₁₁ et R₁₂ forment avec l'atome d'azote auquel ils sont rattachés un hétérocycle à 5 ou 6 chaînons, saturé ou insaturé et comportant éventuellement un autre hétéroatome choisi parmi azote, oxygène et soufre, cet hétérocycle étant non substitué ou substitué par un ou plusieurs substituants choisis parmi les radicaux alkyle, phényle, halogénophényle et phénylalkyle,
R₁₃ représente un radical alkyle ou -CH₂OH,
alk représente un radical alkyle,
étant entendu que les radicaux et portions alkyle et alcoxy contiennent 1 à 6 atomes de carbone en chaîne droite ou ramifiée,
leurs isomères, racémiques et énantiomères et leurs sels avec des acides minéraux ou organiques.

2. Composé de formule (I) selon la revendication 1 pour lequel R₆ représente un radical polyfluoroalkyle choisi parmi les radicaux trifluorométhyle, 2,2,2-trifluoroéthyle, 1,1,2,2-tétrafluoroéthyle, perfluoroéthyle, perfluoropropyle, perfluorobutyle.

3. Composé de formule (I) selon la revendication 1 pour lequel R₆ représente un radical polyfluoroalcoxy choisi parmi les radicaux trifluorométhoxy, perfluoroéthoxy, 2,2,2-trifluoroéthoxy, 1,1,2,2-tétrafluoroéthoxy, 2,2,3,3,3-pentafluoropropoxy, perfluoropropoxy, perfluorobutoxy.

4. Composé de formule (I) selon la revendication 1 pour lequel R₆ représente un radical polyfluoroalkythio choisi parmi les radicaux trifluorométhylthio, perfluoroéthylthio, perfluoropropylthio.

5. Composé de formule (I) selon l'une des revendications 1 à 4 pour lequel R₁₁ et R₁₂ forment avec l'atome d'azote auquel ils sont rattachés un hétérocycle à 5 ou 6 chaînons, saturé ou insaturé et comportant éventuellement un autre hétéroatome choisi parmi azote, oxygène et soufre choisi parmi la pyrrolidine, la pipéridine, la pipérazine, la morpholine et la thiomorpholine, ces hétérocycles étant non substitués ou substitués par un radical alkyle, phényle, halogénophényle ou phénylalkyle.

6. Composé de formule (I) selon la revendication 1 pour lequel R₁ représente un atome de soufre ou de sélénium, R₂ représente un atome d'hydrogène ou un radical alkyle, -R₃-R₄-R₅- représente une chaîne de formule -CH₂-CH₂-CH₂-, -CH(R₇)-CH₂-CH₂-, -CH₂-CF₂-CH(OH)-, -CH₂-CH₂-CH(R₈)-, -CH₂-CH₂-CO, -CH₂-CH₂-S-, -CH₂-CH₂-SO-, -CH₂-CH₂-SO₂-, R₆ représente un radical polyfluoroalkyle, polyfluoroalcoxy ou polyfluoroalkylthio, R₇ représente un radical alkyle, -CH₂OH ou -CH₂NR₁₁R₁₂, R₈ représente un radical -NR₁₁R₁₂, -SO₂-alk-, -SO-alk- ou phényle, R₁₁ représente un atome d'hydrogène ou un radical alkyle ou acyle, R₁₂ représente un atome d'hydrogène ou un radical alkyle, ou bien R₁₁ et R₁₂ forment avec l'atome d'azote auquel ils sont rattachés un hétérocycle à 5 ou 6 chaînons, saturé ou insaturé et comportant éventuellement un autre hétéroatome choisi parmi azote, oxygène ou soufre, étant entendu que les radicaux et portions alkyle et alcoxy contiennent 1 à 6 atomes de carbone en chaîne droite ou ramifiée et les radicaux acyle contiennent 2 à 6 atomes de carbone en chaîne droite ou ramifiée, leurs isomères, racémiques et énantiomères et leurs sels avec des acides minéraux ou organiques.

7. Composé de formule (I) selon la revendication 1 choisi parmi les composés suivants :
-2-imino-8-trifluorométhoxy-5,6-dihydro-2H,4H-thiazolo[5,4,3-ij]quinoléine,
-2-imino-8-trifluorométhyl-5,6-dihydro-2H,4H-thiazolo[5,4,3-ij]quinoléine,
- (R,S)-2-imino-4-méthyl-8-trifluorométhyl-5,6-dihydro-2H,4H-thiazolo[5,4,3-ij]quinoléine et ses énantiomères,
-2-imino-8-trifluorométhoxy-2H,4H-thiazolo[5,4,3-ij]quinoléine-6-one,
- 2-imino-8-trifluorométhoxy-4,5-dihydro-2H-thiazolo-[3,4,5-de][1,4]benzothiazine-6,6-dioxyde,
- (R,S)-2-imino-8-trifluorométhoxy-4,5-dihydro-2H-thiazolo-[3,4,5-de][1,4]benzothiazine-6-oxyde et ses énantiomères.
- (R,S)-2-imino-8-trifluorométhyl-4,5-dihydro-2H-thiazolo[3,4,5-de][1,4]benzothiazine-6-oxyde et ses énantiomères,
- 2-imino-8-trifluorométhyl-4,5-dihydro-2H-thiazolo[3,4,5-de][1,4]benzothiazine,
- (R,S)-2-imino-6-phényl-8-trifluorométhyl-5,6-dihydro-2H,4H-thiazolo[5,4,3-ij]quinoléine et ses énantiomères,
- 2-imino-8-trifluorométhoxy-4,5-dihydro-2H-thiazolo[3,4,5-d,e]-1,4-benzoxazine,
- (R,S)-2-imino-8-trifluorométhyl-5,6-dihydro-2H,4H-thiazolo[5,4,3-ij]quinoléine-4-méthanol et ses énantiomères,
- (R,S)-5,5-difluoro-6-hydroxy-2-imino-8-trifluorométhyl-5,6-dihydro-2H,4H-thiazolo[5,4,3-ij]quinoléine et ses énantiomères,
- (R,S)-éthylméthyl(2-imino-8-trifluorométhyl-5,6-dihydro-2H,4H-thiazolo[5,4,3-ij]quinol-4-ylméthyl)amine et ses énantiomères,
- 2-imino-8-trifluorométhyl-5,6-dihydro-2H,4H-sélénazolo[5,4,3-ij]quinoléine
- (R,S)-2-imino-6-éthylsulfinyl-8-trifluorométhyl-5,6-dihydro-2H,4H-thiazolo[5,4,3-ij]quinoléine et ses énantiomères,
- (R,S)-2-imino-6-éthylsulfonyl-8-trifluorométhyl-5,6-dihydro-2H,4H-thiazolo[5,4,3-ij]quinoléine et ses énantiomères
et leurs sels.

8. Procédé de préparation des composés de formule (I) selon la revendication 1 pour lesquels R₁ représente un atome de soufre ou de sélénium, R₂ représente un atome d'hydrogène, -R₃-R₄-R₅- représente une chaîne de formule -CH₂-CH₂-CH₂-, -CH(R₇)-CH₂-CH₂-, -CH₂-CH₂-CH(R₈)-, -CH₂-CH(R₉)-CH₂, -CH₂-CH₂-S-, -CH₂-CH₂-Se-, -CH₂-CH₂-O-, -CH₂-CH₂-CO-, -CH₂-CH₂-N(R₁₀)-, -CH₂-CO-N(R₁₀)-, -CH₂-CF₂-CH₂-, -CH₂-CF₂-CH(OH)-, -CH₂-CH(R₁₃)-S-, sour réserve que R₈ ne représente pas un radical hydroxy **caractérisé en ce que** l'on fait réagir un thiocyanate de métal alcalin ou un sélénocyanate de métal alcalin sur un dérivé de formule: 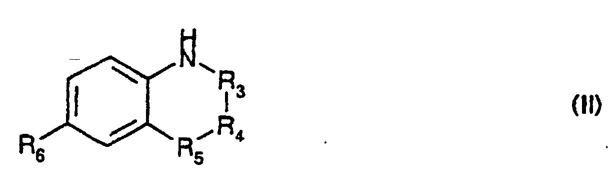 dans laquelle R₆ représente un radical polyfluoroalkyle, polyfluoroalcoxy ou polyfluoroalkylthio, -R₃-R₄-R₅- représente une chaîne de formule -CH₂-CH₂-CH₂-, -CH(R₇)-CH₂-CH₂-, -CH₂-CH₂-CH(R₈)-, -CH₂-CH(R₉)-CH₂, -CH₂-CH₂-S-, -CH₂-CH₂-Se-, -CH₂-CH₂-O-, -CH₂-CH₂-CO, -CH₂-CH₂-N(R₁₀)-, -CH₂-CO-N(R₁₀)-, -CH₂-CF₂-CH₂-, -CH₂-CF₂-CH(OH)-, -CH₂-CH(R₁₃)-S-, R₇, R₈, R₉, R₁₀ et R₁₃ ont les mêmes significations que dans la revendication 1, sous réserve que R₈ ne répresente pas un radical hydroxy, alkyle, -S-alk, -SO-alk ou SO₂-alk isole le produit et le transforme éventuellement en sel.

9. Procédé de préparation des composés de formule (I) selon la revendication 1 pour lesquels R₂ représente un radical alkyle **caractérisé en ce que** l'on alkyle un composé de formule (I) correspondant pour lequel R₂ représente un atome d'hydrogène, isole le produit et le transforme éventuellement en sel.

10. Procédé de préparation des composés de formule (I) selon la revendication 1 pour lesquels R₂ représente un atome d'hydrogène ou un radical alkyle, -R₃-R₄-R₅- représente une chaîne de formule -CH₂-CH₂-CH(R₈)- et R₈ représente un radical hydroxy **caractérisé en ce que** l'on réduit un composé de formule (I) correspondant pour lequel R₂ représente un atome d'hydrogène ou un radical alkyle et -R₃-R₄-R₅- représente une chaîne de formule -CH₂-CH₂-CO-, isole le produit et le transforme éventuellement en sel.

11. Procédé de préparation des composés de formule (I) selon la revendication 1 pour lesquels R₂ représente un atome d'hydrogène ou un radical alkyle et -R₃-R₄-R₅- représente une chaîne de formule -CH₂-CH₂-SO-, -CH₂-CH₂-SO₂-, -CH₂-CH(R₁₃)-SO- ou -CH₂-CH(R₁₃)-SO₂- **caractérisé en ce que** l'on oxyde un composé de formule (I) correspondant pour lequel R₂ représente un atome d'hydrogène ou un radical alkyle et -R₃-R₄-R₅- représente une chaîne de formule -CH₂-CH₂-S- ou -CH₂-CH(R₁₃)-S-, isole le produit et le transforme éventuellement en sel.

12. Procédé de préparation des composés de formule (I) selon la revendication 1 pour lesquels -R₃-R₄-R₅- représente une chaîne de formule -CH(R₇)-CH₂-CH₂- dans laquelle R₇ représente un radical -CH₂-NR₁₁R₁₂ et R₂ représente un atome d'hydrogène **caractérisé en ce que** l'on fait réagir une amine HNR₁₁R₁₂ pour laquelle R₁₁ et R₁₂ ont les mêmes significations que dans la revendication 1 sur un dérivé de formule : 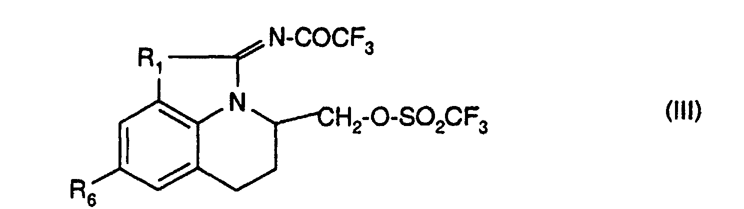 dans laquelle R₁ et R₆ ont les mêmes significations que dans la revendication 1 suivie d'une hydrolyse pour libérer l'imine, isole le produit et le transforme éventuellement en sel.

13. Composé de formule : 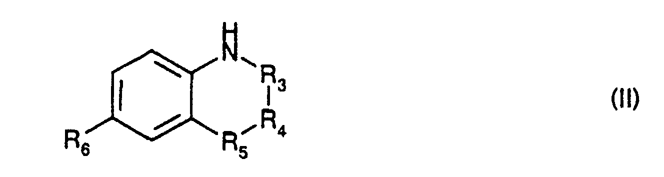 dans laquelle R₆ a les mêmes significations que dans la revendication 1, -R₃-R₄-R₅- représente une chaîne de formule -CH₂-CH₂-CH₂-, -CH(R₇)-CH₂-CH₂-, -CH₂-CH₂-CH(R₈)-, -CH₂-CH(R₉)-CH₂, -CH₂-CH₂-S-, -CH₂-CH₂-Se-, -CH₂-CH₂-O-, -CH₂-CH₂-CO-, -CH₂-CH₂-N(R₁₀)-, -CH₂-CO-N(R₁₀)-, -CH₂-CF₂-CH₂-, -CH₂-CF₂-CH(OH) ou -CH₂-CH(R₁₃)-S-, R₈ représente un radical alkyle, -CH₂-OH, -NR₁₁R₁₂, -CH₂-NR₁₁R₁₂, thiényle, furyle, phényle ou phényle substitué par un substituant choisi parmi les atomes d'halogène et les radicaux alkyle et alcoxy, R₇, R₉, R₁₀, R₁₁, R₁₂, R₁₃ ont les mêmes significations que dans la revendication 1 à l'exception de la 6-trifluorométhyl-1,2,3,4-tétrahydroquinoléine, la 2-méthyl-6-trifluorométhoxy-1,2,3,4-quinoléine, la 6-trifluorométhyl-1,2,3,4-tétrahydroquinoxaline et la 2-oxo-7-trifluorométhyl-3,4-dihydroquinoxaline.

14. A titre de médicament, les composés de formule (I) ainsi que leurs sels pharmaceutiquement acceptables, tels que défini aux revendications 1 à 7.

15. Les compositions pharmaceutiques renfermant un médicament tel que défini à la revendication 14 et un excipient pharmaceutiquement compatible.

## Patentansprüche

1. Verbindung der Formel (I) 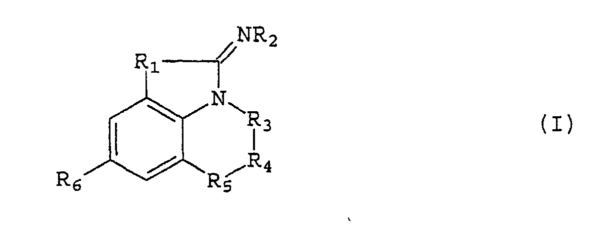 in der
R₁ ein Atom von Schwefel oder Selen darstellt,
R₂ ein Wasserstoffatom oder ein Rest Alkyl ist,
-R₃-R₄-R₅- eine Kette der Formel -CH₂-CH₂-CH₂-, -CH(R₇)-CH₂-CH₂-, -CH₂-CH₂-CH(R₈)-, -CH₂-CH(R₉)-CH₂-, -CH₂-CH₂-S-, -CH₂-CH₂-SO-, -CH₂-CH₂-SO₂-, -CH₂-CH₂-Se-, -CH₂-CH₂-O-, -CH₂-CH₂-CO-, -CH₂-CH₂-N(R₁₀)-, -CH₂-CO-N(R₁₀) -, -CH₂-CF₂-CH₂-, -CH₂-CF₂-CH(OH)-, -CH₂-CH(R₁₃) -S-, -CH₂-CH(R₁₃)-SO- oder -CH₂-CH(R₁₃)-SO₂- bedeutet,
R₆ einen Rest Polyfluoralkyl, Polyfluoralkoxy oder Polyfluoralkylthio darstellt,
R₇ ein Rest Alkyl, -CH₂OH, -CH₂-SO₂-alk oder -CH₂-NR₁₁R₁₂ ist,
R₈ einen Rest Alkyl, Hydroxy, -CH₂OH, -NR₁₁R₁₂, -CH₂-NR₁₁R₁₂, -S-alk, -SO-alk, -SO₂-alk, Thienyl, Furyl, Phenyl oder Phenyl, substituiert durch einen Substituenten, ausgewählt unter den Halogenatomen und den Resten Alkyl und Alkoxy, bedeutet,
R₉ einen Rest Alkyl oder -CH₂OH darstellt,
R₁₀ ein Wasserstoffatom oder ein Rest Alkyl ist,
R₁₁ ein Wasserstoffatom oder einen Rest Alkyl, -CO-alk oder -CO-CF₃ bedeutet,
R₁₂ ein Wasserstoffatom oder einen Rest Alkyl darstellt,
oder auch R₁₁ und R₁₂ zusammen mit dem Stickstoffatom, an das sie gebunden sind, einen gesättigten oder ungesättigten Heterocyclus mit 5 oder 6 Ringgliedern bilden, der gegebenenfalls ein weiteres Heteroatom umfaßt, ausgewählt unter Stickstoff, Sauerstoff und Schwefel, wobei dieser Heterocyclus unsubstituiert oder substituiert ist durch einen oder mehrere Substituenten, ausgewählt unter den Resten Alkyl, Phenyl, Halogenphenyl und Phenylalkyl,
R₁₃ ein Rest Alkyl oder -CH₂OH ist,
alk ein Rest Alkyl ist,
mit der Maßgabe, daß die Reste und Teile Alkyl und Alkoxy 1 bis 6 Kohlenstoffatome in gerader oder verzweigter Kette enthalten,
sowie ihre Isomeren, Racemate und Enantiomeren und ihre Salze mit Mineralsäuren oder organischen Säuren.

2. Verbindung der Formel (I) nach Anspruch 1, worin R₆ einen Rest Polyfluoralkyl darstellt, ausgewählt unter den Resten Trifluormethyl, 2,2,2-Trifluorethyl, 1,1,2,2-Tetrafluorethyl, Perfluorethyl, Perfluorpropyl, Perfluorbutyl.

3. Verbindung der Formel (I) nach Anspruch 1, worin R₆ einen Rest Polyfluoralkoxy darstellt, ausgewählt unter den Resten Trifluormethoxy, Perfluorethoxy, 2,2,2-Trifluorethoxy, 1,1,2,2-Tetrafluorethoxy, 2,2,3,3,3-Pentafluorpropoxy, Perfluorpropoxy, Perfluorbutoxy.

4. Verbindung der Formel (I) nach Anspruch 1, worin R₆ einen Rest Polyfluoralkylthio darstellt, ausgewählt unter den Resten Trifluormethylthio, Perfluorethylthio, Perfluorpropylthio.

5. Verbindung der Formel (I) nach einem der Ansprüche 1 bis 4, worin R₁₁ und R₁₂ zusammen mit dem Stickstoffatom, an das sie gebunden sind, einen gesättigten oder ungesättigten Heterocyclus mit 5 oder 6 Ringgliedern bilden, der gegebenenfalls ein weiteres Heteroatom umfaßt, ausgewählt unter Stickstoff, Sauerstoff und Schwefel, wobei der Heterocyclus ausgewählt wird unter Pyrrolidin, Piperidin, Piperazin, Morpholin und Thiomorpholin und unsubstituiert oder durch einen Rest Alkyl, Phenyl, Halogenphenyl oder Phenylalkyl substituiert ist.

6. Verbindung der Formel (I) nach Anspruch 1, worin R₁ ein Atom von Schwefel oder Selen darstellt, R₂ ein Wasserstoffatom oder ein Rest Alkyl ist, -R₃-R₄-R₅- eine Kette der Formel -CH₂-CH₂-CH₂-, -CH(R₇)-CH₂-CH₂-, -CH₂-CF₂-CH(OH)-, -CH₂-CH₂-CH(R₈)-, -CH₂-CH₂-CO-, -CH₂-CH₂-S-, -CH₂-CH₂-SO- oder -CH₂-CH₂-SO₂- bedeutet, R₆ einen Rest Polyfluoralkyl, Polyfluoralkoxy oder Polyfluoralkylthio darstellt, R₇ ein Rest Alkyl, -CH₂OH, oder -CH₂-NR₁₁R₁₂ ist, R₈ einen Rest -NR₁₁R₁₂, -SO₂-alk, -SO-alk oder Phenyl bedeutet, R₁₁ ein Wasserstoffatom oder einen Rest Alkyl oder Acyl bedeutet, R₁₂ ein Wasserstoffatom oder einen Rest Alkyl darstellt, oder auch R₁₁ und R₁₂ zusammen mit dem Stickstoffatom, an das sie gebunden sind, einen gesättigten oder ungesättigten Heterocyclus mit 5 oder 6 Ringgliedern bilden, der gegebenenfalls ein weiteres Heteroatom umfaßt, ausgewählt unter Stickstoff, Sauerstoff und Schwefel, mit der Maßgabe, daß die Reste und Teile Alkyl und Alkoxy 1 bis 6 Kohlenstoffatome in gerader oder verzweigter Kette und die Reste Acyl 2 bis 6 Kohlenstoffatome in gerader oder verzweigter Kette enthalten, sowie ihre Isomeren, Racemate und Enantiomeren und ihre Salze mit Mineralsäuren oder organischen Säuren.

7. Verbindung der Formel (I) nach Anspruch 1, ausgewählt unter den folgenden Verbindungen:
- 2-Imino-8-trifluormethoxy-5,6-dihydro-2H,4H-thiazolo[5,4,3-ij]-chinolin,
- 2-Imino-8-trifluormethyl-5,6-dihydro-2H,4H-thiazolo[5,4,3-ij]-chinolin,
- (R,S)-2-Imino-4-methyl-8-trifluormethyl-5,6-dihydro-2H,4H-thiazolo[5,4,3-ij]-chinolin und seine Enantiomeren,
- 2-Imino-8-trifluormethoxy-2H,4H-thiazolo[5,4,3-ij]-chinolin-6-on,
- 2-Imino-8-trifluormethoxy-4,5-dihydro-2H-thiazolo[3,4,5-de]-[1,4]-benzothiazin-6,6-dioxid,
- (R,S)-2-Imino-8-trifluormethoxy-4,5-dihydro-2H-thiazolo[3,4,5-de]-[1,4]-benzothiazin-6-oxid und seine Enantiomeren,
- (R,S)-2-Imino-8-trifluormethyl-4,5-dihydro-2H-thiazolo[3,4,5-de]-[1,4]-benzothiazin-6-oxid und seine Enantiomeren,
- 2-Imino-8-trifluormethyl-4,5-dihydro-2H-thiazolo[3,4,5-de]-[1,4]-benzothiazin,
- (R,S)-2-Imino-6-phenyl-8-trifluormethyl-5,6-dihydro-2H,4H-thiazolo[5,4,3-ij]-chinolin und seine Enantiomeren,
- 2-Imino-8-trifluormethoxy-4,5-dihydro-2H-thiazolo[3,4,5-de]-1,4-benzoxazin,
- (R,S)-2-Imino-8-trifluormethyl-5,6-dihydro-2H,4H-thiazolo-[5,4,3-ij]-chinolin-4-methanol und seine Enantiomeren,
- (R,S)-5,5-Difluor-6-hydroxy-2-imino-8-trifluormethyl-5,6-dihydro-2H,4H-thiazolo[5,4,3-ij]-chinolin und seine Enantiomeren,
- (R,S)-Ethylmethyl-(2-imino-8-trifluormethyl-5,6-dihydro-2H,4Hthiazolo[5,4,3-ij]-chinol-4-yl-methyl)-amin und seine Enantiomeren,
- 2-Imino-8-trifluormethyl-5,6-dihydro-2H,4H-selenazolo-[5,4,3-ij]-chinolin,
- (R,S)-2-Imino-6-ethylsulfinyl-8-trifluormethyl-5,6-dihydro-2H,4H-thiazolo-[5,4,3-ij]-chinolin und seine Enantiomeren,
- (R,S)-2-Imino-6-ethylsulfonyl-8-trifluormethyl-5,6-dihydro-2H,4H-thiazolo-[5,4,3-ij]-chinolin und seine Enantiomeren
und ihre Salze.

8. Verfahren zur Herstellung der Verbindungen der Formel (I) nach Anspruch 1, worin R₁ ein Atom von Schwefel oder Selen darstellt, R₂ ein Wasserstoffatom ist, -R₃-R₄-R₅- eine Kette der Formel
-CH₂-CH₂-CH₂-, -CH(R₇)-CH₂-CH₂-, -CH₂-CH₂-CH(R₈)-, -CH₂-CH(R₉)-CH₂-, -CH₂-CH₂-S-, -CH₂-CH₂-Se-, -CH₂-CH₂-O-, -CH₂-CH₂-CO-, -CH₂-CH₂-N(R₁₀)-, -CH₂-CO-N(R₁₀)-, -CH₂-CF₂-CH₂-, -CH₂-CF₂-CH(OH)-, -CH₂-CH(R₁₃)-S- bedeutet, unter dem Vorbehalt, daß R₈ keinen Rest Hydroxy darstellt, **dadurch gekennzeichnet, daß** man ein Alkalimetall-thiocyanat oder ein Alkalimetall-selenocyanat mit einem Derivat der Formel (II) 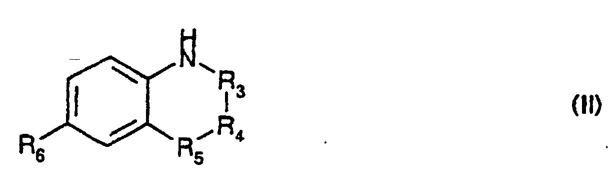 zur Reaktion bringt, in der R₆ einen Rest Polyfluoralkyl, Polyfluoralkoxy oder Polyfluoralkylthio darstellt, -R₃-R₄-R₅- eine Kette der Formel -CH₂-CH₂-CH₂-, -CH(R₇)-CH₂-CH₂-, -CH₂-CH₂-CH(R₈)-, -CH₂-CH(R₉)-CH₂-, -CH₂-CH₂-S-, -CH₂-CH₂-Se-, -CH₂-CH₂-O-, -CH₂-CH₂-CO-, -CH₂-CH₂-N(R₁₀)-, -CH₂-CO-N(R₁₀)-, -CH₂-CF₂-CH₂-, -CH₂-CF₂-CH(OH)-, -CH₂-CH(R₁₃)-S- bedeutet, R₇, R₈, R₉, R₁₀ und R₁₃ die gleichen Bedeutungen wie in Anspruch 1 besitzen, unter dem Vorbehalt, daß R₈ keinen Rest Hydroxy, Alkyl, -S-alk, -SO-alk oder -SO₂-alk darstellt, das Produkt isoliert und gegebenenfalls in Salz überführt.

9. Verfahren zur Herstellung der Verbindungen der Formel (I) nach Anspruch 1, worin R₂ ein Rest Alkyl ist, **dadurch gekennzeichnet, daß** man eine entsprechende Verbindung der Formel (I), worin R₂ ein Wasserstoffatom ist, alkyliert, das Produkt isoliert und gegebenenfalls in Salz überführt.

10. Verfahren zur Herstellung der Verbindungen der Formel (I) nach Anspruch 1, worin R₂ ein Wasserstoffatom oder ein Rest Alkyl ist, -R₃-R₄-R₅- eine Kette der Formel -CH₂-CH₂-CH(R₈)- darstellt und R₆ einen Rest Hydroxy bedeutet, **dadurch gekennzeichnet, daß** man eine entsprechende Verbindung der Formel (I), worin R₂ ein Wasserstoffatom oder ein Rest Alkyl ist und -R₃-R₄-R₅- eine Kette der Formel -CH₂-CH₂-CO- darstellt, reduziert, das Produkt isoliert und gegebenenfalls in Salz überführt.

11. Verfahren zur Herstellung der Verbindungen der Formel (I) nach Anspruch 1, worin R₂ ein Wasserstoffatom oder ein Rest Alkyl ist und -R₃-R₄-R₅- eine Kette der Formel -CH₂-CH₂-SO-, -CH₂-CH₂-SO₂-, -CH₂-CH(R₁₃)-SO- oder -CH₂-CH(R₁₃)-SO₂- darstellt, **dadurch gekennzeichnet, daß** man eine entsprechende Verbindung der Formel (I), worin R₂ ein Wasserstoffatom oder ein Rest Alkyl ist und -R₃-R₄-R₅eine Kette der Formel -CH₂-CH₂-S- oder -CH₂-CH(R₁₃)-S- darstellt, oxidiert, das Produkt isoliert und gegebenenfalls in Salz überführt.

12. Verfahren zur Herstellung der Verbindungen der Formel (I) nach Anspruch 1, worin -R₃-R₄-R₅- eine Kette der Formel -CH(R₇)-CH₂-CH₂- darstellt, R₇ ein Rest -CH₂NR₁₁R₁₂ ist und R₂ ein Wasserstoffatom bedeutet, **dadurch gekennzeichnet, daß** man ein Amin HNR₁₁R₁₂, worin R₁₁ und R₁₂ die gleichen Bedeutungen wie in Anspruch 1 besitzen, mit einem Derivat der Formel (III) 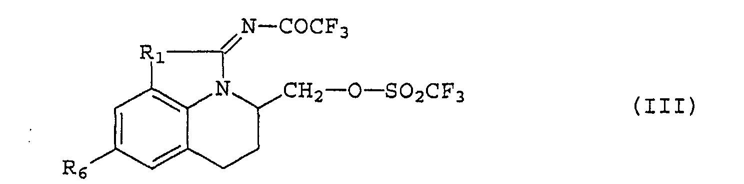 in der R₁ und R₆ die gleichen Bedeutungen wie in Anspruch 1 besitzen, zur Reaktion bringt, eine Hydrolyse anschließt, um das Imin freizusetzen, das Produkt isoliert und gegebenenfalls in Salz überführt.

13. Verbindung der Formel (II) 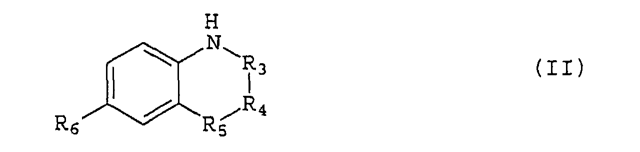 in der R₆ die gleichen Bedeutungen wie in Anspruch 1 besitzt, -R₃-R₄-R₅- eine Kette der Formel -CH₂-CH₂-CH₂-, -CH(R₇)-CH₂-CH₂-, -CH₂-CH₂-CH(R₈)-, -CH₂-CH(R₉) -CH₂-, -CH₂-CH₂-S-, -CH₂-CH₂-Se-, -CH₂-CH₂-O-, -CH₂-CH₂-CO-, -CH₂-CH₂-N(R₁₀)-, -CH₂-CO-N(R₁₀)-, -CH₂-CF₂-CH₂-, -CH₂-CF₂-CH(OH)- oder -CH₂-CH(R₁₃)-S- bedeutet, R₈ einen Rest Alkyl, -CH₂-OH, -NR₁₁R₁₂, -CH-NR₁₁R₁₂, Thienyl, Furyl, Phenyl oder Phenyl, substituiert durch einen Substituenten, ausgewählt unter den Halogenatomen und den Resten Alkyl und Alkoxy, darstellt, R₇, R₉, R₁₀, R₁₁, R₁₂, R₁₃ die gleichen Bedeutungen wie in Anspruch 1 besitzen, mit Ausnahme von 6-Trifluormethyl-1,2,3,4-tetrahydrochinolin, 2-Methyl-6-trifluorinethoxy-1,2,3,4-chinolin, 6-Trifluormethyl-1,2,3,4-tetrahydrochinoxalin und 2-Oxo-7-trifluormethyl-3,4-dihydrochinoxalin.

14. Als Arzneimittel die Verbindungen der Formel (I) sowie ihre pharmazeutisch akzeptablen Salze, wie in den Ansprüchen 1 bis 7 definiert.

15. Pharmazeutische Zusammensetzungen, umfassend ein Arzneimittel wie in Anspruch 14 definiert und einen pharmazeutisch akzeptablen Füllstoff.

## Claims

1. Compound of formula: 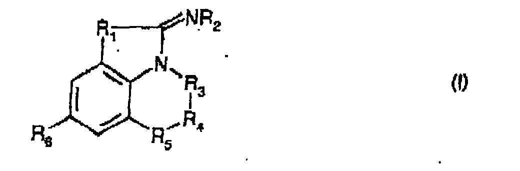 in which
R₁ represents a sulphur or selenium atom,
R₂ represents a hydrogen atom or an alkyl radical,
-R₃-R₄-R₅- represents a chain of formula -CH₂-CH₂-CH₂-, -CH(R₇)-CH₂-CH₂-, -CH₂-CH₂-CH(R₈)-, -CH₂-CH (R₉)-CH₂-, -CH₃-CH₂-S-, -CH₂-CH₂-SO-, -CH₂-CH₂-SO₂-, -CH₂-CH₂-Se-, -CH₂-CH₂-O-, -CH₂-CH₂-CO-, -CH₂-CH₂-N (R₁₀)-, -CH₂-CO-N(R₁₀)-, -CH₂-CF₂-CH₂-, -CH₂-CF₂-CH (OH)-, -CH₂-CH(R₁₃)-S-, -CH₂-CH (R₁₃)-SO- or -CH₂-CH(R₁₃)-SO₂-,
R₆ represents a polyfluoroalkyl, polyfluoroalkoxy or polyfluoroalkylthio radical,
R₇ represents an alkyl, -CH₂OH, -CH₂-SO₂-alk or -CH₂-NR₁₁R₁₂ radical,
R₈ represents a radical alkyl, hydroxyl, -CH₂OH, -NR₁₁R₁₂, -CH₂-NR₁₁R₁₂, -S-alk, -SO-alk, -SO₂-alk, thienyl, furyl, phenyl or phenyl substituted with a substituent chosen from halogen atoms and alkyl and alkoxy radicals,
R₉ represents an alkyl or -CH₂OH radical,
R₁₀ represents a hydrogen atom or an alkyl radical,
R₁₁ represents a hydrogen atom or an alkyl, -CO-alk or -CO-CF₃ radical,
R₁₂ represents a hydrogen atom or an alkyl radical,
or alternatively R₁₁ and R₁₂ form with the nitrogen atom to which they are attached a saturated or unsaturated 5- or 6-membered heterocycle optionally containing another heteroatom chosen from nitrogen, oxygen and sulphur, this heterocycle being unsubstituted or substituted with one or more substituents chosen from alkyl, phenyl, halophenyl and phenylalkyl radicals,
R₁₃ represents an alkyl or -CH₂OH radical,
alk represents an alkyl radical,
it being understood that the alkyl and alkoxy radicals and portions contain 1 to 6 carbon atoms in a straight- or branched-chain,
their isomers, racemates and enantiomers and their salts with inorganic or organic acids.

2. Compound of formula (I) according to Claim 1, for which R₆ represents a polyfluoroalkyl radical chosen from trifluoromethyl, 2,2,2-trifluoroethyl, 1,1,2,2-tetrafluoroethyl, perfluoroethyl, perfluoropropyl and perfluorobutyl radicals.

3. Compound of formula (I) according to Claim 1, for which R₆ represents a polyfluoroalkoxy radical chosen from trifluoromethoxy, perfluoroethoxy, 2,2,2-trifluoroethoxy, 1,1,2,2-tetrafluoroethoxy, 2,2,3,3,3-pentafluoropropoxy, perfluoropropoxy and perfluorobutoxy radicals.

4. Compound of formula (I) according to Claim 1, for which R₆ represents a polyfluoroalkylthio radical chosen from trifluoromethylthio, perfluoroethylthio and perfluoropropylthio radicals.

5. Compound of formula (I) according to one of Claims 1 to 4, for which R₁₁ and R₁₂ form with the nitrogen atom to which they are attached a saturated or unsaturated 5- or 6-membered heterocycle optionally containing another heteroatom chosen from nitrogen, oxygen and sulphur chosen from pyrrolidine, piperidine, piperazine, morpholine and thiomorpholine, these heterocycles being unsubstituted or substituted with an alkyl, phenyl, halophenyl or phenylalkyl radical.

6. Compound of formula (I) according to Claim 1, for which R₁ represents a sulphur or selenium atom,R₂ represents a hydrogen atom or an alkyl radical, -R₃-R₄-R₅- represents a chain of formula -CH₂-CH₂-CH₂-, -CH(R₇)-CH₂-CH₂-, -CH₂-CF₂-CH(OH) -, -CH₂-CH₂-CH(R₈)-, -CH₂-CH₂-CO, -CH₂-CH₂-S-, -CH₂-CH₂-SO-, -CH₂-CH₂-SO₂-, R₆ represents a polyfluoroalkyl, polyfluoroalkoxy or polyfluoroalkylthio radical, R₇ represents an alkyl, -CH₂OH or -CH₂NR₁₁R₁₂ radical, R₈ represents an -NR₁₁R₁₂, -SO₂-alk-, -SO-alk- or phenyl radical, R₁₁ represents a hydrogen atom or an alkyl or acyl radical, R₁₂ represents a hydrogen atom or an alkyl radical, or alternatively R₁₁ and R₁₂ form with the nitrogen atom to which they are attached a saturated or unsaturated 5-or 6-membered heterocycle optionally containing another heteroatom chosen from nitrogen, oxygen or sulphur, it being understood that the alkyl and alkoxy radicals and portions contain 1 to 6 carbon atoms in a straight- or branched-chain and the acyl radicals contain 2 to 6 carbon atoms in a straight- or branched-chain, their isomers, racemates and enantiomers and their salts with inorganic or organic acids.

7. Compound of formula (I) according to Claim 1, chosen from the following compounds:
- 2-Imino-8-trifluoromethoxy-5, 6-dihydro-2H,4H-thiazolo[5,4,3-ij]quinoline,
- 2-Imino-8-trifluoromethyl-5,6-dihydro-2H,4H-thiazolo[5,4,3-ij]quinoline,
- (R,S)-2-Imino-4-methyl-8-trifluoromethyl-5, 6-dihydro-2H,4H-thiazolo[5,4,3-ij]quinoline and its enantiomers,
- 2-Imino-8-trifluoromethoxy-2H,4H-thiazolo[5,4,3-ij]-quinolin-6-one,
- 2-Imino-8-trifluoromethoxy-4,5-dihydro-2H-thiazolo-[3,4,5-de][1,4]benzothiazine-6,6-dioxide,
- (R,S)-2-Imino-8-trifluoromethoxy-4,5-dihydro-2H-thiazolo[3,4,5-de][1,4]benzothiazine-6-oxide and its enantiomers,
- (R,S)-2-Imino-8-trifluoromethyl-4,5-dihydro-2H-thiazolo[3,4,5-de][1,4]benzothiazine-6-oxide and its enantiomers,
- 2-Imino-8-trifluoromethyl-4,5-dihydro-2H-thiazolo-[3,4,5-de] [1,4]benzothiazine,
- (R,S)-2-Imino-6-phenyl-8-trifluoromethyl-5,6-dihydro-2H,4H-thiazolo[5,4,3-ij]quinoline and its enantiomers,
- 2-Imino-8-trifluoromethoxy-4,5-dihydro-2H-thiazolo-[3, 4, 5-de] [1,4]benzoxazine,
- (R,S)-2-Imino-8-trifluoromethyl-5,6-dihydro-2H,4H-thiazolo[5,4,3-ij]quinoline-4-methanol and its enantiomers,
- (R,S) -5,5-Difluoro-6-hydroxy-2-imino-8-trifluoromethyl-5, 6-dihydro-2H, 4H-thiazolo[5,4,3-ij]quinoline and its enantiomers,
- (R,S)-Ethylmethyl (2-imino-8-trifluoromethyl-5,6-dihydro-2H,4H-thiazolo[5,4,3-ij]quinol-4-ylmethyl)-amine and its enantiomers,
- 2-Imino-8-trifluoromethyl-5,6-dihydro-2H,4H-selenazolo[5,4,3-ij]quinoline
- (R,S)-2-Imino-6-ethylsulphinyl-8-trifluoromethyl-5,6-dihydro-2H,4H-thiazolo[5,4,3-ij]quinoline and its enantiomers,
- (R, S) -2-Imino-6-ethylsulphonyl-8-trifluoromethyl-5, 6-dihydro-2H,4H-thiazolo[5,4,3-ij]quinoline and its enantiomers and their salts.

8. Process for the preparation of the compounds of formula (I) according to Claim 1, for which R₁ represents a sulphur or selenium atom, R₂ represents a hydrogen atom, -R₃-R₄-R₅- represents a chain of formula -CH₂-CH₂-CH₂-, -CH(R₇)-CH₂-CH₂-, -CH₂-CH₂-CH(R₈)-, -CH₂-CH(R₉) -CH₂, -CH₂-CH₂-S-, -CH₂-CH₂-Se-, -CH₂-CH₂-O-, -CH₂-CH₂-CO-, -CH₂-CH₂-N (R₁₀), -CH₂-CO-N(R₁₀)-, -CH₂-CF₂-CH₂-, -CH₂-CF₂-CH(OH)-, -CH₂-CH (R₁₃) -S-, with the proviso that R₈ does not represent a hydroxyl radical, **characterized in that** an alkali metal thiocyanate or an alkali metal selenocyanate is reacted with a derivative of formula: 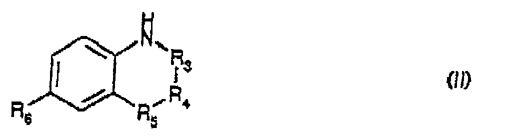 in which R₆ represents a polyfluoroalkyl, polyfluoroalkoxy or polyfluoroalkylthio radical, -R₃-R₄-R₅- represents a chain of formula -CH₂-CH₂-CH₂-, -CH(R₇)-CH₂-CH₂-, -CH₂-CH₂-CH(R₈)-, -CH₂-CH(R₉)-CH₂-, -CH₂-CH₂-S-, -CH₂-CH₂-Se-, -CH₂-CH₂-O-, -CH₂-CH₂-CO-, -CH₂-CH₂-N(R₁₀)-, -CH₂-CO-N(R₁₀)-, -CH₂-CF₂-CH₂-, -CH₂-CF₂-CH(OH)-, -CH₂-CH(R₁₃)-S-, R₇, R₈, R₉, R₁₀ and R₁₃ have the same meanings as in Claim 1, with the proviso that R₈ does not represent a hydroxyl, alkyl, -S-alk, -SO-alk or SO₂-alk radical, the product isolated and optionally converted to a salt.

9. Process for the preparation of the compounds of formula (I) according to Claim 1, for which R₂ represents an alkyl radical, **characterized in that** a corresponding compound of formula (I) for which R₂ represents a hydrogen atom is alkylated, the product isolated and optionally converted to a salt.

10. Process for the preparation of the compounds of formula (I) according to Claim 1, for which R₂ represents a hydrogen atom or an alkyl radical, -R₃-R₄-R₅- represents a chain of formula -CH₂-CH₂-CH(R₈)- and R₈ represents a hydroxyl radical, **characterized in that** a corresponding compound of formula (I) for which R₂ represents a hydrogen atom or an alkyl radical and -R₃-R₄-R₅- represents a chain of formula -CH₂-CH₂-CO- is reduced, the product isolated and optionally converted to a salt.

11. Process for the preparation of the compounds of formula (I) according to Claim 1, for which R₂ represents a hydrogen atom or an alkyl radical and -R₃-R₄-R₅- represents a chain of formula -CH₂-CH₂-SO-, -CH₂-CH₂-SO₂-, -CH₂-CH (R₁₃)-SO- or -CH₂-CH(R₁₃)-SO₂-, **characterized in that** a corresponding compound of formula (I) for which R₂ represents a hydrogen atom or an alkyl radical and -R₃-R₄-R₅- represents a chain of formula -CH₂-CH₂-S- or -CH₂-CH(R₁₃)-S- is oxidized, the product isolated and optionally converted into a salt.

12. Process for the preparation of the compounds of formula (I) according to Claim 1, for which -R₃-R₄-R₅- represents a chain of formula -CH(R₇)-CH₂-CH₂- in which R₇ represents a -CH₂-NR₁₁R₁₂ radical and R₂ represents a hydrogen atom, **characterized in that** an amine HNR₁₁R₁₂ for which R₁₁ and R₁₂ have the same meanings as in Claim 1 is reacted with a derivative of formula: 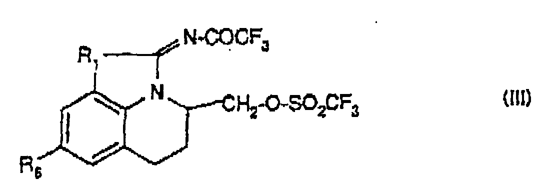 in which R₁ and R₆ have the same meanings as in Claim 1, followed by hydrolysis to release the imine, the product isolated and optionally converted to a salt.

13. Compound of formula: 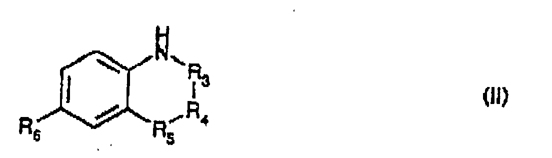 in which R₆ has the same meanings as in Claim 1, -R₃-R₄-R₅- represents a chain of formula -CH₂-CH₂-CH₂-, -CH(R₇)-CH₂-CH₂-, -CH₂-CH₂-CH(R₈)-, -CH₂-CH(R₉)-CH₂-, -CH₂-CH₂-S-, -CH₂-CH₂-Se-, -CH₂-CH₂-O-, -CH₂-CH₂-CO-, -CH₂-CH₂-N(R₁₀)-, -CH₂-CO-N(R₁₀)-, -CH₂-CF₂-CH₂-, -CH₂-CF₂-CH(OH)- or -CH₂-CH(R₁₃)-S-, R₈ represents a radical alkyl, -CH₂-OH, -NR₁₁R₁₂, -CH₂-NR₁₁R₁₂, thienyl, furyl, phenyl or phenyl substituted with a substituent chosen from halogen atoms and alkyl and alkoxy radicals, R₇, R₉, R₁₀, R₁₁, R₁₂ and R₁₃ have the same meanings as in Claim 1, with the exception of 6-trifluoromethyl-1,2,3,4-tetrahydroquinoline, 2-methyl-6-trifluoromethoxy-1,2,3,4-quinoline, 6-trifluoromethyl-1,2,3,4-tetrahydroquinoxaline and 2-oxo-7-trifluoromethyl-3,4-dihydroquinoxaline.

14. As a medicament, the compounds of formula (I) and their pharmaceutically acceptable salts, as defined in Claims 1 to 7.

15. Pharmaceutical compositions containing a medicament as defined in Claim 14 and a pharmaceutically compatible excipient.
